# EUROPEAN PATENT APPLICATION

(11) **EP 4 844 065 A1**
(43) Date of publication of application: **25.11.2026**
(21) Application number: 25741646.1
(22) Date of filing: 17.01.2025
(51) Int. Cl.: C12N 15/113, C12N 15/115, C12N 15/11, A61P 27/02, A61P 35/00, A61P 25/00

(54) **RNA INHIBITOR FOR INHIBITING EXPRESSION OF INSULIN-LIKE GROWTH FACTOR 1 RECEPTOR**

(30) Priority: 19.01.2024 CN 202410080792; 13.12.2024 CN 202411845182
(71) Applicant: Bisirna Therapeutics (Suzhou) Co., Ltd., Suzhou, Jiangsu 215028 (CN)
(72) Inventor: LU, Jianyu, Suzhou, Jiangsu 215028 (CN); HE, Huijun, Suzhou, Jiangsu 215028 (CN); WANG, Mingjie, Suzhou, Jiangsu 215028 (CN); HE, Qionger, Suzhou, Jiangsu 215028 (CN); GAO, Cuicui, Suzhou, Jiangsu 215028 (CN); ZOU, Xiaoming, Suzhou, Jiangsu 215028 (CN)
(74) Representative: Elzaburu S.L.P.
(86) International application number: PCT/CN2025/073153
(87) International publication number: WO 2025/153084

(57) **Abstract**

The present application relates to an RNA inhibitor that inhibits the expression of an insulin-like growth factor 1 receptor (IGF-1R) gene. The RNA inhibitor contains an antisense strand that forms a complementary region with at least 15 consecutive nucleotides of the mRNA encoding IGF-1R (SEQ ID NO: 828), wherein the complementary region contains 0, 1, 2, 3, 4, or 5 mismatches.

## Description

### Technical Field

The present application relates to the field of biomedicine and pharmaceuticals, specifically to an RNA inhibitor for inhibiting expression of the insulin-like growth factor 1 receptor gene and conjugates thereof.

### Background Art

RNA interference (RNAi) is a highly conserved phenomenon in evolution, induced by double-stranded RNA (dsRNA), resulting in efficient and specific degradation of homologous mRNA. This biological process involves RNA molecules disrupting specific mRNA to inhibit gene expression. Because RNAi technology can specifically eliminate or silence the expression of specific genes, it has rapidly become one of the most prominent research tools in the fields of gene function studies and gene therapy, and has been widely used to explore gene functions and to treat metabolic diseases, infectious diseases, and malignant tumors.

Thyroid eye disease (TED) is an autoimmune disorder caused by hyperthyroidism, wherein self-antigens activate IGF-1R-mediated signaling complexes on orbital cells, leading the body's own immune system to attack tissues around and behind the eyes. This disease is characterized by enlargement of extraocular muscles, adipose tissue, and connective tissue, causing symptoms such as dry eyes, proptosis, eyelid retraction, diplopia, strabismus, gritty pain, excessive tearing, redness and swelling, and difficulty in eye movement; severe cases may lead to disfigurement and vision impairment.

IGF-1R (Insulin-like growth factor 1 receptor) is a tetrameric transmembrane receptor tyrosine kinase found on the surface of human cells and serves as the cell surface receptor for IGF-1 (insulin-like growth factor 1 hormone). Studies have shown that IGF-1R is widely expressed in normal human tissues and is highly expressed in thyroid eye disease (TED). Activation of IGF-1R can stimulate cell proliferation, survival, transformation, metastasis, and angiogenesis, whereas inhibition of IGF-1R alleviates and improves symptoms of thyroid eye disease (TED). Tepezza (Teprotumumab), developed by Horizon Therapeutics, is the world's first drug approved by the U.S. FDA for treating chronic (inactive) thyroid eye disease (TED) and is marketed in the United States. This drug is a fully human monoclonal antibody (mAb) and an IGF-1R-targeted inhibitor. Unlike other biologics such as anti-CD20 antibodies used to treat TAO, which only alleviate inflammation, Tepezza not only eliminates inflammation but also effectively reduces proptosis. Patients treated with Tepezza experienced unprecedented reductions in proptosis, a condition previously addressable only surgically after the active phase of the disease had ended. However, Tepezza also presents significant safety concerns, with the most common adverse reactions including muscle spasms, hearing loss, and hyperglycemia. These adverse reactions may result from systemic administration of the drug.

Developing locally administered IGF-1R siRNA is currently a solution to the safety issues associated with monoclonal antibodies. siRNA-based therapeutics offer prolonged dosing intervals due to their long-lasting efficacy, thereby improving patient compliance. Moreover, IGF-1R is also a critical driver of tumor growth and progression; thus, developing IGF-1R-targeted siRNA holds broad potential applications in oncology.

### Summary of the Invention

The present application provides an RNA inhibitor for suppressing IGF-1R gene expression, offering an effective novel therapy for thyroid eye disease, osteoarthritis, and neuropathic pain. IGF-1R is a tetrameric transmembrane receptor tyrosine kinase found on the surface of human cells and serves as the cell surface receptor for IGF-1. The RNA inhibitor targeting IGF-1R gene expression provided herein disrupts the function of IGF-1R mRNA as a translation template, suppresses IGF-1R protein expression, and alleviates and improves symptoms of thyroid eye disease, osteoarthritis, and neuropathic pain. The siRNA therapeutic agent provided herein exhibits one or more of the following therapeutic advantages: high potency, minimal side effects, prolonged duration of action, low dosage, reduced injection-related reactions, and expected good patient compliance. Furthermore, siRNA therapeutics can enhance drug accessibility and improve delivery efficiency through appropriate formulation strategies.

In one aspect, the present application provides an RNA inhibitor for inhibiting expression of the insulin-like growth factor 1 receptor (IGF-1R) gene, comprising an antisense strand, wherein the antisense strand forms a complementary region with at least 15 consecutive nucleotides in the mRNA encoding IGF-1R (SEQ ID NO: 828), wherein the complementary region contains 0, 1, 2, 3, 4, or 5 mismatches; preferably, the complementary region is 15-30 nucleotide pairs in length, more preferably 17-23 nucleotide pairs.

In some embodiments, the antisense strand forms a complementary region with any of the following consecutive sequences of 15, 16, 17, 18, 19, 20, 21, 22, or 23 nucleotides starting from the indicated nucleotide position counted from the 5' end of the mRNA encoding IGF-1R (SEQ ID NO: 828):
nucleotide 1118, nucleotide 1120, nucleotide 1154, nucleotide 1367, nucleotide 1407, nucleotide 1415, nucleotide 1532, nucleotide 1625, nucleotide 1627, nucleotide 1628, nucleotide 1631, nucleotide 3356, nucleotide 3357, nucleotide 3359, nucleotide 3792, nucleotide 4198, nucleotide 4200, nucleotide 4208, nucleotide 4685, nucleotide 5246, nucleotide 5392, nucleotide 5393, nucleotide 6329, nucleotide 6332, nucleotide 6333, nucleotide 6334, nucleotide 10215, nucleotide 10738, nucleotide 10740, nucleotide 10770, nucleotide 10772, nucleotide 10773, nucleotide 10946, nucleotide 10956, nucleotide 10957, nucleotide 1418, nucleotide 1541, nucleotide 2050, nucleotide 2055, nucleotide 2056, nucleotide 2233, nucleotide 2234, nucleotide 2456, nucleotide 2591, nucleotide 2603, nucleotide 2606, nucleotide 2609, nucleotide 2776, nucleotide 2890, nucleotide 2906, nucleotide 2909, nucleotide 2957, nucleotide 2960, nucleotide 3008, nucleotide 3195, nucleotide 3398, nucleotide 3608, nucleotide 3609, nucleotide 3650, nucleotide 3660, nucleotide 3815, nucleotide 3882, nucleotide 3996, nucleotide 4067, nucleotide 4121, nucleotide 4122, nucleotide 4292, nucleotide 4293, nucleotide 4295, nucleotide 4516, nucleotide 4519, nucleotide 5163, nucleotide 5309, nucleotide 5310, nucleotide 5377, nucleotide 5380, nucleotide 5381, nucleotide 5663, nucleotide 5700, nucleotide 5711, nucleotide 5712, nucleotide 5721, nucleotide 5896, nucleotide 5898, nucleotide 6203, nucleotide 6236, nucleotide 6237, nucleotide 6240, nucleotide 6245, nucleotide 6288, nucleotide 6299, nucleotide 6305, nucleotide 6309, nucleotide 6310, nucleotide 6343, nucleotide 6363, nucleotide 6365, nucleotide 6366, nucleotide 6373, nucleotide 6379, nucleotide 6381, nucleotide 6431, nucleotide 6434, nucleotide 6496, nucleotide 6518, nucleotide 6755, nucleotide 6760, nucleotide 6883, nucleotide 6885, nucleotide 6947, nucleotide 6948, nucleotide 6949, nucleotide 6952, nucleotide 6953, nucleotide 6954, nucleotide 7574, nucleotide 7621, nucleotide 7764, nucleotide 7830, nucleotide 7906, nucleotide 8540, nucleotide 8836, nucleotide 8892, nucleotide 8975, nucleotide 8977, nucleotide 9163, nucleotide 9175, nucleotide 9186, nucleotide 9265, nucleotide 9267, nucleotide 9340, nucleotide 9347, nucleotide 9352, nucleotide 9356, nucleotide 9478, nucleotide 9480, nucleotide 9606, nucleotide 9611, nucleotide 9634, nucleotide 9680, nucleotide 9681, nucleotide 9724, nucleotide 9725, nucleotide 9814, nucleotide 10015, nucleotide 10016, nucleotide 10189, nucleotide 10244, nucleotide 10266, nucleotide 10490, nucleotide 10526, nucleotide 10528, nucleotide 10534, nucleotide 10535, nucleotide 10537, nucleotide 10540, nucleotide 10541, nucleotide 10679, nucleotide 10694, nucleotide 10717, nucleotide 10728, nucleotide 10729, nucleotide 10730, nucleotide 10754, nucleotide 10763, nucleotide 10766, nucleotide 10942, nucleotide 11034, nucleotide 11078, nucleotide 11126, nucleotide 11156, nucleotide 11159, nucleotide 11160, nucleotide 11292, nucleotide 11340, nucleotide 11344, nucleotide 11383, nucleotide 11395, nucleotide 11789, nucleotide 11864, nucleotide 11865, nucleotide 11939, nucleotide 12092, nucleotide 12116, nucleotide 12130, nucleotide 12154, nucleotide 12156, nucleotide 12177, nucleotide 12195, nucleotide 1164, nucleotide 1163, nucleotide 1162, nucleotide 1161, nucleotide 1160, nucleotide 1165, nucleotide 1166, nucleotide 1167, nucleotide 1168.

In some embodiments, the antisense strand forms a complementary region with the sequence between nucleotides 1160 and 1188, between nucleotides 6235 and 6465, or between nucleotides 6329 and 6455 of the mRNA encoding IGF-1R (SEQ ID NO: 828), counting from the 5' end.

In some embodiments, the RNA inhibitor is ribonucleic acid; further, the RNA inhibitor is single-stranded ribonucleic acid or double-stranded ribonucleic acid.

In some embodiments, the RNA inhibitor is an antisense oligonucleotide (ASO), shRNA, miRNA, or siRNA.

In some embodiments, it comprises a sense strand capable of forming a complementary duplex with the antisense strand, wherein the antisense strand comprises a sequence forming a duplex complementary region with at least 15 consecutive nucleotides of the sense strand sequence, wherein the duplex complementary region contains 0, 1, 2, 3, 4, or 5 mismatches; preferably, the duplex complementary region is 15-30 nucleotide pairs in length, more preferably 17-23 nucleotide pairs.

In some embodiments, the sense strand and the antisense strand reside on two separate nucleic acid strands; preferably, the RNA inhibitor is siRNA or shRNA, more preferably siRNA.

In some embodiments, the sense strand and the antisense strand reside on the same nucleic acid strand; preferably, the RNA inhibitor is shRNA.

In some embodiments, the total length of the sense strand is 15-50 nucleotides; preferably, the total length of the sense strand is 16-30 nucleotides; more preferably, the total length of the sense strand is 17, 18, 19, 20, or 21 nucleotides.

In some embodiments, the total length of the antisense strand is 19-50 nucleotides; preferably, the total length of the antisense strand is 19-30 nucleotides; more preferably, the total length of the antisense strand is 21, 22, 23, 24, 25, 26, or 27 nucleotides.

In some embodiments, the sense strand and the antisense strand independently optionally comprise a 3' or 5' overhang of 1, 2, or 3 nucleotides.

In some embodiments, both the sense strand and the antisense strand have a 3' overhang of 1-3 nucleotides, or the sense strand has a 3' or 5' overhang of 1-3 nucleotides, or the antisense strand has a 3' or 5' overhang of 1-3 nucleotides.

In some embodiments, the RNA inhibitor comprises at least 15 consecutive nucleotides from any one of the sequences set forth in SEQ ID NO: 199-400 in the antisense strand.

In some embodiments, the RNA inhibitor comprises at least 15 consecutive nucleotides from any one of the sequences set forth in SEQ ID NO: 1-198 in the sense strand.

In some embodiments, the RNA inhibitor comprises a duplex selected from the following: ds-n1, ds-n2, ds-n3, ds-n4, ds-n5, ds-n6, ds-n7, ds-n8, ds-n9, ds-n10, ds-n11, ds-n12, ds-n13, ds-n14, ds-n15, ds-n16, ds-n17, ds-n18, ds-n19, ds-n20, ds-n21, ds-n22, ds-n23, ds-n24, ds-n25, ds-n26, ds-n27, ds-n28, ds-n29, ds-n30, ds-n31, ds-n32, ds-n33, ds-n34, ds-n35, ds-n36, ds-n37, ds-n38, ds-n39, ds-n40, ds-n41, ds-n42, ds-n43, ds-n44, ds-n45, ds-n46, ds-n47, ds-n48, ds-n49, ds-n50, ds-n51, ds-n52, ds-n53, ds-n54, ds-n55, ds-n56, ds-n57, ds-n58, ds-n59, ds-n60, ds-n61, ds-n62, ds-n63, ds-n64, ds-n65, ds-n66, ds-n67, ds-n68, ds-n69, ds-n70, ds-n71, ds-n72, ds-n73, ds-n74, ds-n75, ds-n76, ds-n77, ds-n78, ds-n79, ds-n80, ds-n81, ds-n82, ds-n83, ds-n84, ds-n85, ds-n86, ds-n87, ds-n88, ds-n89, ds-n90, ds-n91, ds-n92, ds-n93, ds-n94, ds-n95, ds-n96, ds-n97, ds-n98, ds-n99, ds-n100, ds-n101, ds-n102, ds-n103, ds-n104, ds-n105, ds-n106, ds-n107, ds-n108, ds-n109, ds-n110, ds-n111, ds-n112, ds-n113, ds-n114, ds-n115, ds-n116, ds-n117, ds-n118, ds-n119, ds-n120, ds-n121, ds-n122, ds-n123, ds-n124, ds-n125, ds-n126, ds-n127, ds-n128, ds-n129, ds-n130, ds-n131, ds-n132, ds-n133, ds-n134, ds-n135, ds-n136, ds-n137, ds-n138, ds-n139, ds-n140, ds-n141, ds-n142, ds-n143, ds-n144, ds-n145, ds-n146, ds-n147, ds-n148, ds-n149, ds-n150, ds-n151, ds-n152, ds-n153, ds-n154, ds-n155, ds-n156, ds-n157, ds-n158, ds-n159, ds-n160, ds-n161, ds-n162, ds-n163, ds-n164, ds-n165, ds-n166, ds-n167, ds-n168, ds-n169, ds-n170, ds-n171, ds-n172, ds-n173, ds-n174, ds-n175, ds-n176, ds-n177, ds-n178, ds-n179, ds-n180, ds-n181, ds-n182, ds-n183, ds-n184, ds-n185, ds-n186, ds-n187, ds-n188, ds-n189, ds-n190, ds-n191, ds-n192, ds-n193, ds-n194, ds-n195, ds-n196, ds-n197, ds-n198, ds-n201, ds-n202, ds-n203, ds-n204.

In some embodiments, the RNA inhibitor comprises a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188, ds-n190, ds-n191, ds-n192, ds-n193, ds-n194, ds-n195, ds-n196, ds-n197, ds-n198.

In some embodiments, the RNA inhibitor comprises a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188.

In some embodiments, the RNA inhibitor comprises a duplex selected from the following: ds-n23, ds-n58, ds-n67, ds-n86, ds-n89, ds-n98, ds-n99, ds-n103, ds-n190, ds-n198, ds-n113.

In some embodiments, the RNA inhibitor further comprises region B1 at the 3' terminus of the antisense strand and region A1 at the 5' terminus of the sense strand, wherein region A1 is 0-6 nucleotides and region B1 is 0-6 nucleotides.

In some embodiments, the RNA inhibitor is characterized in that region B1 is 0, 1, 2, 3, 4, 5, or 6 nucleotides; preferably, region B1 is 0 or 2 nucleotides.

In some embodiments, the RNA inhibitor is characterized in that region A1 is 0, 1, 2, 3, 4, 5, or 6 nucleotides; preferably, region A1 is 0 or 2 nucleotides.

In some embodiments, the RNA inhibitor is characterized in that the antisense strand further comprises region X2 at its 5' terminus, and the sense strand or sense nucleic acid further comprises region X1 at its 3' terminus, wherein regions X1 and X2 are complementary.

In some embodiments, in the RNA inhibitor, X1 is A, U, modified A, or modified U, and X2 is U, A, modified U, or modified A.

In some embodiments, the RNA inhibitor is characterized in that the antisense strand comprises, sequentially from 3' to 5' at its 5' terminus, X2, Y, Z, and N, wherein X2 and Y are independently A, U, modified A, or modified U, Z is G or modified G, and N comprises at least one nucleotide.

In some embodiments, in the RNA inhibitor, X2, Y, and Z are sequentially AAG, AUG, UUG, or UAG from 3' to 5', or partially or fully modified sequences thereof.

In some embodiments, in the RNA inhibitor, X2, Y, and Z are sequentially AAG, AUG, UUG, or UAG from 3' to 5', or partially or fully modified sequences thereof.

In some embodiments, in the RNA inhibitor, N is C or modified C.

In some embodiments, in the RNA inhibitor, X2, Y, Z, and N are sequentially AAGC and UAGC from 3' to 5', or partially or fully modified sequences thereof.

In some embodiments, the antisense strand of the RNA inhibitor comprises at least 15 consecutive nucleotides from any one of the sequences set forth in SEQ ID NO: 401-413.

In some embodiments, the RNA inhibitor is selected from ds-n190-1, ds-n191-1, ds-n192-1, ds-n193-1, ds-n194-1, ds-n195-1, ds-n196-1, ds-n197-1, ds-n198-1, ds-n201-1, ds-n202-1, ds-n203-1, ds-n204-1.

In some embodiments, in the RNA inhibitor, at least one nucleotide is a modified nucleotide.

In some embodiments, in the RNA inhibitor, at least 70%, 80%, 90%, or 95% of the nucleotides are modified nucleotides; preferably, all nucleotides are modified nucleotides.

In some embodiments, the modifications in the RNA inhibitor comprise one or more of the following: 2'-OMe (2'-O-methyl) modification, 2'-F (2'-deoxy-2'-fluoro) modification, 2'-O-MOE (2'-O-methoxyethyl) modification, 2'-deoxy (2'-d) modification, 5'-morpholino (5'-Mo) modification, unlocked nucleic acid (UNA) modification, glycol nucleic acid (GNA) modification, locked nucleic acid (LNA) modification, tricyclo-DNA (tcDNA) modification, (S)-constrained ethyl bicyclic nucleic acid ((S)-cEt-BNA) modification, phosphorothioate (PS) modification, dithiophosphate (PS2) modification, methylphosphonate (MP) modification, methoxypropyl methylphosphonate (MOP) modification, peptide nucleic acid (PNA) modification, 5'-(E)-vinylphosphonate (VP) modification, N6-methyladenosine (m6A) modification, 5-methylcytidine (m5C) modification, 3-methyluridine (m3U) modification, 5-methyluridine (m5U) modification, pseudouridine modification, 2-thiouridine (s2U) modification, propynyluridine (5-pU) modification, linkage of an inverted abasic nucleotide (invAB) at the 5' or 3' end of a nucleotide, replacement of a nucleotide with an inverted abasic nucleotide (invAb), replacement of a nucleotide with 2,4-difluorotolyl ribonucleotide (rF), or replacement of a nucleotide with (S)-glycerol nucleic acid modification; preferred modifications include 2'-OMe, 2'-F, 2'-deoxy, VP, 5'-MP, PS, PS2, MP, MOP, M06, invAb, or invAB modifications.

In some embodiments, in the RNA inhibitor, the 3' or 5' end of the antisense strand or the 3' or 5' end of the sense strand comprises a phosphate or phosphate mimic.

In some embodiments, in the RNA inhibitor, the phosphate mimic includes 5'-(E)-vinylphosphonate, 5'-methylphosphonate, (S)-5'-C-methyl analog, and 5'-thiophosphate (5'-PS).

In some embodiments, in the RNA inhibitor, the 5' end of the antisense strand comprises an (M06) modification:

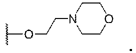

In some embodiments, in the RNA inhibitor, the 3' or 5' end of the antisense strand or the 3' or 5' end of the sense strand comprises an invAB modification.

In some embodiments, in the RNA inhibitor, the inverted abasic nucleotide or M06 is linked via a phosphorothioate bond to the 3' or 5' end of the antisense strand or the 3' or 5' end of the sense strand.

In some embodiments, the RNA inhibitor has the following combination of modifications:
a) the sense strand comprises a first sequence of 16-21 nt in length, wherein the first sequence comprises the following modifications: nucleotides at positions 9, 10, and 11 from the 5' end bear 2'-F modifications;
b) the antisense strand comprises a second sequence of 16-21 nt in length, wherein the second sequence comprises the following modifications: phosphorothioate linkages exist between nucleotides 1 and 2, between nucleotides 2 and 3, and between nucleotides 3 and 4 from the 5' end; a phosphorothioate linkage exists between nucleotides 1 and 2 from the 3' end; and nucleotides at positions 2, 3, 4, 12, 14, and 16 from the 5' end bear 2'-F modifications.

In some embodiments, the RNA inhibitor has the following combination of modifications:
a) the sense strand comprises a first sequence of 16-21 nt in length, wherein the first sequence comprises the following modifications: nucleotides at positions 9, 10, and 11 from the 5' end bear 2'-F modifications, and the remaining nucleotides bear 2'-Ome modifications;
b) the antisense strand comprises a second sequence of 16-21 nt in length, wherein the second sequence comprises the following modifications: phosphorothioate linkages exist between nucleotides 1 and 2, between nucleotides 2 and 3, and between nucleotides 3 and 4 from the 5' end; a phosphorothioate linkage exists between nucleotides 1 and 2 from the 3' end; nucleotides at positions 2, 3, 4, 12, 14, and 16 from the 5' end bear 2'-F modifications, and the remaining nucleotides bear 2'-Ome modifications.

In some embodiments, the RNA inhibitor has the following combination of modifications:
a) the sense strand comprises a first sequence of 16-21 nt in length, wherein the first sequence comprises the following modifications: nucleotides at positions 9, 10, and 11 from the 5' end bear 2'-F modifications;
b) the antisense strand comprises a second sequence of 16-21 nt in length, wherein the second sequence comprises the following modifications: phosphorothioate linkages exist between nucleotides 1 and 2, between nucleotides 2 and 3, and between nucleotides 3 and 4 from the 5' end; a phosphorothioate linkage exists between nucleotides 1 and 2 from the 3' end; and nucleotides at positions 2, 3, 4, 12, 14, and 16 from the 5' end bear 2'-F modifications.

In some embodiments, in the RNA inhibitor, the first sequence is preferably 16 nt, 17 nt, 18 nt, 19 nt, 20 nt, or 21 nt in length, more preferably 21 nt.

In some embodiments, in the RNA inhibitor, the second sequence is preferably 16 nt, 17 nt, 18 nt, 19 nt, 20 nt, or 21 nt in length, more preferably 21 nt.

In some embodiments, the RNA inhibitor has the following combination of modifications:
a) the sense strand is 21 nt in length and comprises the following modifications: nucleotides at positions 9, 10, and 11 from the 5' end bear 2'-F modifications;
b) the antisense strand is 21 nt in length and comprises the following modifications: phosphorothioate linkages exist between nucleotides 1 and 2, between nucleotides 2 and 3, and between nucleotides 3 and 4 from the 5' end; a phosphorothioate linkage exists between nucleotides 1 and 2 from the 3' end; and nucleotides at positions 2, 3, 4, 12, 14, and 16 from the 5' end bear 2'-F modifications.

In some embodiments, the RNA inhibitor has the following combination of modifications:
a) the sense strand is 21 nt in length and comprises the following modifications: nucleotides at positions 9, 10, and 11 from the 5' end bear 2'-F modifications, and the remaining nucleotides bear 2'-Ome modifications;
b) the antisense strand is 21 nt in length and comprises the following modifications: phosphorothioate linkages exist between nucleotides 1 and 2, between nucleotides 2 and 3, and between nucleotides 3 and 4 from the 5' end; a phosphorothioate linkage exists between nucleotides 1 and 2 from the 3' end; nucleotides at positions 2, 3, 4, 12, 14, and 16 from the 5' end bear 2'-F modifications, and the remaining nucleotides bear 2'-Ome modifications.

In some embodiments, in the RNA inhibitor, the antisense strand comprises at least 15 consecutive nucleotides from any one of the sequences set forth in SEQ ID NO: 624-812.

In some embodiments, in the RNA inhibitor, the sense strand comprises at least 15 consecutive nucleotides from any one of the sequences set forth in SEQ ID NO: 414-602.

In some embodiments, the RNA inhibitor is selected from:
ds-m1, ds-m2, ds-m3, ds-m4, ds-m5, ds-m6, ds-m7, ds-m8, ds-m9, ds-m10, ds-m11, ds-m12, ds-m13, ds-m14, ds-m15, ds-m16, ds-m17, ds-m18, ds-m19, ds-m20, ds-m21, ds-m22, ds-m23, ds-m24, ds-m25, ds-m26, ds-m27, ds-m28, ds-m29, ds-m30, ds-m31, ds-m32, ds-m33, ds-m34, ds-m35, ds-m36, ds-m37, ds-m38, ds-m39, ds-m40, ds-m41, ds-m42, ds-m43, ds-m44, ds-m45, ds-m46, ds-m47, ds-m48, ds-m49, ds-m50, ds-m51, ds-m52, ds-m53, ds-m54, ds-m55, ds-m56, ds-m57, ds-m58, ds-m59, ds-m60, ds-m61, ds-m62, ds-m63, ds-m64, ds-m65, ds-m66, ds-m67, ds-m68, ds-m69, ds-m70, ds-m71, ds-m72, ds-m73, ds-m74, ds-m75, ds-m76, ds-m77, ds-m78, ds-m79, ds-m80, ds-m81, ds-m82, ds-m83, ds-m84, ds-m85, ds-m86, ds-m87, ds-m88, ds-m89, ds-m90, ds-m91, ds-m92, ds-m93, ds-m94, ds-m95, ds-m96, ds-m97, ds-m98, ds-m99, ds-m100, ds-m101, ds-m102, ds-m103, ds-m104, ds-m105, ds-m106, ds-m107, ds-m108, ds-m109, ds-m110, ds-m111, ds-m112, ds-m113, ds-m114, ds-m115, ds-m116, ds-m117, ds-m118, ds-m119, ds-m120, ds-m121, ds-m122, ds-m123, ds-m124, ds-m125, ds-m126, ds-m127, ds-m128, ds-m129, ds-m130, ds-m131, ds-m132, ds-m133, ds-m134, ds-m135, ds-m136, ds-m137, ds-m138, ds-m139, ds-m140, ds-m141, ds-m142, ds-m143, ds-m144, ds-m145, ds-m146, ds-m147, ds-m148, ds-m149, ds-m150, ds-m151, ds-m152, ds-m153, ds-m154, ds-m155, ds-m156, ds-m157, ds-m158, ds-m159, ds-m160, ds-m161, ds-m162, ds-m163, ds-m164, ds-m165, ds-m166, ds-m167, ds-m168, ds-m169, ds-m170, ds-m171, ds-m172, ds-m173, ds-m174, ds-m175, ds-m176, ds-m177, ds-m178, ds-m179, ds-m180, ds-m181, ds-m182, ds-m183, ds-m184, ds-m185, ds-m186, ds-m187, ds-m188, ds-m189.

In some embodiments, in the RNA inhibitor,
a) the sense strand comprises a third sequence of 18-21 nt in length, wherein the third sequence comprises the following modifications: phosphorothioate linkages exist between nucleotides 1 and 2 and between nucleotides 2 and 3 from the 5' end; nucleotides at positions 9, 10, 11, and 18 from the 5' end bear 2'-F modifications, and the remaining nucleotides bear 2'-OMe modifications; phosphorothioate linkages exist between nucleotides 1 and 2 and between nucleotides 2 and 3 from the 3' end;
b) the antisense strand comprises a fourth sequence of 19-26 nt in length, wherein the fourth sequence comprises the following modifications: nucleotides at positions 1, 2, 5, 9, 17, and 19 from the 5' end bear 2'-F modifications, and the remaining nucleotides bear 2'-OMe modifications; a phosphorothioate linkage exists between nucleotides 4 and 5; phosphorothioate linkages exist between nucleotides 1 and 2 and between nucleotides 2 and 3 from the 3' end.

In some embodiments, in the RNA inhibitor, the third sequence is preferably 18 nt, 19 nt, 20 nt, 21 nt, or 22 nt in length, more preferably 21 nt.

In some embodiments, in the RNA inhibitor, the fourth sequence is preferably 19 nt, 20 nt, 21 nt, 22 nt, 23 nt, 24 nt, 25 nt, or 26 nt in length, more preferably 26 nt.

In some embodiments, in any of the aforementioned RNA inhibitors,
a) the sense strand is 21 nt in length; phosphorothioate linkages exist between nucleotides 1 and 2 and between nucleotides 2 and 3 from the 5' end; nucleotides at positions 9, 10, 11, and 18 from the 5' end bear 2'-F modifications, and the remaining nucleotides bear 2'-OMe modifications; phosphorothioate linkages exist between nucleotides 1 and 2 and between nucleotides 2 and 3 from the 3' end;
b) the antisense strand is 26 nt in length; nucleotides at positions 1, 2, 5, 9, 17, and 19 from the 5' end bear 2'-F modifications, and the remaining nucleotides bear 2'-OMe modifications; a phosphorothioate linkage exists between nucleotides 4 and 5; phosphorothioate linkages exist between nucleotides 1 and 2 and between nucleotides 2 and 3 from the 3' end.

In some embodiments, in the RNA inhibitor, the antisense strand comprises at least 15 consecutive nucleotides from any one of the sequences set forth in SEQ ID NO: 813-827.

In some embodiments, in the RNA inhibitor, the sense strand comprises at least 15 consecutive nucleotides from any one of the sequences set forth in SEQ ID NO: 603-617.

In some embodiments, the RNA inhibitor comprises a duplex selected from the following:
ds-m190, ds-m191, ds-m192, ds-m193, ds-m194, ds-m195, ds-m196, ds-m197, ds-m198, ds-m199, ds-m200, ds-m201, ds-m202, ds-m203, and ds-m204.

In some embodiments, the RNA inhibitor further comprises a delivery system conjugated to the sense strand and/or the antisense strand, wherein the delivery system enables the RNA inhibitor to reach the target tissue and induce gene silencing of the target RNA.

In some embodiments, the target tissue is ocular tissue, joint tissue, central nervous tissue, peripheral nervous tissue, tumor, liver tissue, kidney tissue, muscle tissue, or adipose tissue.

In some embodiments, the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.

In some embodiments, the ocular tissue is retinal ganglion, endothelial cell, extraocular muscle, or periorbital fat.

In some embodiments, the delivery system is independently conjugated to one or more internal positions of the double-stranded ribonucleic acid.

In some embodiments, the internal positions are on a nucleobase, sugar ring, methylphosphonate linkage, phosphorothioate diester bond, or phosphodiester bond.

In some embodiments, the delivery system is a lipophilic structure, wherein the lipophilic structure comprises a lipophilic group and a linker, and the lipophilic group is linked to the double-stranded ribonucleic acid via the linker.

In some embodiments, the lipophilic structure is selected from aliphatic, alicyclic, and polycyclic alicyclic compounds.

In some embodiments, the lipophilic structure contains saturated or unsaturated C16 or C22.

In some embodiments, the linker is selected from a single bond, ether, thioether, urea, carbonate, amine, amide, maleimide-thioether, disulfide, phosphodiester, sulfonamide bond, product of a click reaction, and carbamate.

In some embodiments, the delivery system is

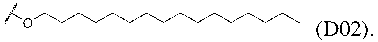

In some embodiments, the delivery system is conjugated to the 5' end and/or 3' end of the antisense strand or antisense nucleic acid fragment.

In some embodiments, the delivery system is conjugated to the 5' end and/or 3' end of the sense strand or sense nucleic acid fragment.

In some embodiments, the delivery system is conjugated to the 5' end of the antisense strand or antisense nucleic acid fragment, and the ligand is conjugated to the 3' end of the sense strand or sense nucleic acid fragment, wherein the two ligands are the same or different.

In some embodiments, the delivery system is conjugated to the 3' end of the antisense strand or antisense nucleic acid fragment, and the ligand is conjugated to the 5' end of the sense strand or sense nucleic acid fragment, wherein the two ligands are the same or different.

In some embodiments, the delivery system is conjugated to the 5' end of the antisense strand or antisense nucleic acid fragment, and the ligand is conjugated to the 5' end of the sense strand or sense nucleic acid fragment, wherein the two ligands are the same or different.

In some embodiments, the delivery system is conjugated to the 3' end of the antisense strand or antisense nucleic acid fragment, and the ligand is conjugated to the 3' end of the sense strand or sense nucleic acid fragment, wherein the two ligands are the same or different.

In some embodiments, the number of delivery systems is 1, 2, 3, 4, 5, or 6.

In some embodiments, the antisense strand comprises at least 15 consecutive nucleotides of the sequence as set forth in any one of SEQ ID NO: 822-827.

In some embodiments, the sense strand comprises at least 15 consecutive nucleotides of the sequence as set forth in any one of SEQ ID NO: 618-623.

In some embodiments, the RNA inhibitor is selected from: Z1, Z2, Z3, Z4, Z5, Z6.

In another aspect, the present application provides a pharmaceutical composition comprising said RNA inhibitor, and/or a physiologically acceptable excipient, and/or carrier, and/or diluent.

In some embodiments, the pharmaceutically acceptable carrier comprises or is selected from an aqueous carrier, liposome, polymer, or polypeptide.

In another aspect, the present application provides the use of an IGF-1R-targeting RNA inhibitor and its pharmaceutical composition in the manufacture of a medicament for treating an IGF-1R-related disease or pathology; preferably, the RNA inhibitor is siRNA; more preferably, the siRNA is administered locally or to the lesion site tissue of a subject in need thereof.

In some embodiments, the IGF-1R-related disease or pathology includes diseases or symptoms associated with elevated IGF-1R levels.

In some embodiments, the IGF-1R-related disease or pathology includes thyroid eye disease, osteoarthritis, and neuropathic pain.

In some embodiments, the RNA inhibitor and its pharmaceutical composition are the RNA inhibitor according to any one of embodiments 1-73 or the pharmaceutical composition according to any one of embodiments 74-75.

In another aspect, the present application provides the use of an RNA inhibitor or said pharmaceutical composition in the manufacture of a medicament for preventing or treating a disease or pathology or reducing the risk of a disease or symptom.

In another aspect, the present application provides a method for preventing or treating an IGF-1R-related disease or symptom, comprising administering to a subject in need thereof an effective amount of an RNA inhibitor and its pharmaceutical composition.

In some embodiments, the RNA inhibitor and its pharmaceutical composition are said RNA inhibitor or said pharmaceutical composition.

In some embodiments, the RNA inhibitor, its pharmaceutically acceptable salt, or said pharmaceutical composition is administered to a subject via intraorbital injection, intra-articular injection, intrathecal injection, subcutaneous administration, intravenous administration, oral administration, rectal administration, or intraperitoneal administration.

In some embodiments, the method comprises administering to the local site or lesion region tissue of a subject in need thereof.

In some embodiments, the local site or lesion region tissue of a subject in need thereof includes ocular tissue, joint tissue, central nervous tissue, peripheral nervous tissue, tumor, liver tissue, kidney tissue, muscle tissue, or adipose tissue.

In some embodiments, the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.

In some embodiments, the ocular tissue is retinal ganglion, endothelial cell, extraocular muscle, or periorbital fat.

In some embodiments, the IGF-1R-related disease or pathology includes diseases or symptoms associated with elevated IGF-1R levels.

In some embodiments, the IGF-1R-related disease or pathology includes thyroid eye disease, osteoarthritis, and neuropathic pain.

Other aspects and advantages of the present application will be readily apparent to those skilled in the art from the detailed description below. Only exemplary embodiments of the present application are shown and described in the detailed description below. As will be recognized by those skilled in the art, the disclosure herein enables those skilled in the art to make modifications to the specific embodiments disclosed without departing from the spirit and scope of the invention described herein.

### Brief Description of the Drawings

The specific features of the invention described herein are set forth in the appended claims. The features and advantages of the invention described herein can be better understood by reference to the exemplary embodiments and drawings described in detail below. The drawings are briefly described as follows:
Figure 1 shows the pharmacodynamic (PD) results of orbital administration of the RNA inhibitor of the present application in cynomolgus monkeys.
Figure 2 shows the structure at the 3' or 5' terminus of the sense strand or antisense strand of the RNA inhibitor described herein.

### Detailed Description

The following specific examples illustrate the embodiments of the invention described herein, and persons skilled in the art can readily understand other advantages and effects of the invention from the disclosure herein.

### Definition of Terms

In the present application, the terms "optional," "optionally," or "optionally" are equivalent in meaning and indicate that the event or condition described thereafter may or may not occur, and such description includes both the case where the event or condition occurs and the case where it does not occur. For example, "optionally substituted alkyl" or "alkyl optionally substituted" includes "alkyl" (wherein H on the alkyl is not substituted/replaced by a non-H substituent) and "substituted alkyl" (wherein H on the alkyl is substituted/replaced by a non-H substituent). As used herein, those skilled in the art will understand that, for any group containing one or more substituents, such groups are not intended to introduce any substitution or substitution pattern that is sterically impractical, synthetically unfeasible, and/or inherently unstable. For example, "optionally modified" includes both unmodified and modified; further, "nucleotide optionally modified" includes both unmodified nucleotide and modified nucleotide.

In the present application, when any variable (e.g., substituent R, e.g., nucleic acid modification) appears more than once in the composition or structure of a compound, its definition is independent in each instance. For example, if a group is substituted by 0-2 R groups, then the group may optionally be substituted by up to two R groups, and R has independent options in each instance. For another example, when multiple nucleotides are modified, each nucleotide is independently and optionally modified, and the type and number of modifications for each nucleotide may be the same or different.

In the present application, the terms "RNA inhibitor," "iRNA," "siRNA," "RNAi agent," "iRNA agent," and "RNA interference agent" are used interchangeably and generally refer to agents comprising RNA as defined herein, which mediate targeted cleavage of RNA transcripts by forming an RNA-induced silencing complex (RISC) within cells. The RNA inhibitor directs sequence-specific degradation of mRNA via a process known as RNA interference (RNAi). The RNA inhibitor described herein can modulate or inhibit gene expression of the IGF-1R gene in cells; in some embodiments, the cells may be cells of a mammalian subject. In some embodiments, the RNA inhibitor can modulate or inhibit the mRNA sequence of the IGF-1R gene (NM_000875.5 Homo sapiens insulin-like growth factor 1 receptor), which mRNA sequence includes the sequence shown in SEQ ID NO: 828.

In certain embodiments, the "RNA inhibitor" used herein is single-stranded siRNA (ssRNAi), which can be introduced into a cell or organism to inhibit target mRNA. The single-stranded RNA inhibitor can bind to the RISC endonuclease Argonaute 2, which then cleaves the target mRNA. Single-stranded RNA inhibitors are generally 15 to 30 nucleotides in length and chemically modified. Design and testing of single-stranded siRNAs are described in U.S. Patent No. 8,101,348 and Lima et al. (2012) Cell 150:883-894, the entire contents of each of which are incorporated herein by reference. Any antisense nucleotide sequence described herein may be used as a single-stranded siRNA as described herein or chemically modified using the methods described in Lima et al. (2012) Cell 150:883-894.

In certain embodiments, the "RNA inhibitor" used herein is shRNA. As used herein, the term "shRNA" (i.e., short hairpin RNA) refers to an artificial single-stranded interfering RNA molecule that contains the sense strand and/or antisense strand of an "siRNA duplex" in a stem-loop or hairpin structure. The stem of this hairpin structure is typically 19 to 29 nucleotides in length, and the loop is typically 4 to 15 nucleotides in length (see, e.g., Siolas, D. et al. (2004) Nat. Biotechnol. 23, 227-231). Typically, shRNA molecules are encoded in DNA gene expression vectors under the control of an RNA polymerase III promoter (e.g., U6 promoter).

In certain embodiments, the "RNA inhibitor" used herein is miRNA. The term "miRNA" or "microRNA" is used herein in its ordinary meaning in the art and refers to small, non-protein-coding RNA molecules that are expressed in various eukaryotes, including mammals, and participate in RNA-based gene regulation. Mature, fully processed miRNAs are approximately 15 to 30 nucleotides in length. A representative set of known endogenous miRNA species is described in the publicly available miRBase sequence database, as described in Griffith-Jones et al., Nucleic Acids Research, 2004, 32:D109-D111 and Griffith-Jones et al., Nucleic Acids Research, 2006, 34:D140-D144, and is accessible via the World Wide Web at the Wellcome Trust Sanger Institute website. Mature, fully processed miRNAs publicly available in the miRBase sequence database are incorporated herein by reference. A representative set of miRNAs is also included in Table 1 below. Each mature miRNA is partially complementary to one or more messenger RNA (mRNA) molecules, which are targets of the miRNA, thereby regulating gene expression associated with the target.

In certain embodiments, the "RNA inhibitor" used herein is ASO. As used herein, the term "ASO" (i.e., antisense oligonucleotide) refers to a nucleic acid molecule that contains the sense strand and/or antisense strand of an "siRNA duplex," which binds to target RNA via RNA-RNA, RNA-DNA, or RNA-PNA (peptide nucleic acid; Egholm et al., 1993 Nature 365, 566) interactions and alters the activity of the target RNA through steric interactions or RNase H-mediated target recognition (for a review, see Stein and Cheng, 1993 Science 261, 1004 and Woolf et al., U.S. Patent No. 5,849,902). Generally, the antisense molecule is complementary to the target sequence along a single contiguous sequence of the antisense molecule. However, in certain embodiments, the antisense molecule may bind to the substrate such that the substrate molecule forms a loop, and/or the antisense molecule may bind such that the antisense molecule itself forms a loop. Thus, the antisense molecule may be complementary to two (or even more) non-contiguous substrate sequences, or two (or even more) non-contiguous sequence portions of the antisense molecule may be complementary to the target sequence or both. For reviews of current antisense strategies, see Schmajuk et al., 1999, J. Biol. Chem., 274, 21783-21789; Delihas et al., 1997, Nature, 15, 751-753; Stein et al., 1997, Antisense N.A. Drug Dev., 7, 151; Crooke, 2000, Methods Enzymol., 313, 3-45; Crooke, 1998, Biotech. Genet. Eng. Rev., 15, 121-157; Crooke, 1997, Adv. Pharmacol., 40, 1-49. Furthermore, antisense DNA or antisense oligonucleotides modified with 2'-MOE and other modifications known in the art can be used to target RNA via DNA-RNA interactions, thereby activating RNase H, which digests the target RNA in the duplex. Antisense oligonucleotides may contain one or more RNase H activation regions capable of activating RNase H cleavage of the target RNA. Antisense DNA can be produced by chemical synthesis or gene expression using a single-stranded DNA gene expression vector or its equivalent. The antisense molecules of the present invention may be chemically modified as generally known in the art or as described herein.

In certain embodiments, the "RNA inhibitor" used herein is double-stranded RNA, referred to herein as "siRNA duplex," "double-stranded RNA inhibitor," "double-stranded siRNA molecule," "double-stranded siRNA," or "dsRNA." The term "siRNA duplex" refers to a complex of ribonucleic acid molecules having a duplex structure comprising two antiparallel and partially complementary nucleic acid strands, with the two strands oriented in "sense" and "antisense" directions relative to the target RNA (i.e., the IGF-1R gene). In some embodiments, the siRNA duplex directs sequence-specific degradation (e.g., of the mRNA sequence of the IGF-1R gene) of mRNA via a process known as RNA interference (RNAi).

Generally, the majority of nucleotides in each strand of the RNA inhibitor are ribonucleotides. Unless otherwise specified, they are ribonucleotides. However, as detailed herein, each or both strands may also include one or more non-ribonucleotides, such as a deoxyribonucleotide and/or a modified nucleotide. Moreover, the "RNA inhibitor" may include chemically modified ribonucleotides. Such modifications may include all types of modifications disclosed herein or known in the art. Any such modification used in an RNA inhibitor molecule is encompassed by the term "RNA inhibitor" for the purposes of this specification and the claims.

The duplex structure may be of any length that allows specific degradation of the desired target RNA via the RISC pathway and may range from about 19 to 36 base pairs in length, for example, about 19-30 base pairs in length, such as about 19, 20, 21, 22, 23, 24, 25, 26, 27, 28, 29, 30, 31, 32, 33, 34, 35, or 36 base pairs in length. Ranges and lengths intermediate to those listed above are also included as part of the present application. In certain embodiments, the RNA inhibitor of the present application is dsRNA with each strand comprising 15-23 nucleotides, which interacts with the target RNA sequence (e.g., the IGF-1R gene) to direct cleavage of the target RNA. In certain embodiments, the RNA inhibitor of the present application is dsRNA of 24-30 nucleotides, which interacts with the target RNA sequence (e.g., the mRNA sequence of the IGF-1R gene) to direct cleavage of the target RNA.

In the present application, the term "antisense strand" generally refers to the strand on the RNA inhibitor (e.g., duplex siRNA) that is substantially complementary to the target nucleic acid (e.g., the mRNA sequence of the target genomic sequence, including pre-mRNA and mRNA molecules, for example, the mRNA sequence of the IGF-1R gene). As used in this application, the term "complementary region" generally refers to a region on the antisense strand that is substantially complementary to the mRNA sequence of the IGF-1R gene as used herein. When the complementary region is not fully complementary, mismatches may occur in the internal or terminal regions of the molecule. Generally, mismatches are most tolerated in terminal regions, for example, within the 5, 4, 3, or 2 nucleotides at the 5' end and/or the 3' end.

As used herein, the term "mismatch" refers to situations such as non-adenine (A) pairing with thymine (T), non-adenine (A) pairing with uracil (U), non-guanine (G) pairing with cytosine (C), absence of hydrogen bonding between bases of opposing nucleotides, or absence of bases between opposing nucleotides.

As used in this application, the term "antisense nucleic acid fragment" refers to a contiguous segment on the antisense strand, wherein the segment may comprise 15 to 35 nucleotides in length. For example, the antisense nucleic acid fragment may be a contiguous segment on the antisense strand having a length of 15, 16, 17, 18, 19, 20, 21, 22, 23, 24, 25, 26, 27, 28, 29, 30, 31, 32, 33, or 34 nucleotides. The antisense nucleic acid fragment is complementary to any contiguous nucleotide segment of a length within the mRNA encoding IGF-1R. In some embodiments, at least three contiguous nucleotides of the antisense nucleic acid fragment are complementary to a corresponding contiguous nucleotide segment of the same length within the mRNA encoding IGF-1R.

As used in this application, the term "sense strand" generally refers to a strand of the RNA inhibitor that includes a region substantially complementary to the region defined herein as the antisense strand. The "sense" strand is sometimes referred to as the "coding" strand, the "passenger" strand, or the "anti-guide" strand. By virtue of their sequences, the antisense strand targets the desired mRNA, while the sense strand targets a different target. Thus, if the antisense strand is incorporated into RISC, the correct target is addressed. Incorporation of the sense strand may lead to off-target effects. These off-target effects can be mitigated by using modifications on the sense strand or by employing a 5' cap.

As used in this application, the term "sense nucleic acid fragment" refers to a contiguous segment on the sense strand, wherein the segment may comprise 15 to 35 nucleotides in length. For example, the sense nucleic acid fragment may be a contiguous segment on the sense strand having a length of 15, 16, 17, 18, 19, 20, 21, 22, 23, 24, 25, 26, 27, 28, 29, 30, 31, 32, 33, or 34 nucleotides.

As used in this application, the term "complementary" refers to the ability of a polynucleotide comprising the antisense strand or antisense nucleic acid fragment of an RNA inhibitor to hybridize (form base-pairing hydrogen bonds) under certain conditions with a polynucleotide comprising the sense strand or sense nucleic acid fragment of the RNA inhibitor or IGF-1R mRNA, thereby forming a duplex or double-helical structure. Complementary sequences include Watson-Crick base pairs or non-Watson-Crick base pairs and encompass natural or modified nucleotides or nucleotide analogs, provided that the aforementioned requirement regarding their hybridization capability is met. "Complementarity" does not require base complementarity at every nucleotide; in some cases, some mismatches may occur.

As used in this application, the term "fully complementary" generally means that all (100%) bases in a contiguous sequence of the antisense strand or antisense nucleic acid fragment of the RNA inhibitor will hybridize with an equal number of bases in a contiguous sequence of the sense strand or sense nucleic acid fragment of the RNA inhibitor or IGF-1R mRNA. The contiguous sequence may comprise all or a portion of the aforementioned sequence. As used herein, the term "partially complementary" generally means that in a pair of hybridizing nucleobase sequences, at least about 70% of the bases in a contiguous sequence of the antisense strand or antisense nucleic acid fragment of the RNA inhibitor will hybridize with an equal number of bases in a contiguous sequence of the sense strand or sense nucleic acid fragment of the RNA inhibitor or IGF-1R mRNA. As used herein, the terms "complementary," "fully complementary," and "substantially complementary" may be used with respect to base matching either between the sense strand or sense nucleic acid fragment and the antisense strand or antisense nucleic acid fragment of the RNA inhibitor, or between the antisense strand or antisense nucleic acid fragment of the RNA inhibitor and the sequence of IGF-1R mRNA. Sequence identity or complementarity is independent of modifications. For example, for the purpose of determining identity or complementarity, a and Af are complementary to U (or T) and are identical to A.

As used herein, the term "nucleotide" refers to a compound composed of a five-carbon sugar (ribose or deoxyribose), a phosphate group, and a base (natural or non-natural base), and is intended to include both unmodified (i.e., natural) nucleotides and modified nucleotides. In some embodiments, the nucleotide is an unmodified ribonucleotide. In some embodiments, the ribonucleotide is a 3'-ribonucleotide. In some embodiments, the ribonucleotide is a 5'-ribonucleotide. In some embodiments, modified or unmodified nucleotides may optionally be further modified.

Natural nucleotides consist of natural bases, natural ribose, and phosphate. The natural nucleotides used herein refer to adenosine ribonucleotide, adenosine deoxyribonucleotide, guanosine ribonucleotide, guanosine deoxyribonucleotide, cytidine ribonucleotide, cytidine deoxyribonucleotide, uridine ribonucleotide, thymidine ribonucleotide, or thymidine deoxyribonucleotide. The term "ribonucleotide" refers to a nucleotide having a hydroxyl group at the 2' position of the sugar moiety of the nucleotide. "Deoxyribonucleoside" refers to a nucleotide having a hydrogen at the 2' position of the sugar moiety of the nucleotide.

The natural bases of RNA include A (adenine), G (guanine), C (cytosine), U (uracil), and T (thymine). As used herein, prefixing a monomer (e.g., nucleotides A, U, C, G, and T, etc.) with "d" indicates that the monomer is 2'-deoxy modified. As used herein, suffixing a monomer (e.g., nucleotides A, U, C, G, and T, etc.) with "f" indicates that the monomer is 2'-deoxy-2'-fluoro modified (2'-F modification). As used herein, prefixing a monomer (e.g., nucleotides A, U, C, G, and T, etc.) with "GNA-" indicates that the monomer is glycol nucleic acid modified (GNA modification). As used herein, Tgn is a synonym for GNA-T and has equivalent meaning. As used herein, lowercase letters (a, u, c, g, t, etc.) indicate that the nucleotides represented by the corresponding uppercase letters (A, U, C, G, and T, etc.) are 2'-O-methyl (2'-OMe) modified.

In the present invention, unless otherwise specified, "G", "C", "A", "T", and "U" refer respectively to guanosine ribonucleotide, cytidine ribonucleotide, adenosine ribonucleotide, thymidine ribonucleotide, and uridine ribonucleotide, with the following structures:

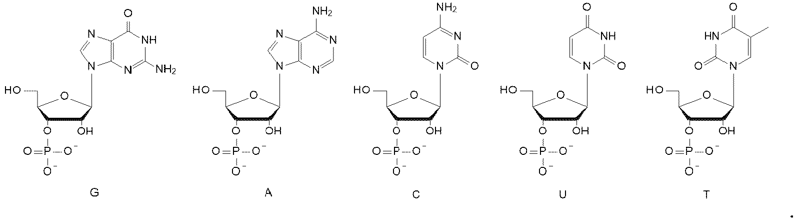

In the present invention, prefixing a nucleotide (e.g., A, U, C, G, and T, etc.) with "d" indicates that the nucleotide is 2'-deoxy modified. Exemplary structures of 2'-deoxy modified nucleotides are as follows:

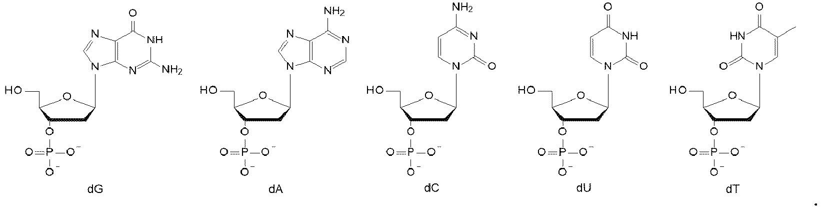

In the present invention, suffixing a nucleotide (e.g., A, U, C, G, and T, etc.) with "f" indicates that the nucleotide is 2'-deoxy-2'-fluoro modified (2'-F modification). Exemplary structures of 2'-F modified nucleotides are as follows:

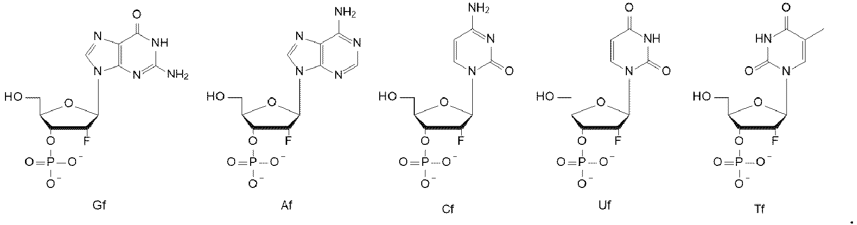

In the present invention, prefixing a nucleotide (e.g., A, U, C, G, and T, etc.) with "GNA-" indicates that the nucleotide is glycol nucleic acid modified (GNA modification). In the present invention, Tgn is a synonym for GNA-T and has equivalent meaning. Exemplary structures of GNA-modified nucleotides are as follows:

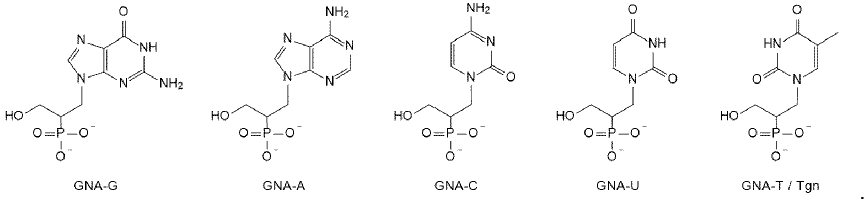

In the present invention, lowercase letters (a, u, c, g, t, etc.) indicate that the nucleotides represented by the corresponding uppercase letters (A, U, C, G, and T, etc.) are 2'-O-methyl (2'-OMe) modified. Exemplary structures of 2'-OMe modified nucleotides are as follows:

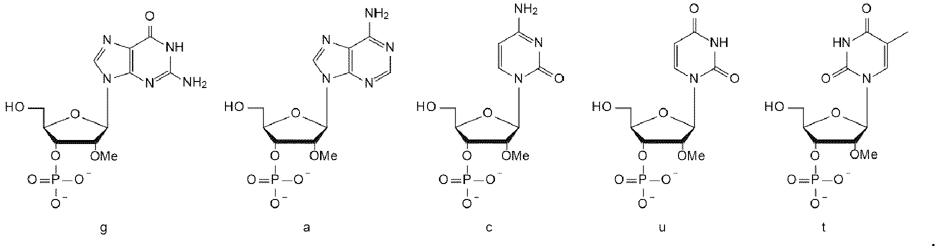

In the present invention, invAB modification refers to the linkage of an inverted abasic nucleotide at the 5' or 3' end of a nucleotide. Exemplary 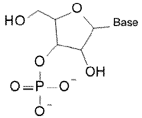 structures after invAB modification: 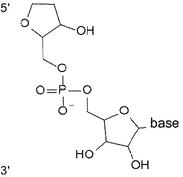 or

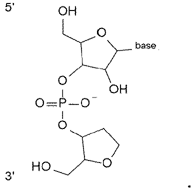

In the present invention, invAb modification refers to replacement of a nucleotide with an inverted abasic nucleotide (invAb) modification. Exemplary 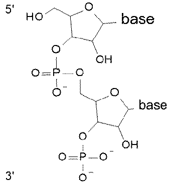 structures after invAb modification:

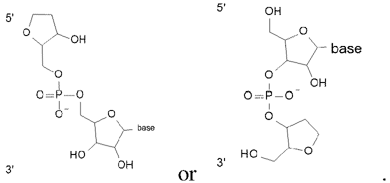

In the present invention, VP modification refers to a 5'-(E)-vinylphosphonate modification at the 5' position of a nucleotide. Exemplary structures after modification of U, u, and dU are as follows:

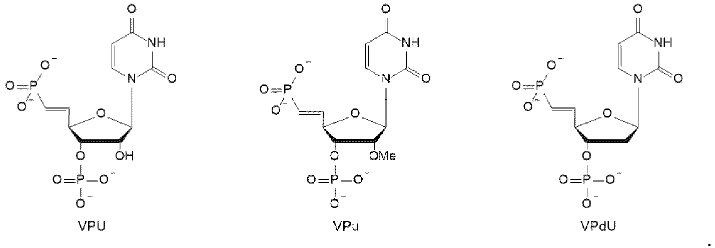

In the present invention, an asterisk ("*") between monomers (e.g., nucleotides A, U, C, G, and T, etc.) indicates that the two monomers are linked via a phosphorothioate bond (i.e., a phosphorothioate diester bond), i.e., they are phosphorothioate (PS) modified.

In the present invention, the absence of an asterisk ("*") between monomers (e.g., nucleotides A, U, C, G, and T, etc.) indicates that the two nucleotides are linked via a phosphate ester bond (i.e., a phosphodiester bond).

For example, "5'-AdUgCf*dT-3'" denotes the following: starting from the 5' end, position 1 is adenosine ribonucleotide, position 2 is uridine deoxyribonucleotide, position 3 is 2'-O-methyl-modified guanosine ribonucleotide, position 4 is 2'-fluoro-modified cytidine ribonucleotide, and position 5 is thymidine deoxyribonucleotide linked to position 4 via a phosphorothioate bond.

As used herein, "optional," "optionally," or "optionally" have equivalent meanings and indicate that the event or condition described thereafter may or may not occur, and such description includes both the case where the event or condition occurs and the case where it does not occur. For example, "optionally substituted alkyl" or "alkyl optionally substituted" includes both "alkyl" (where H atoms on the alkyl are not substituted/replaced by non-H substituents) and "substituted alkyl" (where H atoms on the alkyl are substituted/replaced by non-H substituents). As used herein, those skilled in the art will understand that for any group containing one or more substituents, such groups are not intended to introduce any substitution or substitution pattern that is sterically impractical, synthetically unfeasible, and/or inherently unstable. For example, "optionally modified" includes both unmodified and modified states; furthermore, "nucleotide optionally modified" includes both unmodified nucleotides and modified nucleotides.

As used herein, when any variable (e.g., substituent R, such as nucleic acid modification) appears more than once in the composition or structure of a compound, its definition is independent in each instance. For example, if a group is substituted by 0-2 R groups, then the group may optionally be substituted by up to two R groups, and each R has independent options in each instance. As another example, when multiple nucleotides are modified, each nucleotide is independently and optionally modified, and the type and number of modifications on each nucleotide may be the same or different.

As used herein, unless otherwise stated, "comprising," "including," "at least," "having," "possessing," "at least possessing," "containing," or equivalents are open-ended terms, meaning that in addition to the listed elements, components, or steps, other unspecified elements, components, or steps may also be included.

As used herein, "complementary" or "reverse complementary" may be used interchangeably and refer to the structural relationship between two nucleotides (e.g., on two opposing nucleic acid strands or on opposing regions of a single nucleic acid strand) that allows them to form base pairs with each other (e.g., a purine nucleotide in one nucleic acid complementary to a pyrimidine nucleotide in the opposing nucleic acid can form a base pair by establishing hydrogen bonds with each other). In some embodiments of this application, complementary nucleotides may form base pairs in a Watson-Crick manner or in any other manner that permits formation of a stable duplex. In some embodiments of this application, two nucleic acid strands may possess multiple double-stranded regions formed by complementarity. In some embodiments of this application, in DNA, adenine (A) always pairs with thymine (T); in RNA, adenine (A) pairs with uracil (U); and guanine (G) always pairs with cytosine (C). In some embodiments of this application, complementary nucleotides may also include or entirely consist of non-Watson-Crick base pairs and/or base pairs formed from non-natural and/or modified nucleotides. Such non-Watson-Crick base pairs include, but are not limited to, G:U wobble base pairing or Hoogsteen base pairing. In some embodiments of this application, a nucleotide containing hypoxanthine as its base may base-pair with a nucleotide containing adenine, cytosine, or uracil. In some embodiments of this application, nucleotides containing uracil, guanine, or adenine in the nucleotide sequence disclosed herein may be replaced by nucleotides containing, for example, inosine (in this application, the uppercase letter "I", depending on context, may denote the hypoxanthine base, inosine, or an inosine-containing nucleotide) (this replacement is abbreviated herein as I modification). In some embodiments of this application, adenine and cytosine at any position in an oligonucleotide may be replaced by guanine and uracil, respectively, to form G-U wobble base pairs with the target mRNA.

The degree to which one oligonucleotide is complementary to another oligonucleotide is termed complementarity and is measured by the percentage of bases in each strand that can form hydrogen bonds with each other, as determined by established base-pairing rules. An oligonucleotide sequence need not be "fully complementary" (i.e., "perfectly complementary") to its corresponding nucleic acid sequence. In some embodiments, a first nucleotide sequence may be considered complementary to a second nucleotide sequence if the two sequences exhibit at least 70%, 75%, 80%, 85%, 90%, 95%, 96%, 97%, 98%, or 99% sequence complementarity. In an exemplary embodiment, 18 out of 20 nucleobases of a first nucleotide sequence pair with the corresponding region of a second nucleotide sequence, achieving 90% complementarity. Non-complementary nucleobases, also referred to as "mismatches," may be clustered or dispersed among complementary bases and need not be adjacent to each other or to complementary nucleobases.

The term "mismatch" as used herein includes, but is not limited to:
1) Pairing of two opposing (individually natural or non-natural) nucleotides other than A-T, A-U, or G-C;
2) Absence of hydrogen bonding between two opposing (individually natural or non-natural) nucleotides;
3) Absence of bases between two opposing (individually natural or non-natural) nucleotides.

In some embodiments, mismatches include wobble base pairing and Hoogsteen base pairing.

The term "fully complementary" means that the first and second nucleotide sequences form a hybrid consisting solely of Watson-Crick base pairs in the fully complementary region. An "sufficiently complementary" oligonucleotide may include an internal region (e.g., at least 7, 8, 9, or 10 nucleotides) that is fully complementary to the target RNA.

As used herein, a blocking group refers to a group that may be conjugated, for example, to the 5' end of the oligonucleotide provided herein, which can reduce or inhibit exonuclease-mediated degradation. In some embodiments, the blocking group can reduce or inhibit the RNA interference effect of the oligonucleotide. In some embodiments, the blocking group is cleaved from the oligonucleotide prior to exerting an RNA interference effect. Examples of blocking groups include, but are not limited to, abasic nucleotide residues, inverted abasic nucleotide residues, M03, and M06.

As used herein, double-stranded nucleotide agents may optionally be conjugated to one or more blocking groups. The blocking group may be attached to the 3' end, the 5' end, or both ends of the sense strand, the antisense strand, or both strands. In some embodiments, the blocking group is conjugated to the antisense strand, specifically to the 5' end of the antisense strand. In some embodiments, the blocking group is conjugated to an oligonucleotide (e.g., the 5' end of the antisense strand) via a nucleotide linkage, and the internucleotide linkage is optionally modified as described herein. In some embodiments, the blocking group is linked to the double-stranded nucleotide agent via a phosphorothioate. In some embodiments, the blocking group is linked to the double-stranded nucleotide agent via a phosphodiester bond.

In the present invention, M06 modification refers to the covalent attachment of a monomer (e.g., a nucleotide) at the 5' end or the 3' end of another monomer. 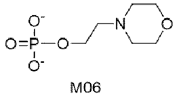 . Exemplarily, 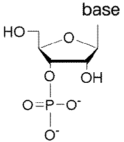 Structure after M06 modification: 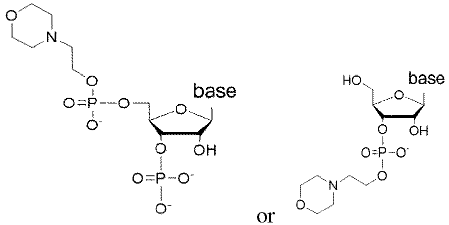 . In the present invention, (M06) 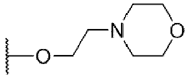 is a residue of the M06 monomer 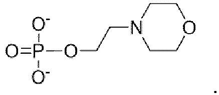 In some embodiments, (M06) is covalently 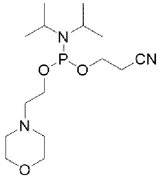 linked to the nucleotide.

In this application, the terms "nucleic acid" and "polynucleotide" are used interchangeably and refer to a polymeric form of nucleotides (deoxyribonucleotides or ribonucleotides or their analogs) of any length. The polynucleotide may have any three-dimensional structure and may perform any function. The polynucleotide may contain any one or more modifications or substitutions, as described herein or known in the art, at one or more bases, sugars, and/or phosphate esters. In some embodiments, the modified nucleotide may be a methylated nucleotide or a nucleotide analog. In some embodiments, the nucleotide structure may be modified either before or after assembly of the polynucleotide polymer. The polynucleotide may be modified post-polymerization, for example, by conjugation with a labeling moiety. The polynucleotide polymer may be blocked by non-nucleotide components. In this application, the terms "nucleic acid" and "polynucleotide" encompass both double-stranded and single-stranded molecules. Unless otherwise specified or required, any embodiment described herein as a polynucleotide includes both the double-stranded form and each of the two complementary single-stranded forms that are known or predicted to constitute the double-stranded form. For example, polynucleotides may include, but are not limited to: genes or gene fragments (e.g., probes, primers, ESTs, or SAGE tags), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, siRNA, miRNA, shRNA, RNAi agents, and primers.

In this application, the term "oligonucleotide" generally refers to a polymer composed of multiple nucleotide residues (deoxyribonucleotides or ribonucleotides, or their related structural variants or synthetic analogs) linked together by phosphodiester bonds (or their related structural variants or synthetic analogs). Thus, although the term "oligonucleotide" generally refers to nucleotide polymers in which the nucleotide residues and the linkages between them are naturally occurring, it should be understood that the scope of this term also includes various analogs, including but not limited to: peptide nucleic acids (PNA), phosphoramidates, phosphorothioates, methylphosphonates, and 2-O-methyl ribonucleic acids. The exact size of the molecule may depend on the specific application. Oligonucleotides are generally shorter in length, typically comprising about 10-30 nucleotide residues, although the term may also refer to molecules of any length, even though the terms "polynucleotide" or "nucleic acid" are generally used for longer oligonucleotides.

In certain embodiments, the oligonucleotide comprises one or more unmodified ribonucleosides (RNA) and/or unmodified deoxyribonucleosides (DNA) and/or one or more modified nucleosides. The term "modified oligonucleotide" generally refers to an oligonucleotide comprising at least one modified nucleoside and/or at least one modified internucleoside linkage.

In this application, the term "modified nucleoside" generally refers to a nucleoside that contains at least one chemical modification compared to naturally occurring RNA or DNA nucleosides. Modified nucleosides contain a modified sugar moiety and/or a modified nucleobase.

In this application, the term "nucleobase" generally refers to a heterocyclic pyrimidine or purine compound, which is a component of all nucleic acids and includes adenine (A), guanine (G), cytosine (C), thymine (T), and uracil (U). Nucleotides may include modified nucleotides or nucleotide mimics, abasic sites (Ab or X), or replacement moieties. As used herein, "nucleobase sequence" generally refers to the sequential order of contiguous nucleobases independent of any sugar, linkage, or nucleobase modifications. The terms "unmodified nucleobase" or "naturally occurring nucleobase" generally refer to the naturally occurring heterocyclic nucleobases of RNA or DNA: the purine bases adenine (A) and guanine (G); and thymine (T), cytosine (C) (including 5-methyl C), and uracil (U). "Modified nucleobase" generally refers to any nucleobase that is not naturally occurring.

In this application, the term "sugar moiety" generally refers to the naturally occurring sugar moiety of a nucleoside or a modified sugar moiety. The term "naturally occurring sugar moiety" generally refers to the furanoribosyl group found in naturally occurring RNA or the deoxyfuranoribosyl group found in naturally occurring DNA. "Modified sugar moiety" refers to a substituted sugar moiety or a sugar surrogate.

In this application, the term "internucleoside linkage" generally refers to the covalent linkage between adjacent nucleosides in an oligonucleotide. "Naturally occurring internucleoside linkage" refers to a 3' to 5' phosphodiester linkage. "Modified internucleoside linkage" refers to any internucleoside linkage other than a naturally occurring internucleoside linkage.

In this application, the terms "target nucleic acid" or "target sequence" generally refer to a contiguous portion of the nucleotide sequence of the mRNA molecule formed during transcription of the IGF-1R gene, including mRNA that is a processed product of the primary transcript. The target portion of the sequence should be at least long enough to serve as a substrate for iRNA-directed cleavage at or near that portion of the nucleotide sequence of the mRNA molecule formed during transcription of the IGF-1R gene. In one embodiment, the target sequence is within the protein-coding region of IGF-1R. The target sequence may be about 19-36 nucleotides in length, for example, preferably about 19-30 nucleotides in length. Ranges and lengths intermediate to those described above are also included as part of this application.

In this application, the term "IGF-1R protein" refers to a human tetrameric transmembrane tyrosine kinase, which is the cell surface receptor for the hormone insulin-like growth factor 1-a hormone structurally similar to the peptide hormone insulin-and plays an important role in growth, development, and anabolic metabolism in adults. Activation of IGF-1R can stimulate cell proliferation, survival, transformation, metastasis, and angiogenesis. In this application, the term "IGF-1R gene" refers to the gene encoding the IGF-1R protein, which encodes a protein with a molecular weight of approximately 320 kDa, composed of two α subunits and two β subunits. The IGF-1R gene may be regulated or inhibited by an RNA inhibitor; in some embodiments, the RNA inhibitor can regulate or inhibit the mRNA sequence of the IGF-1R gene (NM_000875.5 Homo sapiens insulin-like growth factor 1 receptor). The transcriptional sequence of the IGF-1R gene is as shown in SEQ ID NO: 828.

In this application, the term "blocking group" refers to a group that can selectively bind to the duplex siRNA described herein or to a pharmaceutical composition comprising the duplex siRNA, wherein the blocking group is located on the sense strand or sense nucleic acid fragment, the antisense strand or antisense nucleic acid fragment, or on both strands. The blocking group may be attached to the 3' end, the 5' end, or both ends of the sense strand or sense nucleic acid fragment, the antisense strand or antisense nucleic acid fragment, or both strands. In certain embodiments, the blocking group is attached to the antisense strand or antisense nucleic acid fragment, particularly to the 5' end of the antisense strand or antisense nucleic acid fragment. In certain embodiments, the blocking group is attached to an oligonucleotide (e.g., the 5' end of the antisense strand or antisense nucleic acid fragment) via an internucleotide linkage, and the internucleotide linkage is optionally modified as described above. In certain embodiments, the blocking group is linked to the double-stranded oligonucleotide agent via a phosphorothioate linkage. In certain embodiments, the blocking group is linked to the double-stranded oligonucleotide agent via a phosphodiester linkage. In this application, to reduce or inhibit degradation by exonucleases, a "blocking group" may be attached to the 5' end of the antisense strand or antisense nucleic acid fragment of the duplex siRNA described herein. In certain embodiments, the blocking group may reduce or inhibit the RNA interference effect of the oligonucleotide. In certain embodiments, the blocking group is removed from the oligonucleotide prior to exerting the RNA interference effect. For example, examples of blocking groups include, but are not limited to, abasic residues, inverted abasic residues, and (M06).

In this application, the term "ligand" generally refers to any compound or molecule capable of covalently or otherwise chemically binding to a biologically active substance (e.g., an oligonucleotide). In some embodiments, a ligand can interact directly or indirectly with another compound, such as a receptor; the receptor interacting with the ligand may be present on the cell surface, or alternatively may be intracellular and/or intercellular, and the interaction between the ligand and the receptor may lead to a biochemical reaction or may merely be a physical interaction or binding.

In this application, "conjugation" refers to the covalent linkage of two or more chemical moieties, each having a specific function; accordingly, a "conjugate" refers to the compound formed by covalently linking these individual chemical moieties. For example, a "double-stranded ribonucleic acid conjugate" refers to a compound or complex formed by covalently attaching one or more chemical moieties having specific functions (e.g., a delivery system, ligand group, or conjugation group) to double-stranded ribonucleic acid. In some embodiments, the delivery system, ligand group, or conjugation group may be attached to the phosphate group, sugar ring (including covalent attachment via a phosphodiester bond to the atom at the 3' or 5' position of the nucleotide), 2'-hydroxyl group, 5'-hydroxyl group, or base of any nucleotide in the double-stranded ribonucleic acid. In some embodiments, the delivery system, ligand group, or conjugation group may also be attached to the 2' position of the nucleotide, in which case the nucleotides are linked via a 2'-5' phosphodiester bond. In some embodiments, the delivery system, ligand group, or conjugation group may also be attached to the 3' position of the nucleotide, in which case the nucleotides are linked via a 3'-5' phosphodiester bond. In some embodiments, the linkage between the aforementioned delivery system, ligand group, or conjugation group and the nucleotide may be further modified, e.g., by phosphorothioate modification (i.e., linkage via a phosphorothioate diester bond).

In this application, terms such as "induce," "inhibit," "enhance," "elevate," "increase," "reduce," and "decrease" generally denote quantitative differences between two states. For example, an "amount effective to inhibit the activity or gene expression of IGF-1R" means that the level of IGF-1R activity or gene expression in a treated sample will be lower than that in an untreated sample. These terms apply, for example, to levels of gene expression and activity. The terms "reduce" and "decrease" are used interchangeably and generally refer to any change that is less than the original. "Reduce" and "decrease" are relative terms requiring comparison between measurements taken before and after. "Reduce" and "decrease" include complete depletion.

In certain embodiments, the term "decrease" refers to an overall reduction of approximately 5%, 10%, 20%, 25%, 30%, 40%, 50%, 60%, 70%, 80%, 85%, 90%, 95%, or 100% in the level/amount of gene expression of a gene, gene product (e.g., protein), or biomarker in a first sample compared to the corresponding level/amount of gene expression of the gene, gene product (e.g., protein), or biomarker in a second sample, as detectable by standard methods known in the art (such as those described herein). In certain embodiments, the term "decrease" refers to a reduction in the level/amount of gene expression of a gene or biomarker in a first sample, wherein the reduction is at least approximately 0.9-fold, 0.8-fold, 0.7-fold, 0.6-fold, 0.5-fold, 0.4-fold, 0.3-fold, 0.2-fold, 0.1-fold, 0.05-fold, or 0.01-fold of the level/amount of gene expression of the corresponding gene or biomarker in a second sample. In certain embodiments, the first sample is obtained from a subject, while the second sample is a reference sample.

In this application, the term "gene expression" generally refers to the process by which a gene ultimately produces a protein. Gene expression includes, but is not limited to, transcription, post-transcriptional modifications (e.g., splicing, polyadenylation, addition of a 5' cap), and translation.

In this application, the term "pharmaceutically acceptable" generally refers to one or more non-toxic substances that do not interfere with the efficacy of the biological activity of the active ingredient. Such formulations typically may contain salts, excipients, buffers, preservatives, compatible carriers, and optionally other therapeutic agents. Such pharmaceutically acceptable formulations typically may also contain compatible solid or liquid fillers, diluents, or encapsulating materials suitable for administration to humans. When used in medicine, salts should be pharmaceutically acceptable salts, but non-pharmaceutically acceptable salts may conveniently be used to prepare pharmaceutically acceptable salts and should not be excluded from the scope of this application. Such pharmacologically and pharmaceutically acceptable salts include, but are not limited to, salts prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. Pharmaceutically acceptable salts may also be prepared as alkali metal or alkaline earth metal salts, such as sodium, potassium, or calcium salts.

In this application, the term "prevention and/or treatment" not only includes prevention and/or treatment of a disease, but also generally encompasses preventing the onset of the disease, slowing or reversing disease progression, preventing or delaying the onset of one or more symptoms associated with the disease, reducing and/or alleviating one or more symptoms associated with the disease, decreasing the severity and/or duration of the disease and/or any symptoms associated therewith, and/or preventing further increases in the severity of the disease and/or any associated symptoms, preventing, reducing, or reversing any physiological damage caused by the disease, and generally any pharmacological effect beneficial to the patient being treated. The RNAi agent or pharmaceutical composition of this application does not need to achieve a complete cure or eradication of any symptom or manifestation of the disease to constitute a viable therapeutic agent. As recognized in the relevant field, a drug used as a therapeutic agent may reduce the severity of a given disease state without necessarily eliminating every manifestation of the disease to be considered a useful therapeutic agent. Similarly, prophylactic administration of a therapeutic constitutes a viable preventive agent even if it is not completely effective in preventing the onset of the condition. It is sufficient simply to reduce the impact of the disease in a subject (e.g., by reducing the number or severity of its symptoms, enhancing the effectiveness of another therapy, or producing another beneficial effect) or to reduce the likelihood of the disease occurring or worsening.

In this application, the terms "disease" or "disorder" are used interchangeably and generally refer to any deviation of a subject from a normal state, such as any alteration in the state of the body or certain organs that impairs or disrupts the performance of functions, and/or causes symptoms such as discomfort, dysfunction, distress, or even death in the affected individual or those in contact with them. Disease or disorder may also be referred to as distemper, ailment, malady, disorder, sickness, illness, or complaint.

In this application, the term "administration" generally refers to the introduction or delivery of the pharmaceutical formulation described herein into the body of a subject by any route of introduction or delivery. Any method known to those skilled in the art for contacting cells, organs, or tissues with said drug may be employed. The administration may include, but is not limited to, intravenous, intra-arterial, intranasal, intraperitoneal, intramuscular, subcutaneous, transdermal, or oral administration. The daily dose may be divided into one, two, or more doses of a suitable form for administration at one, two, or more times during a period of time.

In the present application, the term "contact" generally refers to two or more substances of different types being brought into contact with each other in any order, by any means, and for any duration. Contact may occur in vivo, ex vivo, or in vitro. In certain embodiments, it may refer to directly contacting cells or tissues with the RNAi agent or composition of the present application. In other embodiments, the term refers to indirectly contacting cells or tissues with the RNA inhibitor or composition of the present application. For example, the methods of the present application include methods wherein a subject is contacted with the RNA inhibitor or composition of the present application, and then the RNA inhibitor or composition contacts cells or tissues via diffusion or any other active or passive transport process known in the art (through which the compound circulates in vivo).

In the present application, the terms "effective amount" or "effective dose" generally refer to an amount sufficient to achieve or at least partially achieve the desired effect. A "therapeutically effective amount" or "therapeutically effective dose" of a drug generally refers to any amount of the drug that, when used alone or in combination with another therapeutic agent, promotes disease regression (as demonstrated by a reduction in the severity of disease symptoms, an increase in the frequency and duration of symptom-free periods of the disease, or prevention of damage or disability caused by the disease). A "prophylactically effective amount" or "prophylactically effective dose" of a drug generally refers to an amount of the drug that, when administered alone or in combination with another therapeutic agent to a subject at risk of developing or experiencing recurrence of a disease, inhibits the development or recurrence of the disease. The ability of a therapeutic or prophylactic agent to promote disease regression or inhibit disease development or recurrence can be evaluated using various methods known to those skilled in the art, such as in human subjects during clinical trials, in animal model systems predictive of efficacy in humans, or by measuring the activity of the agent in in vitro assays. In certain embodiments, an "effective amount" refers to the amount of RNA inhibitor that produces the intended pharmacological, therapeutic, or prophylactic outcome.

In the present application, the term "subject" generally refers to a human or non-human animal (including mammals) requiring diagnosis, prognosis, amelioration, prevention, and/or treatment of a disease, such as humans, non-human primates (apes, gibbons, gorillas, chimpanzees, orangutans, macaques), companion animals (dogs and cats), farm animals (poultry such as chickens, ducks, horses, cattle, goats, sheep, pigs), and laboratory animals (mice, rats, rabbits, guinea pigs). Human subjects include fetal, neonatal, infant, adolescent, and adult subjects. Subjects include animal disease models.

In the present application, the terms "comprising," "including," "having," "may," "containing," and their variants are generally intended to be open-ended transitional phrases, terms, or words that do not exclude the possibility of additional acts or structures. The term "consisting of" generally indicates that no other components (or, similarly, features, integers, steps, etc.) may be present. Unless the context clearly dictates otherwise, singular forms such as "a," "an," and "the" in English, and "a," "a kind of," and "said/the" in Chinese, generally include the plural forms of the referenced entities.

In the present application, the term "about" generally means large, roughly, or around. When the term "about" is used with reference to a numerical range, the stated endpoint or specific value indicates that the specified value may differ from the listed value by up to 10%. Thus, the term "about" may encompass variations of ±10% or less, ±5% or less, ±1% or less, ±0.5% or less, or ±0.1% or less from a specific value.

It should be understood that the term "at least" preceding a number or a series of numbers includes the number adjacent to the term "at least" and all subsequent numbers or integers logically included, as made clear from the context. For example, the number of nucleotides in a nucleic acid molecule must be an integer. For example, "at least 19 nucleotides in a 21-nucleotide nucleic acid molecule" means that 19, 20, or 21 nucleotides possess the indicated property. When "at least" precedes a series of numbers or a range, it should be understood that "at least" modifies each number in the series or range.

It should be understood that "not more than" or "less than" as used herein refers to values or integers adjacent to and logically lower than the phrase, down to zero, as logically inferred from the context. For example, a duplex having an overhang of "not more than 3 nucleotides" has an overhang of 3, 2, 1, or 0 nucleotides. When "not more than" precedes a series of numbers or a range, it should be understood that "not more than" modifies each number in the series or range. Ranges used herein include both the upper and lower limits.

### Detailed Description of the Invention

Antisense strand or antisense nucleic acid fragment and sense strand or sense nucleic acid fragment

In one aspect, the present application provides an RNA inhibitor for inhibiting the expression of the insulin-like growth factor 1 receptor (IGF-1R) gene, comprising an antisense strand that forms a complementary region with at least 15 consecutive nucleotides of the sequence encoding IGF-1R (SEQ ID NO: 828), wherein the complementary region has 0, 1, 2, 3, 4, or 5 mismatches; preferably, the duplex complementary region is 15-30 nucleotide pairs in length, more preferably 17-23 nucleotide pairs. For example, the length of the duplex complementary region may be 17, 18, 19, 20, 21, 22, or 23 nucleotide pairs.

In certain embodiments, the RNA inhibitor comprises a single-stranded oligonucleotide or a double-stranded ribonucleic acid (dsRNA) molecule for inhibiting IGF-1R gene expression in cells, such as cells of a subject (e.g., a mammal). Wherein the dsRNA comprises an antisense strand or antisense nucleic acid fragment having a complementary region complementary to at least a portion of the mRNA formed during expression of the IGF-1R gene. The complementary region is approximately 12-30 nucleotides in length (e.g., approximately 30, 29, 28, 27, 26, 25, 24, 23, 22, 21, 20, 19, 18, 17, 16, 15, 14, 13, or 12 nucleotides in length).

The dsRNA comprises two RNA strands that are complementary and hybridize under conditions of dsRNA use to form a duplex structure (complementary region). One strand of the dsRNA (antisense strand or antisense nucleic acid fragment) comprises a complementary region that is substantially complementary and typically fully complementary. It may be derived from the sequence of the mRNA formed during IGF-1R gene expression. The other strand (sense strand or sense nucleic acid fragment) comprises a region complementary to the antisense strand or antisense nucleic acid fragment, such that when combined under appropriate conditions, the two strands hybridize and form a duplex structure. Typically, the duplex structure is 12 to 30 base pairs in length. Similarly, the length of the complementary region to is 12 to 30 nucleotides, for example, a length between 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20-21, 21-30, 21-29, 21-28, 21-27, 21-26, 21-25, 21-24, 21-23, or 21-22 nucleotides.

In certain embodiments, the dsRNA is approximately 19 to approximately 23 nucleotides in length, or approximately 24 to approximately 30 nucleotides in length. Typically, the dsRNA is long enough to serve as a substrate for Dicer enzyme. For example, it is well known in the art that dsRNA longer than approximately 21-23 nucleotides can serve as a substrate for Dicer. Those skilled in the art also understand that the region of RNA targeted for cleavage is typically a portion of a larger RNA molecule (usually an mRNA molecule). A "portion" of the target refers to a continuous stretch of nucleotides of the mRNA target of sufficient length to allow it to serve as a substrate for RNAi-directed cleavage (i.e., cleavage via the RISC pathway).

Those skilled in the art should also understand that the complementary region is the primary functional part of the dsRNA, for example, a duplex region of approximately 19 to approximately 30 base pairs, e.g., 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20-21, 21-30, 21-29, 21-28, 21-27, 21-26, 21-25, 21-24, 21-23, or 21-22 base pairs.

In certain embodiments, there is at least approximately 80% base complementarity between the sense strand or sense nucleic acid fragment and the antisense strand or antisense nucleic acid fragment.

In certain embodiments, the sense strand or sense nucleic acid fragment and the antisense strand or antisense nucleic acid fragment are each independently 19-30 nucleotides in length.

In certain embodiments, the sense strand or sense nucleic acid fragment and the antisense strand or antisense nucleic acid fragment are each independently 17-25 nucleotides in length.

In certain embodiments, the sense strand or sense nucleic acid fragment and the antisense strand or antisense nucleic acid fragment are each independently 19-23 nucleotides in length.

In some embodiments, the total length of the sense strand is 15-30 nucleotides, preferably 16-23 nucleotides, more preferably 19, 20, or 21 nucleotides.

In some embodiments, the total length of the antisense strand is 19-30 nucleotides, preferably 19-27 nucleotides, more preferably 21, 22, 23, 24, 25, 26, or 27 nucleotides.

In some embodiments, the sense strand or sense nucleic acid fragment is selected from any one of SEQ ID NO: 1-198 or a sequence differing by not more than 3 nucleotides therefrom.

In some embodiments, the sense strand or sense nucleic acid fragment of the RNA inhibitor is selected from sequences differing by one, two, or three nucleotides from the sequences in Table 1, for example, 15, 16, 17, 18, 19, or 20 consecutive nucleotides.

In some embodiments, the antisense strand or antisense nucleic acid fragment is selected from any one of SEQ ID NO: 199-413 or a sequence of at least 15 consecutive nucleotides differing by not more than 3 nucleotides therefrom, for example, 15, 16, 17, 18, 19, or 20 consecutive nucleotides.

In some embodiments, the antisense strand or antisense nucleic acid fragment of the RNA inhibitor is selected from sequences of at least 15 consecutive nucleotides differing by one, two, or three nucleotides from the sequences in Table 1, for example, 15, 16, 17, 18, 19, or 20 consecutive nucleotides.

In some embodiments, the sense strand and antisense strand each independently optionally comprise a 3' or 5' overhang of 1, 2, or 3 nucleotides.

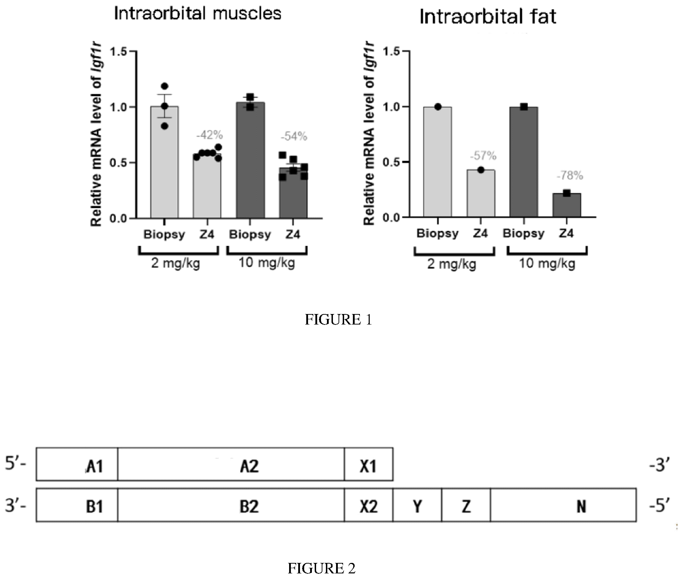

In some embodiments, both the sense strand and antisense strand have a 3' overhang of 1-3 nucleotides in length, or the sense strand has a 3' or 5' overhang of 1-3 nucleotides in length, or the antisense strand has a 3' or 5' overhang of 1-3 nucleotides in length. In some embodiments, the RNA inhibitor of the present application has specific structures near its 5' and 3' termini, as shown in Figure 2.

In certain embodiments, the antisense strand comprises a cleavage region, wherein the cleavage region comprises the nucleotide sequence shown in formula (I),

Formula (I): (3'-5') X2-Y-Z,

wherein cleavage occurs between X2 and Y, X2 is the most 5'-terminal nucleotide of the second strand, Y and Z are the two most 3'-terminal nucleotides of the 5' extension segment, and Z is a guanine nucleotide (G), a natural analog of guanine nucleotide (G), a non-natural analog of guanine nucleotide (G), an adenine nucleotide (A), a natural analog of adenine nucleotide (A), or a non-natural analog of adenine nucleotide (A).

In certain embodiments, Z is a guanine nucleotide (G), a natural analog of guanine nucleotide (G), or a non-natural analog of guanine nucleotide (G).

In certain embodiments, X2 is an adenine nucleotide (A), a natural analog of adenine nucleotide (A), a non-natural analog of adenine nucleotide (A), a uracil nucleotide (U), a natural analog of uracil nucleotide (U), or a non-natural analog of uracil nucleotide (U).

In certain embodiments, formula (I) has a sequence selected from the following (3'-5'): UUG, UAG, AUG, AAG, UUA, UAA, AUA, AAA, UCG, UGG, ACG, AGG, UCA, UGA, ACA, and AGA, or their natural or non-natural analogs.

In certain embodiments, Y is an adenine nucleotide (A), a natural analog of adenine nucleotide (A), a non-natural analog of adenine nucleotide (A), a uracil nucleotide (U), a natural analog of uracil nucleotide (U), or a non-natural analog of uracil nucleotide (U).

In certain embodiments, formula (I) has a sequence selected from the following (3'-5'): UUG, UAG, AUG, AAG, UUA, UAA, AUA, and AAA.

In certain embodiments, the cleavage region further comprises nucleotide N1, wherein N1 is the third nucleotide from the most 3' end of the 5' extension segment, and the cleavage region comprises the nucleotide sequence shown in formula (II),

Formula (II): (3'-5') X2-Y-Z-N1.

In certain embodiments, the cleavage region further comprises segment N, wherein segment N comprises at least one nucleotide, the most 3'-terminal nucleotide of segment N being N1, and the cleavage region comprises the nucleotide sequence shown in formula (III),

Formula (III): (3'-5') X2-Y-Z-N,

wherein segment N is 1-10 nucleotides in length, preferably 1-5 nucleotides, more preferably 1 nucleotide.

In certain embodiments, N is an adenine nucleotide (A), guanine nucleotide (G), cytosine nucleotide (C), uracil nucleotide (U), a natural analog thereof, or a non-natural analog thereof; preferably, N1 is a cytosine nucleotide (C), a natural analog of cytosine nucleotide (C), or a non-natural analog of cytosine nucleotide (C).

In certain embodiments, formula (III) has a sequence selected from the following (3'-5'): AAGC and UAGC.

In some embodiments, the sense strand or sense nucleic acid fragment and antisense strand of the RNA inhibitor are selected from sequences of at least 15 consecutive nucleotides differing by one, two, or three nucleotides from the sequences in Table 1, for example, 15, 16, 17, 18, 19, or 20 consecutive nucleotides.

**Table 1 siRNA Sense Strand and Antisense Strand Sequences**

| **Naked Duplex Sequence Number** | **Naked Sense Strand (5'→3')** | **Number** | **Naked Antisense Strand (5'→3')** | **Number** | **NM_0008 75.5 Position** |
|---|---|---|---|---|---|
| ds-n1 | CUGGCCGACGAGUGGA GAAAU | SEQ ID NO: 1 | AUUUCUCCACUCGUCGGCCA G | SEQ ID NO: 199 | 1118 |
| ds-n2 | GGCCGACGAGUGGAGA AAUCU | SEQ ID NO: 2 | AGAUUUCUCCACUCGUCGGC C | SEQ ID NO: 200 | 1120 |
| ds-n3 | CGACAUCCGCAACGAC UAUCA | SEQ ID NO: 3 | UGAUAGUCGUUGCGGAUGUC G | SEQ ID NO: 201 | 1154 |
| ds-n4 | CUGGAAACUCUUCUAC AACUA | SEQ ID NO: 4 | UAGUUGUAGAAGAGUUUCCA G | SEQ ID NO: 202 | 1367 |
| ds-n5 | AUGACCAAUCUCAAGG AUAUU | SEQ ID NO: 5 | AAUAUCCUUGAGAUUGGUCA U | SEQ ID NO: 203 | 1407 |
| ds-n6 | UCUCAAGGAUAUUGGG CUUUA | SEQ ID NO: 6 | UAAAGCCCAAUAUCCUUGAG A | SEQ ID NO: 204 | 1415 |
| ds-n7 | GGUGUCCAAUAACUAC AUUGU | SEQ ID NO: 7 | ACAAUGUAGUUAUUGGACAC C | SEQ ID NO: 205 | 1532 |
| ds-n8 | GACCACCAUCAACAAU GAGUA | SEQ ID NO: 8 | UACUCAUUGUUGAUGGUGGU C | SEQ ID NO: 206 | 1625 |
| ds-n9 | CCACCAUCAACAAUGA GUACA | SEQ ID NO: 9 | UGUACUCAUUGUUGAUGGUG G | SEQ ID NO: 207 | 1627 |
| ds-n10 | CACCAUCAACAAUGAG UACAA | SEQ ID NO: 10 | UUGUACUCAUUGUUGAUGGU G | SEQ ID NO: 208 | 1628 |
| ds-n11 | CAUCAACAAUGAGUAC AACUA | SEQ ID NO: 11 | UAGUUGUACUCAUUGUUGAU G | SEQ ID NO: 209 | 1631 |
| ds-n12 | GGAGACAGAGUACCCU UUCUU | SEQ ID NO: 12 | AAGAAAGGGUACUCUGUCUC C | SEQ ID NO: 210 | 3356 |
| ds-n13 | GAGACAGAGUACCCUU UCUUU | SEQ ID NO: 13 | AAAGAAAGGUACUCUGUCU C | SEQ ID NO: 211 | 3357 |
| ds-n14 | GACAGAGUACCCUUUC UUUGA | SEQ ID NO: 14 | UCAAAGAAAGGUACUCUGU C | SEQ ID NO: 212 | 3359 |
| ds-n15 | ACAGAUCCUGUGUUCU UCUAU | SEQ ID NO: 15 | AUAGAAGAACACAGGAUCUG U | SEQ ID NO: 213 | 3792 |
| ds-n16 | CUGUGAUGAAGGAGUU CAAUU | SEQ ID NO: 16 | AAUUGAACUCCUUCAUCACA G | SEQ ID NO: 214 | 4198 |
| ds-n17 | GUGAUGAAGGAGUUCA AUUGU | SEQ ID NO: 17 | ACAAUUGAACUCCUUCAUCA C | SEQ ID NO: 215 | 4200 |
| ds-n18 | GGAGUUCAAUUGUCAC CAUGU | SEQ ID NO: 18 | ACAUGGUGACAAUUGAACUC C | SEQ ID NO: 216 | 4208 |
| ds-n19 | GGGCUUGUCCAACGAG CAAGU | SEQ ID NO: 19 | ACUUGCUCGUUGGACAAGCC C | SEQ ID NO: 217 | 4685 |
| ds-n20 | UACCUCAGUGGAUCUU CAGAA | SEQ ID NO: 20 | UUCUGAAGAUCCACUGAGGU A | SEQ ID NO: 218 | 5246 |
| ds-n21 | ACCUUAAUGACAACAC UUAAU | SEQ ID NO: 21 | AUUAAGUGUUGUCAUUAAGG U | SEQ ID NO: 219 | 5392 |
| ds-n22 | CCUUAAUGACAACACU UAAUA | SEQ ID NO: 22 | UAUUAAGUGUUGUCAUUAAG G | SEQ ID NO: 220 | 5393 |
| ds-n23 | AUUUGCAUACACAUCG UCUUU | SEQ ID NO: 23 | AAAGACGAUGUGUAUGCAAA U | SEQ ID NO: 221 | 6329 |
| ds-n24 | UGCAUACACAUCGUCU UUAAU | SEQ ID NO: 24 | AUUAAAGACGAUGUGUAUGC A | SEQ ID NO: 222 | 6332 |
| ds-n25 | GCAUACACAUCGUCUU UAAUG | SEQ ID NO: 25 | CAUUAAAGACGAUGUGUAUG C | SEQ ID NO: 223 | 6333 |
| ds-n26 | CAUACACAUCGUCUUU AAUGU | SEQ ID NO: 26 | ACAUUAAAGACGAUGUGUAU G | SEQ ID NO: 224 | 6334 |
| ds-n27 | GCCAGAGAUGCUGAAG AUACA | SEQ ID NO: 27 | UGUAUCUUCAGCAUCUCUGG C | SEQ ID NO: 225 | 10215 |
| ds-n28 | GUCCAGUAGAUUACGG GUAGU | SEQ ID NO: 28 | ACUACCCGUAAUCUACUGGA C | SEQ ID NO: 226 | 10738 |
| ds-n29 | CCAGUAGAUUACGGGU AGUCA | SEQ ID NO: 29 | UGACUACCCGUAAUCUACUG G | SEQ ID NO: 227 | 10740 |
| ds-n30 | GAUCUGGUUUACAAGA ACUAA | SEQ ID NO: 30 | UUAGUUCUUGUAAACCAGAU C | SEQ ID NO: 228 | 10770 |
| ds-n31 | UCUGGUUUACAAGAAC UAAUU | SEQ ID NO: 31 | AAUUAGUUCUUGUAAACCAG A | SEQ ID NO: 229 | 10772 |
| ds-n32 | CUGGUUUACAAGAACU AAUUA | SEQ ID NO: 32 | UAAUUAGUUCUUGUAAACCA G | SEQ ID NO: 230 | 10773 |
| ds-n33 | GUUUACUAGCUAGCUU UACAA | SEQ ID NO: 33 | UUGUAAAGCUAGCUAGUAAA C | SEQ ID NO: 231 | 10946 |
| ds-n34 | UAGCUUUACAAUAUGC CAAAA | SEQ ID NO: 34 | UUUUGGCAUAUUGUAAAGCU A | SEQ ID NO: 232 | 10956 |
| ds-n35 | AGCUUUACAAUAUGCC AAAAA | SEQ ID NO: 35 | UUUUUGGCAUAUUGUAAAGC U | SEQ ID NO: 233 | 10957 |
| ds-n36 | CAAGGAUAUUGGGCUU UACAA | SEQ ID NO: 36 | UUGUAAAGCCCAAUAUCCUU G | SEQ ID NO: 234 | 1418 |
| ds-n37 | UAACUACAUUGUGGGG AAUAA | SEQ ID NO: 37 | UUAUUCCCCACAAUGUAGUU A | SEQ ID NO: 235 | 1541 |
| ds-n38 | AGAAAACAAAGACCAU UGAUU | SEQ ID NO: 38 | AAUCAAUGGUCUUUGUUUC U | SEQ ID NO: 236 | 2050 |
| ds-n39 | ACAAAGACCAUUGAUU CUGUU | SEQ ID NO: 39 | AACAGAAUCAAUGGUCUUUG U | SEQ ID NO: 237 | 2055 |
| ds-n40 | CAAAGACCAUUGAUUC UGUUA | SEQ ID NO: 40 | UAACAGAAUCAAUGGUCUUU G | SEQ ID NO: 238 | 2056 |
| ds-n41 | UCUCCUUGUCCUUCCU AAAAA | SEQ ID NO: 41 | UUUUUAGGAAGGACAAGGAG A | SEQ ID NO: 239 | 2233 |
| ds-n42 | CUCCUUGUCCUUCCUA AAAAA | SEQ ID NO: 42 | UUUUUUAGGAAGGACAAGGA G | SEQ ID NO: 240 | 2234 |
| ds-n43 | CCAAAGCAAAGGGAC AUAAA | SEQ ID NO: 43 | UUUAUGUCCCCUUUGCUUUG G | SEQ ID NO: 241 | 2456 |
| ds-n44 | CUACAGGGAUCUCAUC AGCUU | SEQ ID NO: 44 | AAGCUGAUGAGAUCCCUGUA G | SEQ ID NO: 242 | 2591 |
| ds-n45 | CAUCAGCUUCACCGUU UACUA | SEQ ID NO: 45 | UAGUAAACGGUGAAGCUGAU G | SEQ ID NO: 243 | 2603 |
| ds-n46 | CAGCUUCACCGUUUAC UACAA | SEQ ID NO: 46 | UUGUAGUAAACGGUGAAGCU G | SEQ ID NO: 244 | 2606 |
| ds-n47 | CUUCACCGUUUACUAC AAGGA | SEQ ID NO: 47 | UCCUUGUAGUAAACGGUGAA G | SEQ ID NO: 245 | 2609 |
| ds-n48 | CCGUUUACGUCAAGGC UGUGA | SEQ ID NO: 48 | UCACAGCCUUGACGUAAACG G | SEQ ID NO: 246 | 2776 |
| ds-n49 | ACGUUCUUUCAGCAUC GAACU | SEQ ID NO: 49 | AGUUCGAUGCUGAAAGAACG U | SEQ ID NO: 247 | 2890 |
| ds-n50 | GAACUCCUCUUCUCAG UUAAU | SEQ ID NO: 50 | AUUAACUGAGAAGAGGAGUU C | SEQ ID NO: 248 | 2906 |
| ds-n51 | CUCCUCUUCUCAGUUA AUCGU | SEQ ID NO: 51 | ACGAUUAACUGAGAAGAGGA G | SEQ ID NO: 249 | 2909 |
| ds-n52 | CGGCAACCUGAGUUAC UACAU | SEQ ID NO: 52 | AUGUAGUAACUCAGGUUGCC G | SEQ ID NO: 250 | 2957 |
| ds-n53 | CAACCUGAGUUACUAC AUUGU | SEQ ID NO: 53 | ACAAUGUAGUAACUCAGGUU G | SEQ ID NO: 251 | 2960 |
| ds-n54 | CUACCUUUACCGGCAC AAUUA | SEQ ID NO: 54 | UAAUUGUGCCGGUAAAGGUA G | SEQ ID NO: 252 | 3008 |
| ds-n55 | UACCGCAAAGUCUUUG AGAAU | SEQ ID NO: 55 | AUUCUCAAAGACUUUGCGGU A | SEQ ID NO: 253 | 3195 |
| ds-n56 | GGAGAGAACUGUCAUU UCUAA | SEQ ID NO: 56 | UUAGAAAUGACAGUUCUCUC C | SEQ ID NO: 254 | 3398 |
| ds-n57 | UGAGAAUCCCAAUGGA UUGAU | SEQ ID NO: 57 | AUCAAUCCAUUGGGAUUCUC A | SEQ ID NO: 255 | 3608 |
| ds-n58 | GAGAAUCCCAAUGGAU UGAUU | SEQ ID NO: 58 | AAUCAAUCCAUUGGGAUUCU C | SEQ ID NO: 256 | 3609 |
| ds-n59 | CGGAUCACAAGUUGAG GAUCA | SEQ ID NO: 59 | UGAUCCUCAACUUGUGAUCC G | SEQ ID NO: 257 | 3650 |
| ds-n60 | GUUGAGGAUCAGCGAG AAUGU | SEQ ID NO: 60 | ACAUUCUCGCUGAUCCUCAA C | SEQ ID NO: 258 | 3660 |
| ds-n61 | CCAGGCCAAAACAGGA UAUGA | SEQ ID NO: 61 | UCAUAUCCUGUUUUGGCCUG G | SEQ ID NO: 259 | 3815 |
| ds-n62 | AUCGUGGGAGGGUUGG UGAUU | SEQ ID NO: 62 | AAUCACCAACCCUCCCACGA U | SEQ ID NO: 260 | 3882 |
| ds-n63 | GAUGUGUACGUUCCUG AUGAG | SEQ ID NO: 63 | CUCAUCAGGAACGUACACAU C | SEQ ID NO: 261 | 3996 |
| ds-n64 | GUCGUUUGGAUGGUC UAUGA | SEQ ID NO: 64 | UCAUAGACCAUCCCAAACGA C | SEQ ID NO: 262 | 4067 |
| ds-n65 | UGAAACCAGAGUGGCC AUUAA | SEQ ID NO: 65 | UUAAUGGCCACUCUGGUUUC A | SEQ ID NO: 263 | 4121 |
| ds-n66 | GAAACCAGAGUGGCCA UUAAA | SEQ ID NO: 66 | UUUAAUGGCCACUCUGGUUU C | SEQ ID NO: 264 | 4122 |
| ds-n67 | ACGGGGCGAUCUCAAA AGUUA | SEQ ID NO: 67 | UAACUUUUGAGAUCGCCCCG U | SEQ ID NO: 265 | 4292 |
| ds-n68 | CGGGGCGAUCUCAAAA GUUAU | SEQ ID NO: 68 | AUAACUUUUGAGAUCGCCCC G | SEQ ID NO: 266 | 4293 |
| ds-n69 | GGGCGAUCUCAAAAGU UAUCU | SEQ ID NO: 69 | AGAUAACUUUUGAGAUCGCC C | SEQ ID NO: 267 | 4295 |
| ds-n70 | GAGAUAUCUAUGAGAC AGACU | SEQ ID NO: 70 | AGUCUGUCUCAUAGAUAUCU C | SEQ ID NO: 268 | 4516 |
| ds-n71 | AUAUCUAUGAGACAGA CUAUU | SEQ ID NO: 71 | AAUAGUCUGUCUCAUAGAUA U | SEQ ID NO: 269 | 4519 |
| ds-n72 | UCUGUGCAAACAGUAA CGUGU | SEQ ID NO: 72 | ACACGUUACUGUUUGCACAG A | SEQ ID NO: 270 | 5163 |
| ds-n73 | CACAUUUGGAUGUUC CUUUU | SEQ ID NO: 73 | AAAAGGAACAUCCCAAAUGU G | SEQ ID NO: 271 | 5309 |
| ds-n74 | ACAUUUGGAUGUUCC UUUUU | SEQ ID NO: 74 | AAAAAGGAACAUCCCAAAUG U | SEQ ID NO: 272 | 5310 |
| ds-n75 | CAUGGGCCUUUAAGAA CCUUA | SEQ ID NO: 75 | UAAGGUUCUUAAAGGCCCAU G | SEQ ID NO: 273 | 5377 |
| ds-n76 | GGGCCUUUAAGAACCU UAAUG | SEQ ID NO: 76 | CAUUAAGGUUCUUAAAGGCC C | SEQ ID NO: 274 | 5380 |
| ds-n77 | GGCCUUUAAGAACCUU AAUGA | SEQ ID NO: 77 | UCAUUAAGGUUCUUAAAGGC C | SEQ ID NO: 275 | 5381 |
| ds-n78 | UCUAGGGACAUGAAAU UUACA | SEQ ID NO: 78 | UGUAAAUUUCAUGUCCCUAG A | SEQ ID NO: 276 | 5663 |
| ds-n79 | CCAAGGCUGUUACCAU UUUAA | SEQ ID NO: 79 | UUAAAAUGGUAACAGCCUUG G | SEQ ID NO: 277 | 5700 |
| ds-n80 | ACCAUUUUAACGCUGC CUAAU | SEQ ID NO: 80 | AUUAGGCAGCGUUAAAAUGG U | SEQ ID NO: 278 | 5711 |
| ds-n81 | CCAUUUUAACGCUGCC UAAUU | SEQ ID NO: 81 | AAUUAGGCAGCGUUAAAAUG G | SEQ ID NO: 279 | 5712 |
| ds-n82 | CGCUGCCUAAUUUUGC CAAAA | SEQ ID NO: 82 | UUUUGGCAAAAUUAGGCAGC G | SEQ ID NO: 280 | 5721 |
| ds-n83 | CAGGUCUCAUUGCUUC UGACU | SEQ ID NO: 83 | AGUCAGAAGCAAUGAGACCU G | SEQ ID NO: 281 | 5896 |
| ds-n84 | GGUCUCAUUGCUUCUG ACUAG | SEQ ID NO: 84 | CUAGUCAGAAGCAAUGAGAC C | SEQ ID NO: 282 | 5898 |
| ds-n85 | CAAACACCACAACAGC AGUAA | SEQ ID NO: 85 | UUACUGCUGUUGUGGUGUUU G | SEQ ID NO: 283 | 6203 |
| ds-n86 | UCAAUGGAUUCAAGCA UUCUA | SEQ ID NO: 86 | UAGAAUGCUUGAAUCCAUUG A | SEQ ID NO: 284 | 6236 |
| ds-n87 | CAAUGGAUUCAAGCAU UCUAA | SEQ ID NO: 87 | UUAGAAUGCUUGAAUCCAUU G | SEQ ID NO: 285 | 6237 |
| ds-n88 | UGGAUUCAAGCAUUCU AAGCU | SEQ ID NO: 88 | AGCUUAGAAUGCUUGAAUCC A | SEQ ID NO: 286 | 6240 |
| ds-n89 | UCAAGCAUUCUAAGCU UUGUU | SEQ ID NO: 89 | AACAAAGCUUAGAAUGCUUG A | SEQ ID NO: 287 | 6245 |
| ds-n90 | CUUCUUCAUGGGUCUU ACAGU | SEQ ID NO: 90 | ACUGUAAGACCCAUGAAGAA G | SEQ ID NO: 288 | 6288 |
| ds-n91 | GUCUUACAGUUCUAUG UUAGA | SEQ ID NO: 91 | UCUAACAUAGAACUGUAAGA C | SEQ ID NO: 289 | 6299 |
| ds-n92 | CAGUUCUAUGUUAGAC CAUGA | SEQ ID NO: 92 | UCAUGGUCUAACAUAGAACU G | SEQ ID NO: 290 | 6305 |
| ds-n93 | UCUAUGUUAGACCAUG AAACA | SEQ ID NO: 93 | UGUUUCAUGGUCUAACAUAG A | SEQ ID NO: 291 | 6309 |
| ds-n94 | CUAUGUUAGACCAUGA AACAU | SEQ ID NO: 94 | AUGUUUCAUGGUCUAACAUA G | SEQ ID NO: 292 | 6310 |
| ds-n95 | CGUCUUUAAUGUCACU UUUAU | SEQ ID NO: 95 | AUAAAAGUGACAUUAAAGAC G | SEQ ID NO: 293 | 6343 |
| ds-n96 | UAACUUUUUACGGUU CAGAU | SEQ ID NO: 96 | AUCUGAACCGUAAAAAGUU A | SEQ ID NO: 294 | 6363 |
| ds-n97 | ACUUUUUUACGGUUCA GAUAU | SEQ ID NO: 97 | AUAUCUGAACCGUAAAAAG U | SEQ ID NO: 295 | 6365 |
| ds-n98 | CUUUUUUACGGUUCAG AUAUU | SEQ ID NO: 98 | AAUAUCUGAACCGUAAAAAA G | SEQ ID NO: 296 | 6366 |
| ds-n99 | ACGGUUCAGAUAUUCA UCUAU | SEQ ID NO: 99 | AUAGAUGAAUAUCUGAACCG U | SEQ ID NO: 297 | 6373 |
| ds-n100 | CAGAUAUUCAUCUAUA CGUCU | SEQ ID NO: 100 | AGACGUAUAGAUGAAUAUCU G | SEQ ID NO: 298 | 6379 |
| ds-n101 | GAUAUUCAUCUAUACG UCUGU | SEQ ID NO: 101 | ACAGACGUAUAGAUGAAUAU C | SEQ ID NO: 299 | 6381 |
| ds-n102 | GUUCUUGAUCUUUGUG GAUUU | SEQ ID NO: 102 | AAAUCCACAAAGAUCAAGAA C | SEQ ID NO: 300 | 6431 |
| ds-n103 | CUUGAUCUUUGUGGAU UUAAU | SEQ ID NO: 103 | AUUAAAUCCACAAAGAUCAA G | SEQ ID NO: 301 | 6434 |
| ds-n104 | CUCCAAGAAACUUCUU AUGCU | SEQ ID NO: 104 | AGCAUAAGAAGUUUCUUGGA G | SEQ ID NO: 302 | 6496 |
| ds-n105 | UGUACUAGAGUGCGUG ACUUU | SEQ ID NO: 105 | AAAGUCACGCACUCUAGUAC A | SEQ ID NO: 303 | 6518 |
| ds-n106 | CUGUUACAGUGCAAGA CAUGA | SEQ ID NO: 106 | UCAUGUCUUGCACUGUAACA G | SEQ ID NO: 304 | 6755 |
| ds-n107 | ACAGUGCAAGACAUGA UACAA | SEQ ID NO: 107 | UUGUAUCAUGUCUUGCACUG U | SEQ ID NO: 305 | 6760 |
| ds-n108 | CUUAGGUUGUGACACA CAUAU | SEQ ID NO: 108 | AUAUGUGUGUCACAACCUAA G | SEQ ID NO: 306 | 6883 |
| ds-n109 | UAGGUUGUGACACACA UAUAU | SEQ ID NO: 109 | AUAUAUGUGUGUCACAACCU A | SEQ ID NO: 307 | 6885 |
| ds-n110 | GCAGACACUAGGCAUU UGGAU | SEQ ID NO: 110 | AUCCAAAUGCCUAGUGUCUG C | SEQ ID NO: 308 | 6947 |
| ds-n111 | CAGACACUAGGCAUUU GGAUU | SEQ ID NO: 111 | AAUCCAAAUGCCUAGUGUCU G | SEQ ID NO: 309 | 6948 |
| ds-n112 | AGACACUAGGCAUUUG GAUUA | SEQ ID NO: 112 | UAAUCCAAAUGCCUAGUGUC U | SEQ ID NO: 310 | 6949 |
| ds-n113 | CACUAGGCAUUUGGAU UACUA | SEQ ID NO: 113 | UAGUAAUCCAAAUGCCUAGU G | SEQ ID NO: 311 | 6952 |
| ds-n114 | ACUAGGCAUUUGGAUU ACUAU | SEQ ID NO: 114 | AUAGUAAUCCAAAUGCCUAG U | SEQ ID NO: 312 | 6953 |
| ds-n115 | CUAGGCAUUUGGAUUA CUAUU | SEQ ID NO: 115 | AAUAGUAAUCCAAAUGCCUA G | SEQ ID NO: 313 | 6954 |
| ds-n116 | GGUAUUAUAUGUAGGA GUUUU | SEQ ID NO: 116 | AAAACUCCUACAUAUAAUAC C | SEQ ID NO: 314 | 7574 |
| ds-n117 | CCAGUUUCAAUCACUG UAGAA | SEQ ID NO: 117 | UUCUACAGUGAUUGAAACUG G | SEQ ID NO: 315 | 7621 |
| ds-n118 | AUCUACCUCACUCUUA UUUUU | SEQ ID NO: 118 | AAAAAUAAGAGUGAGGUAG AU | SEQ ID NO: 316 | 7764 |
| ds-n119 | CCUGACAAUAGGCCGU UGAUA | SEQ ID NO: 119 | UAUCAACGGCCUAUUGUCAG G | SEQ ID NO: 317 | 7830 |
| ds-n120 | AGGCUGUAUUCCGGGG UCAAA | SEQ ID NO: 120 | UUUGACCCCGGAAUACAGCC U | SEQ ID NO: 318 | 7906 |
| ds-n121 | GGUAGACAGAGAUGUA CCAAA | SEQ ID NO: 121 | UUUGGUACAUCUCUGUCUAC C | SEQ ID NO: 319 | 8540 |
| ds-n122 | GGGAUCUUAAGGUCAU UGAGA | SEQ ID NO: 122 | UCUCAAUGACCUUAAGAUCC C | SEQ ID NO: 320 | 8836 |
| ds-n123 | GUAGGUGACCCCUUGG AAUAA | SEQ ID NO: 123 | UUAUUCCAAGGGGUCACCUA C | SEQ ID NO: 321 | 8892 |
| ds-n124 | GCUGGUUAUAAGGGUU UUGUU | SEQ ID NO: 124 | AACAAAACCCUUAUAACCAG C | SEQ ID NO: 322 | 8975 |
| ds-n125 | UGGUUAUAAGGGUUUU GUUUA | SEQ ID NO: 125 | UAAACAAAACCCUUAUAACC A | SEQ ID NO: 323 | 8977 |
| ds-n126 | CACCCACCUUGACUAU ACCAA | SEQ ID NO: 126 | UUGGUAUAGUCAAGGUGGGU G | SEQ ID NO: 324 | 9163 |
| ds-n127 | CUAUACCAAGGCAUCA UCUAU | SEQ ID NO: 127 | AUAGAUGAUGCCUUGGUAUA G | SEQ ID NO: 325 | 9175 |
| ds-n128 | CAUCAUCUAUCCACAG UUCUA | SEQ ID NO: 128 | UAGAACUGUGGAUAGAUGAU G | SEQ ID NO: 326 | 9186 |
| ds-n129 | CUUAAGCUGAGUUGUG GCAUU | SEQ ID NO: 129 | AAUGCCACAACUCAGCUUAA G | SEQ ID NO: 327 | 9265 |
| ds-n130 | UAAGCUGAGUUGUGGC AUUUU | SEQ ID NO: 130 | AAAAUGCCACAACUCAGCUU A | SEQ ID NO: 328 | 9267 |
| ds-n131 | ACUGAGGCACAUCAUG GAAUU | SEQ ID NO: 131 | AAUUCCAUGAUGUGCCUCAG U | SEQ ID NO: 329 | 9340 |
| ds-n132 | CACAUCAUGGAAUUGA UGUGA | SEQ ID NO: 132 | UCACAUCAAUUCCAUGAUGU G | SEQ ID NO: 330 | 9347 |
| ds-n133 | CAUGGAAUUGAUGUGA GCAUU | SEQ ID NO: 133 | AAUGCUCACAUCAAUUCCAU G | SEQ ID NO: 331 | 9352 |
| ds-n134 | GAAUUGAUGUGAGCAU UAAGA | SEQ ID NO: 134 | UCUUAAUGCUCACAUCAAUU C | SEQ ID NO: 332 | 9356 |
| ds-n135 | GGGAUGUCAUGACGUU UGACA | SEQ ID NO: 135 | UGUCAAACGUCAUGACAUCC C | SEQ ID NO: 333 | 9478 |
| ds-n136 | GAUGUCAUGACGUUUG ACAUA | SEQ ID NO: 136 | UAUGUCAAACGUCAUGACAU C | SEQ ID NO: 334 | 9480 |
| ds-n137 | GAAAAGUGGCUUCGUA AAAUA | SEQ ID NO: 137 | UAUUUUACGAAGCCACUUUU C | SEQ ID NO: 335 | 9606 |
| ds-n138 | GUGGCUUCGUAAAAUA GAAGA | SEQ ID NO: 138 | UCUUCUAUUUUACGAAGCCA C | SEQ ID NO: 336 | 9611 |
| ds-n139 | AGUCACUGUGGAACUA CCAAA | SEQ ID NO: 139 | UUUGGUAGUUCCACAGUGAC U | SEQ ID NO: 337 | 9634 |
| ds-n140 | GGUGCUUUGGGAUAAA AGAUU | SEQ ID NO: 140 | AAUCUUUUAUCCCAAAGCAC C | SEQ ID NO: 338 | 9680 |
| ds-n141 | GUGCUUUGGAUAAAA GAUUU | SEQ ID NO: 141 | AAAUCUUUUAUCCCAAAGCA C | SEQ ID NO: 339 | 9681 |
| ds-n142 | CCAGAUUCUAGGCCAG UUUGU | SEQ ID NO: 142 | ACAAACUGGCCUAGAAUCUG G | SEQ ID NO: 340 | 9724 |
| ds-n143 | CAGAUUCUAGGCCAGU UUGUU | SEQ ID NO: 143 | AACAAACUGGCCUAGAAUCU G | SEQ ID NO: 341 | 9725 |
| ds-n144 | GUGGCAAGAUCACACU GAGAU | SEQ ID NO: 144 | AUCUCAGUGUGAUCUUGCCA C | SEQ ID NO: 342 | 9814 |
| ds-n145 | GGGGUUUGUUCUUUUC GUUAA | SEQ ID NO: 145 | UUAACGAAAAGAACAAACCC C | SEQ ID NO: 343 | 10015 |
| ds-n146 | GGGUUUGUUCUUUUCG UUAAU | SEQ ID NO: 146 | AUUAACGAAAAGAACAAACC C | SEQ ID NO: 344 | 10016 |
| ds-n147 | GAUGUUAUUUAGACUG AACCU | SEQ ID NO: 147 | AGGUUCAGUCUAAAUAACAU C | SEQ ID NO: 345 | 10189 |
| ds-n148 | ACAGGUCAGAGGGUUU CAUUU | SEQ ID NO: 148 | AAAUGAAACCCUCUGACCUG U | SEQ ID NO: 346 | 10244 |
| ds-n149 | UGGCCUUCAUCUUAGA UGACU | SEQ ID NO: 149 | AGUCAUCUAAGAUGAAGGCC A | SEQ ID NO: 347 | 10266 |
| ds-n150 | CAUUUGGUUUCAACAA AGAAA | SEQ ID NO: 150 | UUUCUUUGUUGAAACCAAAU G | SEQ ID NO: 348 | 10490 |
| ds-n151 | UGGAAACUGAUCUCGG AGUUA | SEQ ID NO: 151 | UAACUCCGAGAUCAGUUCC A | SEQ ID NO: 349 | 10526 |
| ds-n152 | GAAACUGAUCUCGGAG UUAAG | SEQ ID NO: 152 | CUUAACUCCGAGAUCAGUUU C | SEQ ID NO: 350 | 10528 |
| ds-n153 | GAUCUCGGAGUUAAGG CGAAU | SEQ ID NO: 153 | AUUCGCCUUAACUCCGAGAU C | SEQ ID NO: 351 | 10534 |
| ds-n154 | AUCUCGGAGUUAAGGC GAAUU | SEQ ID NO: 154 | AAUUCGCCUUAACUCCGAGA U | SEQ ID NO: 352 | 10535 |
| ds-n155 | CUCGGAGUUAAGGCGA AUUGU | SEQ ID NO: 155 | ACAAUUCGCCUUAACUCCGA G | SEQ ID NO: 353 | 10537 |
| ds-n156 | GGAGUUAAGGCGAAUU GUUCA | SEQ ID NO: 156 | UGAACAAUUCGCCUUAACUC C | SEQ ID NO: 354 | 10540 |
| ds-n157 | GAGUUAAGGCGAAUUG UUCAA | SEQ ID NO: 157 | UUGAACAAUUCGCCUUAACU C | SEQ ID NO: 355 | 10541 |
| ds-n158 | CUUUCUGGUCAACAUU GUUUU | SEQ ID NO: 158 | AAAACAAUGUUGACCAGAAA G | SEQ ID NO: 356 | 10679 |
| ds-n159 | UGUUUUAACUAGUCAC UCAUU | SEQ ID NO: 159 | AAUGAGUGACUAGUUAAAAC A | SEQ ID NO: 357 | 10694 |
| ds-n160 | CGUUUUCAAUAGGGCU CUUAA | SEQ ID NO: 160 | UUAAGAGCCCUAUUGAAAAC G | SEQ ID NO: 358 | 10717 |
| ds-n161 | GGGCUCUUAAGUCCAG UAGAU | SEQ ID NO: 161 | AUCUACUGGACUUAAGAGCC C | SEQ ID NO: 359 | 10728 |
| ds-n162 | GGCUCUUAAGUCCAGU AGAUU | SEQ ID NO: 162 | AAUCUACUGGACUUAAGAGC C | SEQ ID NO: 360 | 10729 |
| ds-n163 | GCUCUUAAGUCCAGUA GAUUA | SEQ ID NO: 163 | UAAUCUACUGGACUUAAGAG C | SEQ ID NO: 361 | 10730 |
| ds-n164 | GUAGUCAGUUGACGAA GAUCU | SEQ ID NO: 164 | AGAUCUUCGUCAACUGACUA C | SEQ ID NO: 362 | 10754 |
| ds-n165 | UGACGAAGAUCUGGUU UACAA | SEQ ID NO: 165 | UUGUAAACCAGAUCUUCGUC A | SEQ ID NO: 363 | 10763 |
| ds-n166 | CGAAGAUCUGGUUUAC AAGAA | SEQ ID NO: 166 | UUCUUGUAAACCAGAUCUUC G | SEQ ID NO: 364 | 10766 |
| ds-n167 | ACCUGUUUACUAGCUA GCUUU | SEQ ID NO: 167 | AAAGCUAGCUAGUAAACAGG U | SEQ ID NO: 365 | 10942 |
| ds-n168 | CCCUAGUUUAUAACAA AGGAA | SEQ ID NO: 168 | UUCCUUUGUUAUAAACUAGG G | SEQ ID NO: 366 | 11034 |
| ds-n169 | CUGUAUCUUCAGUAUC UUGGU | SEQ ID NO: 169 | ACCAAGAUACUGAAGAUACA G | SEQ ID NO: 367 | 11078 |
| ds-n170 | GAUCAUUUUUACUGG UCAUU | SEQ ID NO: 170 | AAUGACCAGUAAAAAAUGAU C | SEQ ID NO: 368 | 11126 |
| ds-n171 | AGUGUAGCUACUUUAA CAGAU | SEQ ID NO: 171 | AUCUGUUAAAGUAGCUACAC U | SEQ ID NO: 369 | 11156 |
| ds-n172 | GUAGCUACUUUAACAG AUGGA | SEQ ID NO: 172 | UCCAUCUGUUAAAGUAGCUA C | SEQ ID NO: 370 | 11159 |
| ds-n173 | UAGCUACUUUAACAGA UGGAA | SEQ ID NO: 173 | UUCCAUCUGUUAAAGUAGCU A | SEQ ID NO: 371 | 11160 |
| ds-n174 | UGGACAUGGAAGACU UGACU | SEQ ID NO: 174 | AGUCAAGUCUUCCCAUGUCC A | SEQ ID NO: 372 | 11292 |
| ds-n175 | ACAGCAUACACAGUGA UCACA | SEQ ID NO: 175 | UGUGAUCACUGUGUAUGCUG U | SEQ ID NO: 373 | 11340 |
| ds-n176 | CAUACACAGUGAUCAC AUAAA | SEQ ID NO: 176 | UUUAUGUGAUCACUGUGUAU G | SEQ ID NO: 374 | 11344 |
| ds-n177 | ACACAGGCCAUUUGCU UACAU | SEQ ID NO: 177 | AUGUAAGCAAAUGGCCUGUG U | SEQ ID NO: 375 | 11383 |
| ds-n178 | UGCUUACAUGCCUCGU AUCAU | SEQ ID NO: 178 | AUGAUACGAGGCAUGUAAGC A | SEQ ID NO: 376 | 11395 |
| ds-n179 | GAGGUUUGCCAGAGUU UGUCU | SEQ ID NO: 179 | AGACAAACUCUGGCAAACCU C | SEQ ID NO: 377 | 11789 |
| ds-n180 | GGAAUGGAUGCACCGC AAAUA | SEQ ID NO: 180 | UAUUUGCGGUGCAUCCAUUC C | SEQ ID NO: 378 | 11864 |
| ds-n181 | GAAUGGAUGCACCGCA AAUAA | SEQ ID NO: 181 | UUAUUUGCGGUGCAUCCAUU C | SEQ ID NO: 379 | 11865 |
| ds-n182 | AAGGUAAUAACAUGGC CAAUU | SEQ ID NO: 182 | AAUUGGCCAUGUUAUUACCU U | SEQ ID NO: 380 | 11939 |
| ds-n183 | UAUACUCUGGAUUCUU UACAU | SEQ ID NO: 183 | AUGUAAAGAAUCCAGAGUAU A | SEQ ID NO: 381 | 12092 |
| ds-n184 | GGAAAAAAGAAACUGU CUAUU | SEQ ID NO: 184 | AAUAGACAGUUUCUUUUUC C | SEQ ID NO: 382 | 12116 |
| ds-n185 | GUCUAUUUGAAUGGC UGAAG | SEQ ID NO: 185 | CUUCAGCCAUUCAAAAUAGA C | SEQ ID NO: 383 | 12130 |
| ds-n186 | AGGCAACGUUAGUUUC UCUUA | SEQ ID NO: 186 | UAAGAGAAACUAACGUUGCC U | SEQ ID NO: 384 | 12154 |
| ds-n187 | GCAACGUUAGUUUCUC UUACU | SEQ ID NO: 187 | AGUAAGAGAAACUAACGUUG C | SEQ ID NO: 385 | 12156 |
| ds-n188 | CUGCUUUUUUCUAGUA AAGUA | SEQ ID NO: 188 | UACUUUACUAGAAAAAAGCA G | SEQ ID NO: 386 | 12177 |
| ds-n189 | GUACUACAUGGUUUAA GUUAA | SEQ ID NO: 189 | UUAACUUAAACCAUGUAGUA C | SEQ ID NO: 387 | 12195 |
| ds-n190 | AACGACUAUCAGCAGC UGAAU | SEQ ID NO: 190 | AUUCAGCUGCUGAUAGUCGU UGC | SEQ ID NO: 388 | 1164 |
| ds-n191 | CAACGACUAUCAGCAG CUGAA | SEQ ID NO: 191 | UUCAGCUGCUGAUAGUCGUU GCG | SEQ ID NO: 389 | 1163 |
| ds-n192 | GCAACGACUAUCAGCA GCUGA | SEQ ID NO: 192 | UCAGCUGCUGAUAGUCGUUG CGG | SEQ ID NO: 390 | 1162 |
| ds-n193 | CGCAACGACUAUCAGC AGCUU | SEQ ID NO: 193 | AAGCUGCUGAUAGUCGUUGC GGA | SEQ ID NO: 391 | 1161 |
| ds-n194 | CCGCAACGACUAUCAG CAGCU | SEQ ID NO: 194 | AGCUGCUGAUAGUCGUUGCG GAU | SEQ ID NO: 392 | 1160 |
| ds-n195 | ACGACUAUCAGCAGCU GAAGU | SEQ ID NO: 195 | ACUUCAGCUGCUGAUAGUCG UUG | SEQ ID NO: 393 | 1165 |
| ds-n196 | CGACUAUCAGCAGCUG AAGCU | SEQ ID NO: 196 | AGCUUCAGCUGCUGAUAGUC GUU | SEQ ID NO: 394 | 1166 |
| ds-n197 | GACUAUCAGCAGCUGA AGCGU | SEQ ID NO: 197 | ACGCUUCAGCUGCUGAUAGU CGU | SEQ ID NO: 395 | 1167 |
| ds-n198 | ACUAUCAGCAGCUGAA GCGCU | SEQ ID NO: 198 | AGCGCUUCAGCUGCUGAUAG UCG | SEQ ID NO: 396 | 1168 |
| ds-n201 | AUUUGCAUACACAUCG UCUUU | SEQ ID NO: 23 | AAAGACGAUGUGUAUGCAAA UGU | SEQ ID NO: 397 | 6329 |
| ds-n202 | CUUUUUUACGGUUCAG AUAUU | SEQ ID NO: 98 | AAUAUCUGAACCGUAAAAAA GUU | SEQ ID NO: 398 | 6366 |
| ds-n203 | ACGGUUCAGAUAUUCA UCUAU | SEQ ID NO: 99 | AUAGAUGAAUAUCUGAACCG UAA | SEQ ID NO: 399 | 6373 |
| ds-n204 | CUUGAUCUUUGUGGAU UUAAU | SEQ ID NO: 103 | AUUAAAUCCACAAAGAUCAA GAA | SEQ ID NO: 400 | 6434 |
| ds-n190-1 | AACGACUAUCAGCAGC UGAAU | SEQ ID NO: 190 | CGAAUUCAGCUGCUGAUAGU CGUUGC | SEQ ID NO: 401 | 1164 |
| ds-n191-1 | CAACGACUAUCAGCAG CUGAA | SEQ ID NO: 191 | CGAUUCAGCUGCUGAUAGUC GUUGCG | SEQ ID NO: 402 | 1163 |
| ds-n192-1 | GCAACGACUAUCAGCA GCUGA | SEQ ID NO: 192 | CGAUCAGCUGCUGAUAGUCG UUGCGG | SEQ ID NO: 403 | 1162 |
| ds-n193-1 | CGCAACGACUAUCAGC AGCUU | SEQ ID NO: 193 | CGAAAGCUGCUGAUAGUCGU UGCGGA | SEQ ID NO: 404 | 1161 |
| ds-n194-1 | CCGCAACGACUAUCAG CAGCU | SEQ ID NO: 194 | CGAAGCUGCUGAUAGUCGUU GCGGAU | SEQ ID NO: 405 | 1160 |
| ds-n195-1 | ACGACUAUCAGCAGCU GAAGU | SEQ ID NO: 195 | CGAACUUCAGCUGCUGAUAG UCGUUG | SEQ ID NO: 406 | 1165 |
| ds-n196-1 | CGACUAUCAGCAGCUG AAGCU | SEQ ID NO: 196 | CGAAGCUUCAGCUGCUGAUA GUCGUU | SEQ ID NO: 407 | 1166 |
| ds-n197-1 | GACUAUCAGCAGCUGA AGCGU | SEQ ID NO: 197 | CGAACGCUUCAGCUGCUGAU AGUCGU | SEQ ID NO: 408 | 1167 |
| ds-n198-1 | ACUAUCAGCAGCUGAA GCGCU | SEQ ID NO: 198 | CGAAGCGCUUCAGCUGCUGA UAGUCG | SEQ ID NO: 409 | 1168 |
| ds-n201-1 | AUUUGCAUACACAUCG UCUUU | SEQ ID NO: 23 | CGAAAAGACGAUGUGUAUGC AAAUGU | SEQ ID NO: 410 | 6329 |
| ds-n202-1 | CUUUUUUACGGUUCAG AUAUU | SEQ ID NO: 98 | CGAAAUAUCUGAACCGUAAA AAAGUU | SEQ ID NO: 411 | 6366 |
| ds-n203-1 | ACGGUUCAGAUAUUCA UCUAU | SEQ ID NO: 99 | CGAAUAGAUGAAUAUCUGAA CCGUAA | SEQ ID NO: 412 | 6373 |
| ds-n204-1 | CUUGAUCUUUGUGGAU UUAAU | SEQ ID NO: 103 | CGAAUUAAAUCCACAAAGAU CAAGAA | SEQ ID NO: 413 | 6434 |

modified nucleotide

In order to enhance the in vivo stability of the aforementioned RNA inhibitors without impairing-and even potentially enhancing-their activity, the sense strand and/or antisense strand of the aforementioned RNA inhibitors may be modified by introducing modifying groups onto the nucleotides, either throughout the entire strand or at specific positions. In certain embodiments, one or more nucleotides on the sense strand and/or antisense strand are modified to form modified nucleotides.

As used herein, the term "nucleotide" refers to a compound composed of a five-carbon sugar (ribose or deoxyribose), a phosphate group, and a base (natural or non-natural), and is intended to encompass both unmodified (i.e., natural) nucleotides and modified nucleotides. In some embodiments, the nucleotide is an unmodified ribonucleotide. In some embodiments, the ribonucleotide is a 3'-ribonucleotide. In some embodiments, the ribonucleotide is a 5'-ribonucleotide. In some embodiments, modified or unmodified nucleotides may optionally be further modified.

Natural nucleotides consist of natural bases, natural ribose (or deoxyribose), and phosphate. As used herein, natural nucleotides refer to adenosine ribonucleotide, adenosine deoxyribonucleotide, guanosine ribonucleotide, guanosine deoxyribonucleotide, cytidine ribonucleotide, cytidine deoxyribonucleotide, uridine ribonucleotide, thymidine ribonucleotide, or thymidine deoxyribonucleotide. "Ribonucleotide" refers to a nucleotide having a hydroxyl group at the 2'-position of the sugar moiety. "Deoxyribonucleotide" refers to a nucleotide having a hydrogen atom at the 2'-position of the sugar moiety.

Natural bases in RNA include A (adenine), G (guanine), C (cytosine), U (uracil), and T (thymine).

As used herein, prefixing a monomer (e.g., nucleotides A, U, C, G, and T, etc.) with "d" indicates that the monomer is modified with a 2'-deoxy modification.

As used herein, suffixing a monomer (e.g., nucleotides A, U, C, G, and T, etc.) with "f" indicates that the monomer is modified with a 2'-deoxy-2'-fluoro modification (2'-F modification).

As used herein, prefixing a monomer (e.g., nucleotide) with "GNA-" indicates that the monomer is modified with a glycol nucleic acid modification (GNA modification). As used herein, Tgn is the designation for GNA-T and is equivalent in meaning.

As used herein, lowercase letters (a, u, c, g, t, etc.) indicate that the corresponding nucleotides represented by uppercase letters (A, U, C, G, and T, etc.) are modified with 2'-O-methyl (2'-OMe).

As used herein, invAB modification refers to the linkage of an inverted abasic nucleotide at the 5' or 3' terminus of a nucleotide. For example, 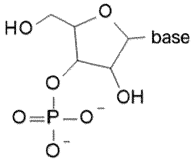 the structure after invAB modification:

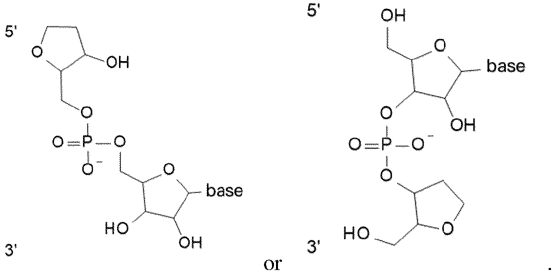

As used herein, M06 modification refers to the linkage of 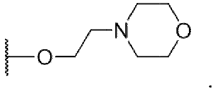 For example, 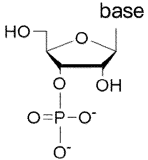 the structure after M06 modification via a phosphodiester bond: 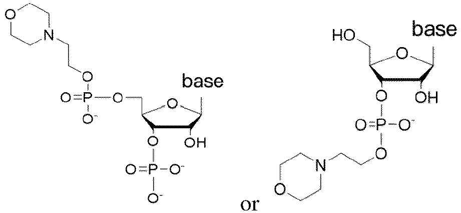 . In the present invention, (M06) 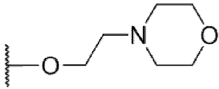 is the residue of the M06 monomer 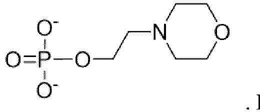 In some embodiments, (M06) is linked to the nucleotide via 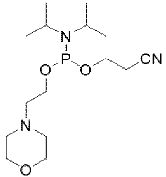 bond.

As used herein, an asterisk ("*") placed between monomers (e.g., nucleotides A, U, C, G, and T, etc.) indicates that the two monomers are connected via a phosphorothioate linkage (i.e., a phosphorothioate diester bond), meaning they are modified with phosphorothioate (PS).

As used herein, the absence of an asterisk ("*") between nucleotides (e.g., A, U, C, G, and T, etc.) indicates that the two nucleotides are connected via a phosphate ester bond (i.e., a phosphodiester bond).

For example, "5'-Phosphorothioate-invAB" denotes that (invAB) is linked to the 5' end of a monomer via a phosphorothioate bond. Similarly, "5'-Phosphorothioate-M06" denotes that (M06) is linked to the 5' end of a monomer via a phosphorothioate bond.

All nucleotides in the small inhibitory nucleic acid molecules described herein may be natural or unmodified nucleotides, or at least one nucleotide may be a modified nucleotide, wherein said modification is one or a combination of the following:
(1) modification of the phosphodiester bonds within the nucleotide sequence of said small inhibitory nucleic acid molecule;
(2) modification of the 2'-OH of the ribose in the nucleotide sequence of said small inhibitory nucleic acid molecule;
(3) modification of the bases in the nucleotide sequence of said small inhibitory nucleic acid molecule.

The chemical modifications of the present invention are well known to those skilled in the art. The modification of the phosphodiester bond refers to modification of the oxygen in the phosphodiester bond, including phosphorothioate modification and boranophosphate modification. Both modifications can stabilize the siRNA structure while maintaining high specificity and high affinity of base pairing.

The ribose modification refers to modification of the 2'-OH of the pentose sugar in nucleotides, i.e., introduction of certain substituents at the hydroxyl position of the ribose, for example, 2'-fluoro modification, 2'-O-methyl modification, 2'-O-ethyleneglycol methoxy modification, 2,4'-dinitrophenol modification, locked nucleic acid (LNA), 2'-amino modification, and 2'-deoxy modification.

The base modification refers to modification of the base of nucleotides, for example, 5'-bromouracil modification, 5'-iodouracil modification, N-methyluracil modification, and 2,6-diaminopurine modification.

In some embodiments, the ribose modification comprises fluorination and/or methoxy substitution at the 2'-OH.

In some embodiments, the ribose modification further comprises modification with UNA, LNA, or GNA.

The term "LNA" denotes a bicyclic nucleoside analog containing a C2*-C4* bivalent (bridge) and is referred to as "locked nucleic acid." It may denote an LNA monomer, or, when used in the context of an "LNA oligonucleotide," LNA denotes an oligonucleotide containing one or more such bicyclic nucleoside analogs. In certain aspects, the bicyclic nucleoside analog is an LNA nucleotide, and thus these terms may be used interchangeably; in such embodiments, both are characterized by the presence of a linking group (such as a bridge) between C2' and C4' of the ribose sugar ring.

The UNA (unlocked nucleic acid) structure is similar to RNA but lacks the C2-C3 bond of the ribose ring, and its structure is shown below: 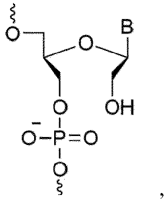 where B represents a base.

GNA (glycol nucleic acid) is a chemical substance similar to DNA or RNA but differs in composition and is not found in any naturally occurring organism currently known. GNA contains an acyclic three-carbon propylene glycol (1,2-propanediol) backbone that replaces the (deoxy)ribose of DNA and RNA, representing the simplest chemically stable nucleic acid structure. The (S)-GNA structure is shown below. 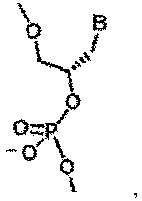 where B represents a base.

In certain embodiments, the RNA inhibitor comprises at least one chemically modified nucleotide.

In certain embodiments, the RNA inhibitor comprises chemically modified nucleotides at all positions.

In certain embodiments, the RNA inhibitor comprises one or more of the following modifications: 2'-OMe (2'-O-methyl) modification, 2'-F (2'-deoxy-2'-fluoro) modification, 2'-O-MOE (2'-O-methoxyethyl) modification, 2'-deoxy (2'-d) modification, 5'-morpholino (5'-Mo) modification, unlocked nucleic acid (UNA) modification, glycol nucleic acid (GNA) modification, locked nucleic acid (LNA) modification, tricyclo-DNA (tcDNA) modification, (S)-constrained ethyl bicyclic nucleic acid ((S)-cEt-BNA) modification, phosphorothioate (PS) modification, dithiophosphate (PS2) modification, methylphosphonate (MP) modification, methoxypropyl methylphosphonate (MOP) modification, peptide nucleic acid (PNA) modification, 5'-(E)-vinyl phosphate (VP) modification (VP), N6-methyladenosine (m6A) modification, 5-methylcytidine (m5C) modification, 3-methyluridine (m3U) modification, 5-methyluridine (m5U) modification, pseudouridine modification, 2-thiouridine (s2U) modification, propargyluridine (5-pU) modification, linkage of an inverted abasic nucleotide (invAB) at the 5' or 3' end of a nucleotide, replacement of a nucleotide with an inverted abasic nucleotide (invAb) modification, replacement of a nucleotide with a 2,4-difluorotoluene ribonucleotide (rF) modification, or replacement of a nucleotide with an (S)-glycerol nucleic acid modification; preferred modifications include 2'-OMe modification, 2'-F modification, 2'-deoxy modification, VP modification, 5'-MP modification, PS modification, PS2 modification, MP modification, MOP modification, invAb modification, and invAB modification.

In certain embodiments, the RNA inhibitor comprises a sense strand of 21 nt in length, wherein counting from the 5' end, the nucleotide at position 9 of the sense strand bears a 2'-F modification.

In certain embodiments, the RNA inhibitor comprises a sense strand of 21 nt in length, wherein counting from the 5' end, the nucleotide at position 10 of the sense strand bears a 2'-F modification.

In certain embodiments, the RNA inhibitor comprises a sense strand of 21 nt in length, wherein counting from the 5' end, the nucleotide at position 11 of the sense strand bears a 2'-F modification.

In certain embodiments, the RNA inhibitor comprises a sense strand of 21 nt in length, wherein counting from the 5' end, the nucleotides at positions 9, 10, and 11 of the sense strand bear 2'-F modifications, and the remaining nucleotides bear 2'-OMe modifications.

In certain embodiments, the RNA inhibitor comprises a sense strand of 21 nt in length, wherein counting from the 5' end, the 5' end of the nucleotide at position 1 of the sense strand bears an invAB modification.

In certain embodiments, the inverted abasic nucleotide is linked via a phosphorothioate bond to the 5' end of the nucleotide at position 1 of the sense strand.

In certain embodiments, the RNA inhibitor comprises an antisense strand of 21 nt in length, wherein counting from the 5' end, a phosphorothioate linkage exists between nucleotides at positions 1 and 2 of the antisense strand.

In certain embodiments, the RNA inhibitor comprises an antisense strand of 21 nt in length, wherein counting from the 5' end, a phosphorothioate linkage exists between nucleotides at positions 2 and 3 of the antisense strand.

In certain embodiments, the RNA inhibitor comprises an antisense strand of 21 nt in length, wherein counting from the 5' end, a phosphorothioate linkage exists between nucleotides at positions 3 and 4 of the antisense strand.

In certain embodiments, the RNA inhibitor comprises an antisense strand of 21 nt in length, wherein counting from the 3' end, a phosphorothioate linkage exists between nucleotides at positions 1 and 2 of the antisense strand.

In certain embodiments, the RNA inhibitor comprises an antisense strand of 21 nt in length, wherein counting from the 5' end, phosphorothioate linkages exist between nucleotides at positions 1 and 2, between positions 2 and 3, and between positions 3 and 4; and counting from the 3' end, a phosphorothioate linkage exists between nucleotides at positions 1 and 2 of the antisense strand.

In certain embodiments, the RNA inhibitor comprises an antisense strand of 21 nt in length, wherein counting from the 5' end, the nucleotide at position 2 of the antisense strand bears a 2'-F modification.

In certain embodiments, the RNA inhibitor comprises an antisense strand of 21 nt in length, wherein counting from the 5' end, the nucleotide at position 3 of the antisense strand bears a 2'-F modification.

In certain embodiments, the RNA inhibitor comprises an antisense strand of 21 nt in length, wherein counting from the 5' end, the nucleotide at position 4 of the antisense strand bears a 2'-F modification.

In certain embodiments, the RNA inhibitor comprises an antisense strand of 21 nt in length, wherein counting from the 5' end, the nucleotide at position 12 of the antisense strand bears a 2'-F modification.

In certain embodiments, the RNA inhibitor comprises an antisense strand of 21 nt in length, wherein counting from the 5' end, the nucleotide at position 14 of the antisense strand bears a 2'-F modification.

In certain embodiments, the RNA inhibitor comprises an antisense strand of 21 nt in length, wherein counting from the 5' end, the nucleotide at position 16 of the antisense strand bears a 2'-F modification.

In certain embodiments, the RNA inhibitor comprises an antisense strand of 21 nt in length, wherein counting from the 5' end, the nucleotides at positions 2, 3, 4, 12, 14, and 16 of the antisense strand bear 2'-F modifications, and the remaining nucleotides bear 2'-OMe modifications.

In certain embodiments, the RNA inhibitor comprises an antisense strand of 21 nt in length, wherein counting from the 5' end, phosphorothioate linkages exist between nucleotides at positions 1 and 2, between positions 2 and 3, and between positions 3 and 4; and counting from the 3' end, a phosphorothioate linkage exists between nucleotides at positions 1 and 2; and the nucleotides at positions 2, 3, 4, 12, 14, and 16 bear 2'-F modifications, with the remaining nucleotides bearing 2'-OMe modifications.

In certain embodiments, the RNA inhibitor,
a) wherein when the sense strand is 21 nt in length, the sense strand comprises the following modifications: counting from the 5' end, the nucleotides at positions 9, 10, and 11 bear 2'-F modifications, the remaining nucleotides bear 2'-OMe modifications, and the 5' end of the nucleotide at position 1 bears an invAB modification,
b) when the antisense strand is 21 nt in length, the antisense strand comprises the following modifications: counting from the 5' end, phosphorothioate linkages exist between nucleotides at positions 1 and 2, between positions 2 and 3, and between positions 3 and 4; and counting from the 3' end, a phosphorothioate linkage exists between nucleotides at positions 1 and 2; and the nucleotides at positions 2, 3, 4, 12, 14, and 16 bear 2'-F modifications, with the remaining nucleotides bearing 2'-OMe modifications.

In certain embodiments, the RNA inhibitor comprises a sense strand of 21 nt in length, wherein counting from the 5' end, the nucleotide at position 18 of the sense strand bears a 2'-F modification.

In certain embodiments, the RNA inhibitor comprises a sense strand of 21 nt in length, wherein counting from the 5' end, the nucleotides at positions 9, 10, 11, and 18 of the sense strand bear 2'-F modifications, and the remaining nucleotides bear 2'-OMe modifications.

In certain embodiments, the RNA inhibitor comprises a sense strand of 21 nt in length, wherein counting from the 5' end, a phosphorothioate linkage exists between nucleotides at positions 1 and 2 of the sense strand.

In certain embodiments, the RNA inhibitor comprises a sense strand of 21 nt in length, wherein counting from the 5' end, a phosphorothioate linkage exists between nucleotides at positions 2 and 3 of the sense strand.

In certain embodiments, the RNA inhibitor comprises a sense strand of 21 nt in length, wherein counting from the 3' end, a phosphorothioate linkage exists between nucleotides at positions 1 and 2 of the sense strand.

In certain embodiments, the RNA inhibitor comprises a sense strand of 21 nt in length, wherein counting from the 3' end, a phosphorothioate linkage exists between nucleotides at positions 2 and 3 of the sense strand.

In certain embodiments, the RNA inhibitor comprises a sense strand of 21 nt in length, wherein counting from the 5' end, phosphorothioate linkages exist between nucleotides at positions 1 and 2 and between positions 2 and 3; the nucleotides at positions 9, 10, 11, and 18 bear 2'-F modifications, and the remaining nucleotides bear 2'-OMe modifications; and counting from the 3' end, phosphorothioate linkages exist between nucleotides at positions 1 and 2 and between positions 2 and 3 of the sense strand.

In certain embodiments, the RNA inhibitor comprises an antisense strand of 26 nt in length, wherein counting from the 5' end, the nucleotide at position 1 of the antisense strand bears a 2'-F modification.

In certain embodiments, the RNA inhibitor comprises an antisense strand of 26 nt in length, wherein counting from the 5' end, the nucleotide at position 2 of the antisense strand bears a 2'-F modification.

In certain embodiments, the RNA inhibitor comprises an antisense strand of 26 nt in length, wherein counting from the 5' end, the nucleotide at position 5 of the antisense strand bears a 2'-F modification.

In certain embodiments, the RNA inhibitor comprises an antisense strand of 26 nt in length, wherein counting from the 5' end, the nucleotide at position 9 of the antisense strand bears a 2'-F modification.

In certain embodiments, the RNA inhibitor comprises an antisense strand of 26 nt in length, wherein counting from the 5' end, the nucleotide at position 17 of the antisense strand bears a 2'-F modification.

In certain embodiments, the RNA inhibitor comprises an antisense strand of 26 nt in length, wherein counting from the 5' end, the nucleotide at position 19 of the antisense strand bears a 2'-F modification.

In certain embodiments, the RNA inhibitor comprises an antisense strand of 26 nt in length, wherein counting from the 5' end, the nucleotides at positions 1, 2, 5, 9, 17, and 19 of the antisense strand bear 2'-F modifications, and the remaining nucleotides bear 2'-OMe modifications.

In certain embodiments, the RNA inhibitor comprises an antisense strand of 26 nt in length, wherein counting from the 5' end, a phosphorothioate linkage exists between nucleotides at positions 4 and 5.

In certain embodiments, the RNA inhibitor comprises an antisense strand of 26 nt in length, wherein counting from the 3' end, a phosphorothioate linkage exists between nucleotides at positions 1 and 2.

In certain embodiments, the RNA inhibitor comprises an antisense strand of 26 nt in length, wherein counting from the 3' end, a phosphorothioate linkage exists between nucleotides at positions 2 and 3.

In certain embodiments, the RNA inhibitor comprises an antisense strand of 26 nt in length, wherein counting from the 5' end, the nucleotides at positions 1, 2, 5, 9, 17, and 19 bear 2'-F modifications, the remaining nucleotides bear 2'-OMe modifications, and a phosphorothioate linkage exists between nucleotides at positions 4 and 5; and counting from the 3' end, phosphorothioate linkages exist between nucleotides at positions 1 and 2 and between positions 2 and 3.

In certain embodiments, the RNA inhibitor, wherein
a) when the sense strand is 21 nt in length, counting from the 5' end, phosphorothioate linkages exist between nucleotides at positions 1 and 2 and between positions 2 and 3; the nucleotides at positions 9, 10, 11, and 18 bear 2'-F modifications, and the remaining nucleotides bear 2'-OMe modifications; and counting from the 3' end, phosphorothioate linkages exist between nucleotides at positions 1 and 2 and between positions 2 and 3;
b) when the antisense strand is 26 nt in length, counting from the 5' end, the nucleotides at positions 1, 2, 5, 9, 17, and 19 bear 2'-F modifications, the remaining nucleotides bear 2'-OMe modifications, and a phosphorothioate linkage exists between nucleotides at positions 4 and 5; and counting from the 3' end, phosphorothioate linkages exist between nucleotides at positions 1 and 2 and between positions 2 and 3.

In certain embodiments, the RNA inhibitor comprises a phosphate or phosphate mimic at the 5'-end of the antisense strand.

In certain embodiments, the phosphate mimic includes 5'-(E)-vinylphosphonate, 5'-methylphosphonate, (S)-5'-C-methyl analog, and 5'-phosphorothioate (5'-PS).

In certain embodiments, the RNA inhibitor comprises (M06) at the 5'-end of the antisense strand:

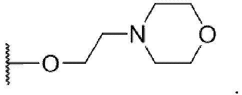

In certain embodiments, (M06) is linked to the 5'-end of the antisense strand via a phosphate or phosphorothioate bond.

In certain embodiments, the RNA inhibitor comprises an invAB modification at the 5'-end of the first nucleotide of the antisense strand.

In certain embodiments, the inverted abasic nucleotide is linked via a phosphorothioate bond to the 5' end of the first nucleotide of the antisense strand.

The modified sequences are shown in Table 2.

**Table 2 Modified duplexes**

| **Corresponding unmodified duplex** | **Modified duplex** | **Modified sense strand (5'→3')** | **No .** | **Modified antisense strand (5'→3')** | **No .** | **NM_000 875.5 position** |
|---|---|---|---|---|---|---|
| ds-n1 | ds-m1 | (invAB)*cuggccgaCfGfAfgu ggagaaau | SEQ ID N O: 41 4 | a*Uf*Uf*UfcuccacuCfgUfcGfgc ca*g | SE Q ID N O: 62 4 | 1118 |
| ds-n2 | ds-m2 | (invAB)*ggccgacgAfGfUfgg agaaaucu | SEQ ID N O: 41 5 | a*Gf*Af*UfuucuccaCfuCfgUfcg gc*c | SE Q ID N O: 62 5 | 1120 |
| ds-n3 | ds-m3 | (invAB)*cgacauccGfCfAfac gacuauca | SEQ ID N O: 41 6 | u*Gf*Af*UfagucguuGfcGfgAfu guc*g | SEQ ID N O: 62 6 | 1154 |
| ds-n4 | ds-m4 | (invAB)*cuggaaacUfCfUfuc uacaacua | SE Q ID N O: 41 7 | u*Af*Gf*UfuguagaaGfaGfuUfu cca*g | SE Q ID N O: 62 7 | 1367 |
| ds-n5 | ds-m5 | (invAB)*augaccaaUfCfUfca aggauauu | SE Q ID N O: 41 8 | a*Af*Uf*AfuccuugaGfaUfuGfg uca*u | SE Q ID N O: 62 8 | 1407 |
| ds-n6 | ds-m6 | (invAB)*ucucaaggAfUfAfuu gggcuuua | SE Q ID N O: 41 9 | u*Af*Af*AfgcccaauAfuCfcUfu gag*a | SE Q ID N O: 62 9 | 1415 |
| ds-n7 | ds-m7 | (invAB)*ggugucccaAfUfAfac uacauugu | SE Q ID N O: 42 0 | a*Cf*Af*AfuguaguuAfuUfgGfa cac*c | SE Q ID N O: 63 0 | 1532 |
| ds-n8 | ds-m8 | (invAB)*gaccaccaUfCfAfaca augagua | SE Q ID NO: 42 1 | u*Af*Cf*UfcauuguuGfaUfgGfu ggu*c | SE Q ID NO: 63 1 | 1625 |
| ds-n9 | ds-m9 | (invAB)*ccaccaucAfAfCfaa ugaguaca | SE Q ID N O: 42 2 | u*Gf*Uf*AfcucauugUfuGfaUfg gug*g | SE Q ID N O: 63 2 | 1627 |
| ds-n10 | ds-m10 | (invAB)*caccaucaAfCfAfau gaguacaa | SE Q ID N O: 42 3 | u*Uf*Gf*UfacucauuGfuUfgAfu ggu*g | SE Q ID N O: 63 3 | 1628 |
| ds-n11 | ds-m11 | (invAB)*caucaacaAfUfGfag uacaacua | SE Q ID N O: 42 4 | u*Af*Gf*UfuguacucAfuUfgUfu gau*g | SE Q ID N O: 63 4 | 1631 |
| ds-n12 | ds-m12 | (invAB)*ggagacagAfGfUfac ccuuucuu | SE Q ID N O: 42 5 | a*Af*Gf*AfaaggguaCfuCfuGfu cuc*c | SE Q ID N O: 63 5 | 3356 |
| ds-n13 | ds-m13 | (invAB)*gagacagaGfUfAfcc cuuucuuu | SE Q ID N O: 42 6 | a*Af*Af*GfaaaggguAfcUfcUfg ucu*c | SE Q ID N O: 63 6 | 3357 |
| ds-n14 | ds-m14 | (invAB)*gacagaguAfCfCfcu uucuuuga | SE Q ID N O: 42 7 | u*Cf*Af*AfagaaaggGfuAfcUfc ugu*c | SE Q ID N O: 63 7 | 3359 |
| ds-n15 | ds-m15 | (invAB)*acagauccUfGfUfgu ucuucuau | SE Q ID N O: 42 8 | a*Uf*Af*GfaagaacaCfaGfgAfuc ug*u | SE Q ID N O: 63 8 | 3792 |
| ds-n16 | ds-m16 | (invAB)*cugugaugAfAfGfga guucaauu | SE Q ID N O: 42 9 | a*Af*Uf*UfgaacuccUfuCfaUfca ca*g | SE Q ID N O: 63 9 | 4198 |
| ds-n17 | ds-m17 | (invAB)*gugaugaaGfGfAfgu ucaauugu | SE Q ID N O: 43 0 | a*Cf*Af*AfuugaacuCfcUfuCfau ca*c | SE Q ID N O: 64 0 | 4200 |
| ds-n18 | ds-m18 | (invAB)*ggaguucaAfUfUfgu caccaugu | SE Q ID N O: 43 1 | a*Cf*Af*UfggugacaAfuUfgAfa cuc*c | SE Q ID N O: 64 1 | 4208 |
| ds-n19 | ds-m19 | (invAB)*gggcuuguCfCfAfac gagcaagu | SE Q ID N O: 43 2 | a*Cf*Uf*UfgcucguuGfgAfcAfa gcc*c | SE Q ID N O: 64 2 | 4685 |
| ds-n20 | ds-m20 | (invAB)*uaccucagUfGfGfau cuucagaa | SE Q ID N O: 43 3 | u*Uf*Cf*UfgaagaucCfaCfuGfag gu*a | SE Q ID N O: 64 3 | 5246 |
| ds-n21 | ds-m21 | (invAB)*accuuaauGfAfCfaa cacuuaau | SE Q ID N O: 43 4 | a*Uf*Uf*AfaguguugUfcAfuUfa agg*u | SE Q ID N O: 64 4 | 5392 |
| ds-n22 | ds-m22 | (invAB)*ccuuaaugAfCfAfac acuuaaua | SE Q ID N O: 43 5 | u*Af*Uf*UfaaguguuGfuCfaUfu aag*g | SE Q ID N O: 64 5 | 5393 |
| ds-n23 | ds-m23 | (invAB)*auuugcauAfCfAfca ucgucuuu | SE Q ID N O: 43 6 | a*Af*Af*GfacgauguGfuAfuGfc aaa*u | SE Q ID N O: 64 6 | 6329 |
| ds-n24 | ds-m24 | (invAB)*ugcauacaCfAfUfcg ucuuuaau | SE Q ID N O: 43 7 | a*Uf*Uf*AfaagacgaUfgUfgUfa ugc*a | SE Q ID N O: 64 7 | 6332 |
| ds-n25 | ds-m25 | (invAB)*gcauacacAfUfCfgu cuuuaaug | SE Q ID N O: 43 8 | c*Af*Uf*UfaaagacgAfuGfuGfu aug*c | SE Q ID N O: 64 8 | 6333 |
| ds-n26 | ds-m26 | (invAB)*cauacacaUfCfGfuc uuuaaugu | SE Q ID N O: 43 9 | a*Cf*Af*UfuaaagacGfaUfgUfgu au*g | SE Q ID N O: 64 9 | 6334 |
| ds-n27 | ds-m27 | (invAB)*gccagagaUfGfCfug aagauaca | SE Q ID N O: 44 0 | u*Gf*Uf*AfucuucagCfaUfcUfc ugg*c | SE Q ID N O: 65 0 | 10215 |
| ds-n28 | ds-m28 | (invAB)*guccaguaGfAfUfua cggguagu | SE Q ID N O: 44 1 | a*Cf*Uf*AfcccguaaUfcUfaCfug ga*c | SE Q ID N O: 65 1 | 10738 |
| ds-n29 | ds-m29 | (invAB)*ccaguagaUfUfAfcg gguaguca | SE Q ID N O: 44 2 | u*Gf*Af*CfuacccguAfaUfcUfac ug*g | SE Q ID N O: 65 2 | 10740 |
| ds-n30 | ds-m30 | (invAB)*gaucugguUfUfAfca agaacuaa | SE Q ID N O: 44 3 | u*Uf*Af*GfuucuuguAfaAfcCfa gau*c | SE Q ID N O: 65 3 | 10770 |
| ds-n31 | ds-m31 | (invAB)*ucugguuuAfCfAfag aacuaauu | SE Q ID N O: 44 4 | a*Af*Uf*UfaguucuuGfuAfaAfc cag*a | SE Q ID N O: 65 4 | 10772 |
| ds-n32 | ds-m32 | (invAB)*cugguuuaCfAfAfga acuaauua | SE Q ID NO: 44 5 | u*Af*Af*UfuaguucuUfgUfaAfa cca*g | SE Q ID NO: 65 5 | 10773 |
| ds-n33 | ds-m33 | (invAB)*guuuacuaGfCfUfag cuuuacaa | SE Q ID N O: 44 6 | u*Uf*Gf*UfaaagcuaGfcUfaGfua aa*c | SE Q ID N O: 65 6 | 10946 |
| ds-n34 | ds-m34 | (invAB)*uagcuuuaCfAfAfua ugccaaaa | SE Q ID N O: 44 7 | u*Uf*Uf*UfggcauauUfgUfaAfa gcu*a | SE Q ID N O: 65 7 | 10956 |
| ds-n35 | ds-m35 | (invAB)*agcuuuacAfAfUfau gccaaaaa | SE Q ID N O: 44 8 | u*Uf*Uf*UfuggcauaUfuGfuAfa agc*u | SE Q ID N O: 65 8 | 10957 |
| ds-n36 | ds-m36 | (invAB)*caaggauaUfUfGfgg cuuuacaa | SE Q ID N O: 44 9 | u*Uf*Gf*UfaaagcccAfaUfaUfcc uu*g | SE Q ID N O: 65 9 | 1418 |
| ds-n37 | ds-m37 | (invAB)*uaacuacaUfUfGfug gggaauaa | SE Q ID N O: 45 0 | u*Af*Uf*UfuccccacAfaUfgUfag uu*a | SE Q ID N O: 66 0 | 1541 |
| ds-n38 | ds-m38 | (invAB)*agaaaacaAfAfGfac cauugauu | SE Q ID N O: 45 1 | a*Af*Uf*CfaauggucUfuUfgUfu uuc*u | SE Q ID N O: 66 1 | 2050 |
| ds-n39 | ds-m39 | (invAB)*acaaagacCfAfUfug auucuguu | SE Q ID N O: 45 2 | a*Af*Cf*AfgaaucaaUfgGfuCfuu ug*u | SE Q ID N O: 66 2 | 2055 |
| ds-n40 | ds-m40 | (invAB)*caaagaccAfUfUfga uucuguua | SE Q ID N O: 45 3 | u*Af*Af*CfagaaucaAfuGfgUfc uuu*g | SE Q ID N O: 66 3 | 2056 |
| ds-n41 | ds-m41 | (invAB)*ucuccuugUfCfCfuu ccuaaaaa | SE Q ID N O: 45 4 | u*Uf*Uf*UfuaggaagGfaCfaAfg gag*a | SE Q ID N O: 66 4 | 2233 |
| ds-n42 | ds-m42 | (invAB)*cuccuuguCfCfUfuc cuaaaaaa | SE Q ID N O: 45 5 | u*Uf*Uf*UfuuaggaaGfgAfcAfa gga*g | SE Q ID N O: 66 5 | 2234 |
| ds-n43 | ds-m43 | (invAB)*ccaaagcaAfAfGfgg gacauaaa | SE Q ID N O: 45 6 | u*Uf*Uf*AfugucccccUfuUfgCfu uug*g | SE Q ID N O: 66 6 | 2456 |
| ds-n44 | ds-m44 | (invAB)*cuacagggAfUfCfuc aucagcuu | SE Q ID N O: 45 7 | a*Af*Gf*CfugaugagAfuCfcCfu gua*g | SE Q ID N O: 66 7 | 2591 |
| ds-n45 | ds-m45 | (invAB)*caucagcuUfCfAfcc guuuacua | SE Q ID N O: 45 8 | u*Af*Gf*UfaaacgguGfaAfgCfu gau*g | SE Q ID N O: 66 8 | 2603 |
| ds-n46 | ds-m46 | (invAB)*cagcuucaCfCfGfuu uacuacaa | SE Q ID N O: 45 9 | u*Uf*Gf*UfaguaaacGfgUfgAfa gcu*g | SE Q ID N O: 66 9 | 2606 |
| ds-n47 | ds-m47 | (invAB)*cuucaccgUfUfUfac uacaagga | SE Q ID N O: 46 0 | u*Cf*Cf*UfuguaguaAfaCfgGfu gaa*g | SE Q ID N O: 67 0 | 2609 |
| ds-n48 | ds-m48 | (invAB)*ccguuuacGfUfCfaa ggcuguga | SE Q ID N O: 46 1 | u*Cf*Af*CfagccuugAfcGfuAfaa cg*g | SE Q ID N O: 67 1 | 2776 |
| ds-n49 | ds-m49 | (invAB)*acguucuuUfCfAfgc aucgaacu | SE Q ID N O: 46 2 | a*Gf*Uf*UfcgaugcuGfaAfaGfaa cg*u | SE Q ID N O: 67 2 | 2890 |
| ds-n50 | ds-m50 | (invAB)*gaacuccuCfUfUfcu caguuaau | SE Q ID N O: 46 3 | a*Uf*Uf*AfacugagaAfgAfgGfa guu*c | SE Q ID N O: 67 3 | 2906 |
| ds-n51 | ds-m51 | (invAB)*cuccucuuCfUfCfag uuaaucgu | SE Q ID N O: 46 4 | a*Cf*Gf*AfuaacugAfgAfaGfag ga*g | SE Q ID N O: 67 4 | 2909 |
| ds-n52 | ds-m52 | (invAB)*cggcaaccUfGfAfgu uacuacau | SE Q ID N O: 46 5 | a*Uf*Gf*UfaguaacuCfaGfgUfu gcc*g | SE Q ID N O: 67 5 | 2957 |
| ds-n53 | ds-m53 | (invAB)*caaccugaGfUfUfac uacauugu | SE Q ID N O: 46 6 | a*Cf*Af*AfuguaguaAfcUfcAfg guu*g | SE Q ID N O: 67 6 | 2960 |
| ds-n54 | ds-m54 | (invAB)*cuaccuuuAfCfCfgg cacaauua | SE Q ID N O: 46 7 | u*Af*Af*UfugugccgGfuAfaAfg gua*g | SE Q ID N O: 67 7 | 3008 |
| ds-n55 | ds-m55 | (invAB)*uaccgcaaAfGfUfcu uugagaau | SE Q ID N O: 46 8 | a*Uf*Uf*CfucaaagaCfuUfuGfcg gu*a | SE Q ID N O: 67 8 | 3195 |
| ds-n56 | ds-m56 | (invAB)*ggagagaaCfUfGfuc auuucuaa | SE Q ID NO: 46 9 | u*Uf*Af*GfaaaugacAfgUfuCfu cuc*c | SE Q ID NO: 67 9 | 3398 |
| ds-n57 | ds-m57 | (invAB)*ugagaaucCfCfAfau ggauugau | SE Q ID N O: 47 0 | a*Uf*Cf*AfauccauuGfgGfaUfuc uc*a | SE Q ID N O: 68 0 | 3608 |
| ds-n58 | ds-m58 | (invAB)*gagaauccCfAfAfug gauugauu | SE Q ID N O: 47 1 | a*Af*Uf*CfaauccauUfgGfgAfu ucu*c | SE Q ID N O: 68 1 | 3609 |
| ds-n59 | ds-m59 | (invAB)*cggaucacAfAfGfuu gaggauca | SE Q ID N O: 47 2 | u*Gf*Af*UfccucaacUfuGfuGfa ucc*g | SE Q ID N O: 68 2 | 3650 |
| ds-n60 | ds-m60 | (invAB)*guugaggaUfCfAfgc gagaaugu | SE Q ID N O: 47 3 | a*Cf*Af*UfucucgcuGfaUfcCfuc aa*c | SE Q ID N O: 68 3 | 3660 |
| ds-n61 | ds-m61 | (invAB)*ccaggccaAfAfAfca ggauauga | SE Q ID N O: 47 4 | u*Cf*Af*UfauccuguUfuUfgGfc cug*g | SE Q ID N O: 68 4 | 3815 |
| ds-n62 | ds-m62 | (invAB)*aucgugggAfGfGfgu uggugauu | SE Q ID N O: 47 5 | a*Af*Uf*CfaccaaccCfuCfcCfac ga*u | SE Q ID N O: 68 5 | 3882 |
| ds-n63 | ds-m63 | (invAB)*gauguguaCfGfUfuc cugaugag | SE Q ID N O: 47 6 | c*Uf*Cf*AfucaggaaCfgUfaCfac au*c | SE Q ID N O: 68 6 | 3996 |
| ds-n64 | ds-m64 | (invAB)*gucguuugGfGfAfu ggucuauga | SE Q ID N O: 47 7 | u*Cf*Af*UfagaccauCfcCfaAfac ga*c | SE Q ID N O: 68 7 | 4067 |
| ds-n65 | ds-m65 | (invAB)*ugaaaccaGfAfGfug gccauuaa | SE Q ID N O: 47 8 | u*Uf*Af*AfuggccacUfcUfgGfu uuc*a | SE Q ID N O: 68 8 | 4121 |
| ds-n66 | ds-m66 | (invAB)*gaaaccagAfGfUfgg ccauuaaa | SE Q ID N O: 47 9 | u*Uf*Uf*AfauggccaCfuCfuGfg uuu*c | SE Q ID N O: 68 9 | 4122 |
| ds-n67 | ds-m67 | (invAB)*acggggcgAfUfCfuc aaaaguua | SE Q ID N O: 48 0 | u*Af*Af*CfuuuugagAfuCfgCfc ccg*u | SE Q ID N O: 69 0 | 4292 |
| ds-n68 | ds-m68 | (invAB)*cggggcgaUfCfUfca aaaguuau | SE Q ID N O: 48 1 | a*Uf*Af*AfcuuuugaGfaUfcGfc ccc*g | SE Q ID N O: 69 1 | 4293 |
| ds-n69 | ds-m69 | (invAB)*gggcgaucUfCfAfaa aguuaucu | SE Q ID N O: 48 2 | a*Gf*Af*UfaacuuuuGfaGfaUfc gcc*c | SE Q ID N O: 69 2 | 4295 |
| ds-n70 | ds-m70 | (invAB)*gagauaucUfAfUfga gacagacu | SE Q ID N O: 48 3 | a*Gf*Uf*CfugucucaUfaGfaUfau cu*c | SE Q ID N O: 69 3 | 4516 |
| ds-n71 | ds-m71 | (invAB)*auaucuauGfAfGfac agacuauu | SE Q ID N O: 48 4 | a*Af*Uf*AfgucugucUfcAfuAfg aua*u | SE Q ID N O: 69 4 | 4519 |
| ds-n72 | ds-m72 | (invAB)*ucugugcaAfAfCfag uaacgugu | SE Q ID N O: 48 5 | a*Cf*Af*CfguuacugUfuUfgCfac ag*a | SE Q ID N O: 69 5 | 5163 |
| ds-n73 | ds-m73 | (invAB)*cacauuugGfGfAfug uuccuuuu | SE Q ID N O: 48 6 | a*Af*Af*AfggaacauCfcCfaAfau gu*g | SE Q ID N O: 69 6 | 5309 |
| ds-n74 | ds-m74 | (invAB)*acauuuggGfAfUfgu uccuuuuu | SE Q ID N O: 48 7 | a*Af*Af*AfaggaacaUfcCfcAfaa ug*u | SE Q ID N O: 69 7 | 5310 |
| ds-n75 | ds-m75 | (invAB)*caugggccUfUfUfaa gaaccuua | SE Q ID N O: 48 8 | u*Af*Af*GfguucuuaAfaGfgCfc cau*g | SE Q ID N O: 69 8 | 5377 |
| ds-n76 | ds-m76 | (invAB)*gggccuuuAfAfGfaa ccuuaaug | SE Q ID N O: 48 9 | c*Af*Uf*UfaagguucUfuAfaAfg gcc*c | SE Q ID N O: 69 9 | 5380 |
| ds-n77 | ds-m77 | (invAB)*ggccuuuaAfGfAfac cuuaauga | SE Q ID N O: 49 0 | u*Cf*Af*UfuaagguuCfuUfaAfa ggc*c | SE Q ID N O: 70 0 | 5381 |
| ds-n78 | ds-m78 | (invAB)*ucuagggaCfAfUfga aauuuaca | SE Q ID N O: 49 1 | u*Gf*Uf*AfaauuucaUfgUfcCfc uag*a | SE Q ID N O: 70 1 | 5663 |
| ds-n79 | ds-m79 | (invAB)*ccaaggcuGfUfUfac cauuuuaa | SE Q ID N O: 49 2 | u*Uf*Af*AfaauggauaAfcAfgCfc uug*g | SE Q ID N O: 70 2 | 5700 |
| ds-n80 | ds-m80 | (invAB)*accauuuuAfAfCfgc ugccuaau | SE Q ID NO: 49 3 | a*Uf*Uf*AfggcagcgUfuAfaAfa ugg*u | SE Q ID NO: 70 3 | 5711 |
| ds-n81 | ds-m81 | (invAB)*ccauuuuaAfCfGfcu gccuaauu | SE Q ID N O: 49 4 | a*Af*Uf*UfaggcagcGfuUfaAfaa ug*g | SE Q ID N O: 70 4 | 5712 |
| ds-n82 | ds-m82 | (invAB)*cgcugccuAfAfUfuu ugccaaaa | SE Q ID N O: 49 5 | u*Uf*Uf*UfggcaaaaUfuAfgGfc agc*g | SE Q ID N O: 70 5 | 5721 |
| ds-n83 | ds-m83 | (invAB)*caggucucAfUfUfgc uucugacu | SE Q ID N O: 49 6 | a*Gf*Uf*CfagaagcaAfuGfaGfac cu*g | SE Q ID N O: 70 6 | 5896 |
| ds-n84 | ds-m84 | (invAB)*ggucucauUfGfCfuu cugacuag | SE Q ID N O: 49 7 | c*Uf*Af*GfucagaagCfaAfuGfag ac*c | SE Q ID N O: 70 7 | 5898 |
| ds-n85 | ds-m85 | (invAB)*caaacaccAfCfAfaca gcaguaa | SE Q ID N O: 49 8 | u*Uf*Af*CfugcuguuGfuGfgUfg uuu*g | SE Q ID N O: 70 8 | 6203 |
| ds-n86 | ds-m86 | (invAB)*ucaauggaUfUfCfaa gcauucua | SE Q ID N O: 49 9 | u*Af*Gf*AfaugcuugAfaUfcCfa uug*a | SE Q ID N O: 70 9 | 6236 |
| ds-n87 | ds-m87 | (invAB)*caauggauUfCfAfag cauucuaa | SE Q ID N O: *50* 0 | u*Uf*Af*GfaaugcuuGfaAfuCfc auu*g | SE Q ID N O: 71 0 | 6237 |
| ds-n88 | ds-m88 | (invAB)*uggauucaAfGfCfau ucuaagcu | SE Q ID N O: *50* 1 | a*Gf*Cf*UfuagaaugCfuUfgAfa ucc*a | SE Q ID N O: 71 1 | 6240 |
| ds-n89 | ds-m89 | (invAB)*ucaagcauUfCfUfaa gcuuuguu | SE Q ID N O: *50* 2 | a*Af*Cf*AfaagcuuaGfaAfuGfcu ug*a | SE Q ID N O: 71 2 | 6245 |
| ds-n90 | ds-m90 | (invAB)*cuucuucaUfGfGfgu cuuacagu | SE Q ID N O: 50 3 | a*Cf*Uf*GfuaagaccCfaUfgAfag aa*g | SE Q ID N O: 71 3 | 6288 |
| ds-n91 | ds-m91 | (invAB)*gucuuacaGfUfUfcu auguuaga | SE Q ID N O: 50 4 | u*Cf*Uf*AfacauagaAfcUfgUfaa ga*c | SE Q ID N O: 71 4 | 6299 |
| ds-n92 | ds-m92 | (invAB)*caguucuaUfGfUfua gaccauga | SE Q ID N O: 50 5 | u*Cf*Af*UfggucuaaCfaUfaGfaa cu*g | SE Q ID N O: 71 5 | 6305 |
| ds-n93 | ds-m93 | (invAB)*ucuauguuAfGfAfcc augaaaca | SE Q ID N O: 50 6 | u*Gf*Uf*UfucauggguCfuAfaCfa uag*a | SE Q ID N O: 71 6 | 6309 |
| ds-n94 | ds-m94 | (invAB)*cuauguuaGfAfCfca ugaaacau | SE Q ID N O: 50 7 | a*Uf*Gf*UfuucauggUfcUfaAfc aua*g | SE Q ID N O: 71 7 | 6310 |
| ds-n95 | ds-m95 | (invAB)*cgucuuuaAfUfGfuc acuuuuau | SE Q ID N O: 50 8 | a*Uf*Af*AfaagugacAfuUfaAfa gac*g | SE Q ID N O: 71 8 | 6343 |
| ds-n96 | ds-m96 | (invAB)*uaacuuuuUfUfAfcg guucagau | SE Q ID N O: 50 9 | a*Uf*Cf*UfgaaccguAfaAfaAfag uu*a | SE Q ID N O: 71 9 | 6363 |
| ds-n97 | ds-m97 | (invAB)*acuuuuuuAfCfGfgu ucagauau | SE Q ID N O: 51 0 | a*Uf*Af*UfcugaaccGfuAfaAfaa ag*u | SE Q ID N O: 72 0 | 6365 |
| ds-n98 | ds-m98 | (invAB)*cuuuuuuaCfGfGfuu cagauauu | SE Q ID N O: 51 1 | a*Af*Uf*AfucugaacCfgUfaAfaa aa*g | SE Q ID N O: 72 1 | 6366 |
| ds-n99 | ds-m99 | (invAB)*acgguucaGfAfUfau ucaucuau | SE Q ID N O: 51 2 | a*Uf*Af*GfaugaauaUfcUfgAfac cg*u | SE Q ID N O: 72 2 | 6373 |
| ds-n100 | ds-m100 | (invAB)*cagauauuCfAfUfcu auacgucu | SE Q ID N O: 51 3 | a*Gf*Af*CfguauagaUfgAfaUfa ucu*g | SE Q ID N O: 72 3 | 6379 |
| ds-n101 | ds-m101 | (invAB)*gauauucaUfCfUfau acgucugu | SE Q ID N O: 51 4 | a*Cf*Af*GfacguauaGfaUfgAfau au*c | SE Q ID N O: 72 4 | 6381 |
| ds-n102 | ds-m102 | (invAB)*guucuugaUfCfUfuu guggauuu | SE Q ID N O: 51 5 | a*Af*Af*UfccacaaaGfaUfcAfag aa*c | SE Q ID N O: 72 5 | 6431 |
| ds-n103 | ds-m103 | (invAB)*cuugaucuUfUfGfug gauuuaau | SE Q ID N O: 51 6 | a*Uf*Uf*AfaauccacAfaAfgAfuc aa*g | SE Q ID N O: 72 6 | 6434 |
| ds-n104 | ds-m104 | (invAB)*cuccaagaAfAfCfuu cuuaugcu | SE Q ID NO: 51 7 | a*Gf*Cf*AfuaagaagUfuUfcUfu gga*g | SE Q ID NO: 72 7 | 6496 |
| ds-n105 | ds-m105 | (invAB)*uguacuagAfGfUfgc gugacuuu | SE Q ID N O: 51 8 | a*Af*Af*GfucacgcaCfuCfuAfgu ac*a | SE Q ID N O: 72 8 | 6518 |
| ds-n106 | ds-m106 | (invAB)*cuguuacaGfUfGfca agacauga | SE Q ID N O: 51 9 | u*Cf*Af*UfgucuugcAfcUfgUfa aca*g | SE Q ID N O: 72 9 | 6755 |
| ds-n107 | ds-m107 | (invAB)*acagugcaAfGfAfca ugauacaa | SE Q ID N O: 52 0 | u*Uf*Gf*UfaucauguCfuUfgCfa cug*u | SE Q ID N O: 73 0 | 6760 |
| ds-n108 | ds-m108 | (invAB)*cuuagguuGfUfGfac acacauau | SE Q ID N O: 52 1 | a*Uf*Af*UfgugugucAfcAfaCfc uaa*g | SE Q ID N O: 73 1 | 6883 |
| ds-n109 | ds-m109 | (invAB)*uagguuguGfAfCfac acauauau | SE Q ID N O: 52 2 | a*Uf*Af*UfaugugugUfcAfcAfa ccu*a | SE Q ID N O: 73 2 | 6885 |
| ds-n110 | ds-m110 | (invAB)*gcagacacUfAfGfgc auuuggau | SE Q ID N O: 52 3 | a*Uf*Cf*CfaaaugccUfaGfuGfuc ug*c | SE Q ID N O: 73 3 | 6947 |
| ds-n111 | ds-m111 | (invAB)*cagacacuAfGfGfca uuuggauu | SE Q ID N O: 52 4 | a*Af*Uf*CfcaaaugcCfuAfgUfgu cu*g | SE Q ID N O: 73 4 | 6948 |
| ds-n112 | ds-m112 | (invAB)*agacacuaGfGfCfau uuggauua | SE Q ID N O: 52 5 | u*Af*Af*UfccaaaugCfcUfaGfug uc*u | SE Q ID N O: 73 5 | 6949 |
| ds-n113 | ds-m113 | (invAB)*cacuaggcAfUfUfug gauuacua | SE Q ID N O: 52 6 | u*Af*Gf*UfaauccaaAfuGfcCfua gu*g | SE Q ID N O: 73 6 | 6952 |
| ds-n114 | ds-m114 | (invAB)*acuaggcaUfUfUfgg auuacuau | SE Q ID N O: 52 7 | a*Uf*Af*GfuaauccaAfaUfgCfcu ag*u | SE Q ID N O: 73 7 | 6953 |
| ds-n115 | ds-m115 | (invAB)*cuaggcauUfUfGfga uuacuauu | SE Q ID N O: 52 8 | a*Af*Uf*AfguaauccAfaAfuGfcc ua*g | SE Q ID N O: 73 8 | 6954 |
| ds-n116 | ds-m116 | (invAB)*gguauuauAfUfGfua ggaguuu | SE Q ID N O: 52 9 | a*Af*Af*AfcuccuacAfuAfuAfa uac*c | SE Q ID N O: 73 9 | 7574 |
| ds-n117 | ds-m117 | (invAB)*ccaguuucAfAfUfca cuguagaa | SE Q ID N O: 53 0 | u*Uf*Cf*UfacagugaUfuGfaAfac ug*g | SE Q ID N O: 74 0 | 7621 |
| ds-n118 | ds-m118 | (invAB)*aucuaccuCfAfCfuc uuauuuuu | SE Q ID N O: 53 1 | a*Af*Af*AfauaagagUfgAfgGfu aga*u | SE Q ID N O: 74 1 | 7764 |
| ds-n119 | ds-m119 | (invAB)*ccugacaaUfAfGfgc cguugaua | SE Q ID N O: 53 2 | u*Af*Uf*CfaacggccUfaUfuGfuc ag*g | SE Q ID N O: 74 2 | 7830 |
| ds-n120 | ds-m120 | (invAB)*aggcuguaUfUfCfcg gggucaaa | SE Q ID N O: 53 3 | u*Uf*Uf*GfaccccggAfaUfaCfag cc*u | SE Q ID N O: 74 3 | 7906 |
| ds-n121 | ds-m121 | (invAB)*gguagacaGfAfGfau guaccaaa | SE Q ID N O: 53 4 | u*Uf*Uf*GfguacaucUfcUfgUfc uac*c | SE Q ID N O: 74 4 | 8540 |
| ds-n122 | ds-m122 | (invAB)*gggaucuuAfAfGfgu cauugaga | SE Q ID N O: 53 5 | u*Cf*Uf*CfaaugaccUfuAfaGfau cc*c | SE Q ID N O: 74 5 | 8836 |
| ds-n123 | ds-m123 | (invAB)*guaggugaCfCfCfcu uggaauaa | SE Q ID N O: 53 6 | u*Uf*Af*UfuccaaggGfgUfcAfc cua*c | SE Q ID N O: 74 6 | 8892 |
| ds-n124 | ds-m124 | (invAB)*gcugguuaUfAfAfgg guuuugu u | SE Q ID N O: 53 7 | a*Af*Cf*AfaaacccuUfaUfaAfcc ag*c | SE Q ID N O: 74 7 | 8975 |
| ds-n125 | ds-m125 | (invAB)*ugguuauaAfGfGfgu uuuguuua | SE Q ID N O: 53 8 | u*Af*Af*AfcaaaaccCfuUfaUfaa cc*a | SE Q ID N O: 74 8 | 8977 |
| ds-n126 | ds-m126 | (invAB)*cacccaccUfUfGfac uauaccaa | SE Q ID N O: 53 9 | u*Uf*Gf*GfuauagucAfaGfgUfg ggu*g | SE Q ID N O: 74 9 | 9163 |
| ds-n127 | ds-m127 | (invAB)*cuauaccaAfGfGfca ucaucuau | SE Q ID N O: 54 0 | a*Uf*Af*GfaugaugcCfuUfgGfu aua*g | SE Q ID N O: 75 0 | 9175 |
| ds-n128 | ds-m128 | (invAB)*caucaucuAfUfCfca caguucua | SE Q ID NO: 54 1 | u*Af*Gf*AfacuguggAfuAfgAfu gau*g | SE Q ID NO: 75 1 | 9186 |
| ds-n129 | ds-m129 | (invAB)*cuuaagcuGfAfGfuu guggcauu | SE Q ID N O: 54 2 | a*Af*Uf*GfccacaacUfcAfgCfuu aa*g | SE Q ID N O: 75 2 | 9265 |
| ds-n130 | ds-m130 | (invAB)*uaagcugaGfUfUfgu ggcauuuu | SE Q ID N O: 54 3 | a*Af*Af*AfugccacaAfcUfcAfgc uu*a | SE Q ID N O: 75 3 | 9267 |
| ds-n131 | ds-m131 | (invAB)*acugaggcAfCfAfuc auggaauu | SE Q ID N O: 54 4 | a*Af*Uf*UfccaugauGfuGfcCfuc ag*u | SE Q ID N O: 75 4 | 9340 |
| ds-n132 | ds-m132 | (invAB)*cacaucauGfGfAfau ugauguga | SE Q ID N O: 54 5 | u*Cf*Af*CfaucaauuCfcAfuGfau gu*g | SE Q ID N O: 75 5 | 9347 |
| ds-n133 | ds-m133 | (invAB)*cauggaauUfGfAfug ugagcauu | SE Q ID N O: 54 6 | a*Af*Uf*GfcucacauCfaAfuUfcc au*g | SE Q ID N O: 75 6 | 9352 |
| ds-n134 | ds-m134 | (invAB)*gaauugauGfUfGfag cauuaaga | SE Q ID N O: 54 7 | u*Cf*Uf*UfaaugcucAfcAfuCfaa uu*c | SE Q ID N O: 75 7 | 9356 |
| ds-n135 | ds-m135 | (invAB)*gggaugucAfUfGfac guuugaca | SE Q ID N O: 54 8 | u*Gf*Uf*CfaaacgucAfuGfaCfau cc*c | SE Q ID N O: 75 8 | 9478 |
| ds-n136 | ds-m136 | (invAB)*gaugucauGfAfCfgu uugacaua | SE Q ID N O: 54 9 | u*Af*Uf*GfucaaacgUfcAfuGfac au*c | SE Q ID N O: 75 9 | 9480 |
| ds-n137 | ds-m137 | (invAB)*gaaaagugGfCfUfuc guaaaaua | SE Q ID N O: 55 0 | u*Af*Uf*UfuuacgaaGfcCfaCfuu uu*c | SE Q ID N O: 76 0 | 9606 |
| ds-n138 | ds-m138 | (invAB)*guggcuucGfUfAfaa auagaaga | SE Q ID N O: 55 1 | u*Cf*Uf*UfcuauuuuAfcGfaAfg cca*c | SE Q ID N O: 76 1 | 9611 |
| ds-n139 | ds-m139 | (invAB)*agucacugUfGfGfaa cuaccaaa | SE Q ID N O: 55 2 | u*Uf*Uf*GfguaguucCfaCfaGfu gac*u | SE Q ID N O: 76 2 | 9634 |
| ds-n140 | ds-m140 | (invAB)*ggugcuuuGfGfGfau aaaagauu | SE Q ID N O: 55 3 | a*Af*Uf*CfuuuuaucCfcAfaAfgc ac*c | SE Q ID N O: 76 3 | 9680 |
| ds-n141 | ds-m141 | (invAB)*gugcuuugGfGfAfua aaagauuu | SE Q ID N O: 55 4 | a*Af*Af*UfcuuuuauCfcCfaAfag ca*c | SE Q ID N O: 76 4 | 9681 |
| ds-n142 | ds-m142 | (invAB)*ccagauucUfAfGfgc caguuugu | SE Q ID N O: 55 5 | a*Cf*Af*AfacuggccUfaGfaAfuc ug*g | SE Q ID N O: 76 5 | 9724 |
| ds-n143 | ds-m143 | (invAB)*cagauucuAfGfGfcc aguuuguu | SE Q ID N O: 55 6 | a*Af*Cf*AfaacuggcCfuAfgAfau cu*g | SE Q ID N O: 76 6 | 9725 |
| ds-n144 | ds-m144 | (invAB)*guggcaagAfUfCfac acugagau | SE Q ID N O: 55 7 | a*Uf*Cf*UfcagugugAfuCfuUfg cca*c | SE Q ID N O: 76 7 | 9814 |
| ds-n145 | ds-m145 | (invAB)*ggggguuugUfUfCfu uuucguuaa | SE Q ID N O: 55 8 | u*Uf*Af*AfcgaaaagAfaCfaAfac cc*c | SE Q ID N O: 76 8 | 10015 |
| ds-n146 | ds-m146 | (invAB)*ggguuuguUfCfUfu uucguuaau | SE Q ID N O: 55 9 | a*Uf*Uf*AfacgaaaaGfaAfcAfaa cc*c | SE Q ID N O: 76 9 | 10016 |
| ds-n147 | ds-m147 | (invAB)*gauguuauUfUfAfga cugaaccu | SE Q ID N O: 56 0 | a*Gf*Gf*UfucagucuAfaAfuAfa cau*c | SE Q ID N O: 77 0 | 10189 |
| ds-n148 | ds-m148 | (invAB)*acaggucaGfAfGfgg uuucauuu | SE Q ID N O: 56 1 | a*Af*Af*UfgaaacccUfcUfgAfcc ug*u | SE Q ID N O: 77 1 | 10244 |
| ds-n149 | ds-m149 | (invAB)*uggccuucAfUfCfuu agaugacu | SE Q ID N O: 56 2 | a*Gf*Uf*CfaucuaagAfuGfaAfg gcc*a | SE Q ID N O: 77 2 | 10266 |
| ds-n150 | ds-m150 | (invAB)*cauuugguUfUfCfaa caaagaaa | SE Q ID N O: 56 3 | u*Uf*Uf*CfuuuguugAfaAfcCfa aau*g | SE Q ID N O: 77 3 | 10490 |
| ds-n151 | ds-m151 | (invAB)*uggaaacuGfAfUfcu cggaguua | SE Q ID N O: 56 4 | u*Af*Af*CfuccgagaUfcAfgUfu ucc*a | SE Q ID N O: 77 4 | 10526 |
| ds-n152 | ds-m152 | (invAB)*gaaacugaUfCfUfcg gaguuaag | SE Q ID NO: 56 5 | c*Uf*Uf*AfacuccgaGfaUfcAfgu uu*c | SE Q ID NO: 77 5 | 10528 |
| ds-n153 | ds-m153 | (invAB)*gaucucggAfGfUfua aggcgaau | SE Q ID N O: 56 6 | a*Uf*Uf*CfgccuuaaCfuCfcGfag au*c | SE Q ID N O: 77 6 | 10534 |
| ds-n154 | ds-m154 | (invAB)*aucucggaGfUfUfaa ggcgaauu | SE Q ID N O: 56 7 | a*Af*Uf*UfcgccuuaAfcUfcCfga ga*u | SE Q ID N O: 77 7 | 10535 |
| ds-n155 | ds-m155 | (invAB)*cucggaguUfAfAfgg cgaauugu | SE Q ID N O: 56 8 | a*Cf*Af*AfuucgccuUfaAfcUfcc ga*g | SE Q ID N O: 77 8 | 10537 |
| ds-n156 | ds-m156 | (invAB)*ggaguuaaGfGfCfga auuguuca | SE Q ID N O: 56 9 | u*Gf*Af*AfcaauucgCfcUfuAfac uc*c | SE Q ID N O: 77 9 | 10540 |
| ds-n157 | ds-m157 | (invAB)*gaguuaagGfCfGfaa uuguucaa | SE Q ID N O: 57 0 | u*Uf*Gf*AfacaauucGfcCfuUfaa cu*c | SE Q ID N O: 78 0 | 10541 |
| ds-n158 | ds-m158 | (invAB)*cuuucuggUfCfAfac auuguuuu | SE Q ID N O: 57 1 | a*Af*Af*AfcaauguuGfaCfcAfga aa*g | SE Q ID N O: 78 1 | 10679 |
| ds-n159 | ds-m159 | (invAB)*uguuuuaaCfUfAfgu cacucauu | SE Q ID N O: 57 2 | a*Af*Uf*GfagugacuAfgUfuAfa aac*a | SE Q ID N O: 78 2 | 10694 |
| ds-n160 | ds-m160 | (invAB)*cguuuucaAfUfAfgg gcucuuaa | SE Q ID N O: 57 3 | u*Uf*Af*AfgagcccuAfuUfgAfa aac*g | SE Q ID N O: 78 3 | 10717 |
| ds-n161 | ds-m161 | (invAB)*gggcucuuAfAfGfuc caguagau | SE Q ID N O: 57 4 | a*Uf*Cf*UfacuggacUfuAfaGfag cc*c | SE Q ID N O: 78 4 | 10728 |
| ds-n162 | ds-m162 | (invAB)*ggcucuuaAfGfUfcc aguagauu | SE Q ID N O: 57 5 | a*Af*Uf*CfuacuggaCfuUfaAfga gc*c | SE Q ID N O: 78 5 | 10729 |
| ds-n163 | ds-m163 | (invAB)*gcucuuaaGfUfCfca guagauua | SE Q ID N O: 57 6 | u*Af*Af*UfcuacuggAfcUfuAfa gag*c | SE Q ID N O: 78 6 | 10730 |
| ds-n164 | ds-m164 | (invAB)*guagucagUfUfGfac gaagaucu | SE Q ID N O: 57 7 | a*Gf*Af*UfcuucgucAfaCfuGfac ua*c | SE Q ID N O: 78 7 | 10754 |
| ds-n165 | ds-m165 | (invAB)*ugacgaagAfUfCfug guuuacaa | SE Q ID N O: 57 8 | u*Uf*Gf*UfaaaccagAfuCfuUfcg uc*a | SE Q ID N O: 78 8 | 10763 |
| ds-n166 | ds-m166 | (invAB)*cgaagaucUfGfGfuu uacaagaa | SE Q ID N O: 57 9 | u*Uf*Cf*UfuguaaacCfaGfaUfcu uc*g | SE Q ID N O: 78 9 | 10766 |
| ds-n167 | ds-m167 | (invAB)*accuguuuAfCfUfag cuagcuuu | SE Q ID N O: 58 0 | a*Af*Af*GfcuagcuaGfuAfaAfca gg*u | SE Q ID N O: 79 0 | 10942 |
| ds-n168 | ds-m168 | (invAB)*cccuaguuUfAfUfaa caaaggaa | SE Q ID N O: 58 1 | u*Uf*Cf*CfuuuguuaUfaAfaCfu agg*g | SE Q ID N O: 79 1 | 11034 |
| ds-n169 | ds-m169 | (invAB)*cuguaucuUfCfAfgu aucuuggu | SE Q ID N O: 58 2 | a*Cf*Cf*AfagauacuGfaAfgAfua ca*g | SE Q ID N O: 79 2 | 11078 |
| ds-n170 | ds-m170 | (invAB)*gaucauuuUfUfUfac uggucauu | SE Q ID N O: 58 3 | a*Af*Uf*GfaccaguaAfaAfaAfug au*c | SE Q ID N O: 79 3 | 11126 |
| ds-n171 | ds-m171 | (invAB)*aguguagcUfAfCfuu uaacagau | SE Q ID N O: 58 4 | a*Uf*Cf*UfguuaaagUfaGfcUfac ac*u | SE Q ID N O: 79 4 | 11156 |
| ds-n172 | ds-m172 | (invAB)*guagcuacUfUfUfaa cagaugga | SE Q ID N O: 58 5 | u*Cf*Cf*AfucuguuaAfaGfuAfg cua*c | SE Q ID N O: 79 5 | 11159 |
| ds-n173 | ds-m173 | (invAB)*uagcuacuUfUfAfac agauggaa | SE Q ID N O: 58 6 | u*Uf*Cf*CfaucuguuAfaAfgUfa gcu*a | SE Q ID N O: 79 6 | 11160 |
| ds-n174 | ds-m174 | (invAB)*uggacaugGfGfAfag acuugacu | SE Q ID N O: 58 7 | a*Gf*Uf*CfaagucuuCfcCfaUfgu cc*a | SE Q ID N O: 79 7 | 11292 |
| ds-n175 | ds-m175 | (invAB)*acagcauaCfAfCfag ugaucaca | SE Q ID N O: 58 8 | u*Gf*Uf*GfaucacugUfgUfaUfg cug*u | SE Q ID N O: 79 8 | 11340 |
| ds-n176 | ds-m176 | (invAB)*cauacacaGfUfGfau cacauaaa | SE Q ID NO: 58 9 | u*Uf*Uf*AfugugaucAfcUfgUfg uau*g | SE Q ID NO: 79 9 | 11344 |
| ds-n177 | ds-m177 | (invAB)*acacaggcCfAfUfuu gcuuacau | SE Q ID N O: 59 0 | a*Uf*Gf*UfaagcaaaUfgGfcCfug ug*u | SE Q ID N O: 80 0 | 11383 |
| ds-n178 | ds-m178 | (invAB)*ugcuuacaUfGfCfcu cguaucau | SE Q ID N O: 59 1 | a*Uf*Gf*AfuacgaggCfaUfgUfaa gc*a | SE Q ID N O: 80 1 | 11395 |
| ds-n179 | ds-m179 | (invAB)*gagguuugCfCfAfga guuugucu | SE Q ID N O: 59 2 | a*Gf*Af*CfaaacucuGfgCfaAfac cu*c | SE Q ID N O: 80 2 | 11789 |
| ds-n180 | ds-m180 | (invAB)*ggaauggaUfGfCfac cgcaaaua | SE Q ID N O: 59 3 | u*Af*Uf*UfugcggugCfaUfcCfa uuc*c | SE Q ID N O: 80 3 | 11864 |
| ds-n181 | ds-m181 | (invAB)*gaauggauGfCfAfcc gcaaauaa | SE Q ID N O: 59 4 | u*Uf*Af*UfuugcgguGfcAfuCfc auu*c | SE Q ID N O: 80 4 | 11865 |
| ds-n182 | ds-m182 | (invAB)*aagguaauAfAfCfau ggccaauu | SE Q ID N O: 59 5 | a*Af*Uf*UfggccaugUfuAfuUfa ccu*u | SE Q ID N O: 80 5 | 11939 |
| ds-n183 | ds-m183 | (invAB)*uauacucuGfGfAfuu cuuuacau | SE Q ID N O: 59 6 | a*Uf*Gf*UfaaagaauCfcAfgAfgu au*a | SE Q ID N O: 80 6 | 12092 |
| ds-n184 | ds-m184 | (invAB)*ggaaaaaaGfAfAfac ugucuauu | SE Q ID N O: 59 7 | a*Af*Uf*AfgacaguuUfcUfuUfu uuc*c | SE Q ID N O: 80 7 | 12116 |
| ds-n185 | ds-m185 | (invAB)*gucuauuuUfGfAfau ggcugaag | SE Q ID N O: 59 8 | c*Uf*Uf*CfagccauuCfaAfaAfua ga*c | SE Q ID N O: 80 8 | 12130 |
| ds-n186 | ds-m186 | (invAB)*aggcaacgUfUfAfgu uucucuua | SE Q ID N O: 59 9 | u*Af*Af*GfagaaacuAfaCfgUfu gcc*u | SE Q ID N O: 80 9 | 12154 |
| ds-n187 | ds-m187 | (invAB)*gcaacguuAfGfUfuu cucuuacu | SE Q ID N O: 60 0 | a*Gf*Uf*AfagagaaaCfuAfaCfgu ug*c | SE Q ID N O: 81 0 | 12156 |
| ds-n188 | ds-m188 | (invAB)*cugcuuuuUfUfCfua guaaagua | SE Q ID N O: 60 1 | u*Af*Cf*UfuuacuagAfaAfaAfa gca*g | SE Q ID N O: 81 1 | 12177 |
| ds-n189 | ds-m189 | (invAB)*guacuacaUfGfGfuu uaaguuaa | SE Q ID N O: 60 2 | u*Uf*Af*AfcuuaaacCfaUfgUfag ua*c | SE Q ID N O: 81 2 | 12195 |
| ds-n190-1 | ds-m190 | a*a*cgacuaUfCfAfgcagcuGf a*a*u | SE Q ID N O: 60 3 | (M06)*CfGfaa*UfucaGfcugcuga UfaGfucguu*g*c | SE Q ID N O: 81 3 | 1164 |
| ds-n191-1 | ds-m191 | c*a*acgacuAfUfCfagcagcUf g*a*a | SE Q ID N O: 60 4 | (M06)*CfGfau*UfcagCfugcugau AfgUfcguug*c*g | SE Q ID N O: 81 4 | 1163 |
| ds-n192-1 | ds-m192 | g*c*aacgacUfAfUfcagcagCf u*g*a | SE Q ID N O: 60 5 | (M06)*CfGfau*CfagcUfgcugaua GfuCfguugc*g*g | SE Q ID N O: 81 5 | 1162 |
| ds-n193-1 | ds-m193 | c*g*caacgaCfUfAfucagcaGf c*u*u | SE Q ID N O: 60 6 | (M06)*CfGfaa*AfgcuGfcugauag UfcGfuugcg*g*a | SE Q ID N O: 81 6 | 1161 |
| ds-n194-1 | ds-m194 | c*c*gcaacgAfCfUfaucagcAf g*c*u | SE Q ID N O: 60 7 | (M06)*CfGfaa*GfcugCfugauagu CfgUfugcgg*a*u | SE Q ID N O: 81 7 | 1160 |
| ds-n195-1 | ds-m195 | a*c*gacuauCfAfGfcagcugAf a*g*u | SE Q ID N O: 60 8 | (M06)*CfGfaa*CfuucAfgcugcug AfuAfgucgu*u*g | SE Q ID N O: 81 8 | 1165 |
| ds-n196-1 | ds-m196 | c*g*acuaucAfGfCfagcugaAf g*c*u | SE Q ID N O: 60 9 | (M06)*CfGfaa*GfcuuCfagcugcu GfaUfagucg*u*u | SE Q ID N O: 81 9 | 1166 |
| ds-n197-1 | ds-m197 | g*a*cuaucaGfCfAfgcugaaGf c*g*u | SE Q ID N O: 61 0 | (M06)*CfGfaa*CfgcuUfcagcugc UfgAfuaguc*g*u | SE Q ID N O: 82 0 | 1167 |
| ds-n198-1 | ds-m198 | a*c*uaucagCfAfGfcugaagCf g*c*u | SE Q ID N O: 61 1 | (M06)*CfGfaa*GfcgcUfucagcug CfuGfauagu*c*g | SE Q ID N O: 82 1 | 1168 |
| ds-n190-1 | ds-m199 | a*a*c*g*acuaUfCfAfgcagcu Gfa*a*u | SE Q ID N O: 61 2 | (invAB)*CfGfaa*Ufucagcugcug aUfaGfucguu*g*c | SE Q ID N O: 82 2 | 1164 |
| ds-n198-1 | ds-m200 | a*c*u*a*ucagCfAfGfcugaag Cfg*c*u | SE Q ID NO: 61 3 | (invAB)*CfGfaa*Gfcgcuucagcu gCfuGfauagu*c*g | SE Q ID NO: 82 3 | 1168 |
| ds-n201-1 | ds-m201 | a*u*u*u*gcauAfCfAfcaucgu Cfu*u*u | SE Q ID N O: 61 4 | (invAB)*CfGfaa*Afagacgaugug uAfuGfcaaau*g*u | SE Q ID N O: 82 4 | 6329 |
| ds-n202-1 | ds-m202 | c*u*u*u*uuuaCfGfGfuucaga Ufa*u*u | SE Q ID N O: 61 5 | (invAB)*CfGfaa*Afuaucugaacc gUfaAfaaaag*u*u | SE Q ID N O: 82 5 | 6366 |
| ds-n203-1 | ds-m203 | a*c*g*g*uucaGfAfUfauucau Cfu*a*u | SE Q ID N O: 61 6 | (invAB)*CfGfaa*Ufagaugaauau cUfgAfaccgu*a*a | SE Q ID N O: 82 6 | 6373 |
| ds-n204-1 | ds-m204 | c*u*u*g*aucuUfUfGfuggau uUfa*a*u | SE Q ID N O: 61 7 | (invAB)*CfGfaa*Ufuaaauccacaa AfgAfucaag*a*a | SE Q ID N O: 82 7 | 6434 |
| ds-n190-1 | Z1 | [D02]*a*a*c*g*acuaUfCfAf gcagcuGfa*a*u | SE Q ID N O: 61 8 | (invAB)*CfGfaa*Ufucagcugcug aUfaGfucguu*g*c | SE Q ID N O: 82 2 | 1164 |
| ds-n198-1 | Z2 | [D02]*a*c*u*a*ucagCfAfGf cugaagCfg*c*u | SE Q ID N O: 61 9 | (invAB)*CfGfaa*Gfcgcuucagcu gCfuGfauagu*c*g | SE Q ID N O: 82 3 | 1168 |
| ds-n201-1 | Z3 | [D02]*a*u*u*u*gcauAfCfAf caucguCfu*u*u | SE Q ID N O: 62 0 | (invAB)*CfGfaa*Afagacgaugug uAfuGfcaaau*g*u | SE Q ID N O: 82 4 | 6329 |
| ds-n202-1 | Z4 | [D02]*c*u*u*u*uuuaCfGfGf uucagaUfa*u*u | SE Q ID N O: 62 1 | (invAB)*CfGfaa*Afuaucugaacc gUfaAfaaaag*u*u | SE Q ID N O: 82 5 | 6366 |
| ds-n203-1 | Z5 | [D02]*a*c*g*g*uucaGfAfUf auucauCfu*a*u | SE Q ID N O: 62 2 | (invAB)*CfGfaa*Ufagaugaauau cUfgAfaccgu*a*a | SE Q ID N O: 82 6 | 6373 |
| ds-n204-1 | Z6 | [D02]*c*u*u*g*aucuUfUfGf uggauuUfa*a*u | SE Q ID N O: 62 3 | (invAB)*CfGfaa*Ufuaaauccacaa AfgAfucaag*a*a | SE Q ID N O: 82 7 | 6434 |

wherein the lowercase letters "g", "c", "a", and "u" denote 2'-methoxy modified nucleotides; the uppercase letters "Gf", "Cf", "Af", and "Uf" denote 2'-fluoro modified nucleotides; and "*" indicates a phosphorothioate linkage between the two nucleotides adjacent to the "*"." (D02)*, (InvAB)*, (M06)*, *(D02), *(InvAB), and *(M06) indicate that D02, invAB, and M06 are linked to nucleotides via a phosphorothioate bond. invAB refers to an inverted abasic nucleotide bonded to the 5' or 3' end of a nucleotide. (M06) refers to a structure bonded to the 5' or 3' end of a monomer (e.g., a nucleotide) as described below:
(M06) 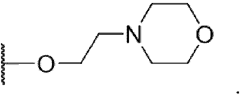 It may be linked to a nucleotide via a phosphate or a phosphorothioate bond. [D02] refers to a structure bonded to the 5' or 3' end as described below: 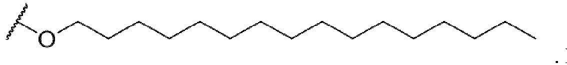 It may be linked to a nucleotide via a phosphate or a phosphorothioate bond. Taking 5'-[D02]*A-3' as an example, in certain embodiments, its structure is:

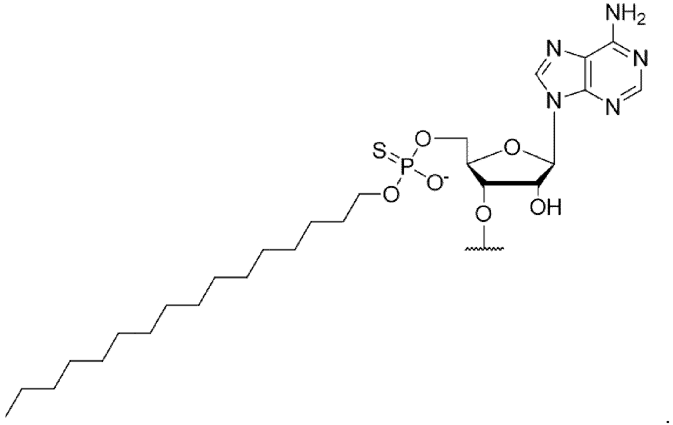

### RNA inhibitor conjugated with a ligand

Another aspect of the RNA inhibitors disclosed herein relates to conjugating interfering nucleic acids with ligands to enhance the stability, activity, cellular distribution, or cellular uptake of the RNAi agents.

In certain embodiments, the distribution, targeting, or stability of the RNA inhibitor is altered by introducing a ligand for receptors in the target tissue. For example, compared to species lacking the ligand, a specific ligand can provide enhanced affinity for a selected target (e.g., a molecule, cell or cell type, compartment (e.g., cellular or organ compartment, body tissue, organ, or region)).

Ligands may include naturally occurring substances such as proteins (e.g., human serum albumin (HSA), low-density lipoprotein (LDL), or globulins); carbohydrates (e.g., dextran, schizophyllan, chitin, chitosan, inulin, cyclodextrin, N-acetylglucosamine, N-acetylgalactosamine, or hyaluronic acid); or lipids. The ligand may also be a recombinant or synthetic molecule, such as a synthetic polymer, for example, a synthetic polyamino acid.

The ligand may also include targeting moieties, such as cell- or tissue-targeting agents that bind to specified cell types, e.g., renal cells, such as lectins, glycoproteins, lipids, or proteins, e.g., antibodies. Targeting moieties may be thyroid-stimulating hormone, melanocyte-stimulating hormone, lectins, glycoproteins, surfactant protein A, mucin carbohydrates, multivalent lactose, multivalent galactose, N-acetyl-galactosamine, N-acetyl-glucosamine, multivalent mannose, multivalent fucose, glycosylated polyamino acids, multivalent galactose, transferrin, bisphosphonates, polyglutamic acid, polyaspartic acid, lipids, cholesterol, steroids, bile acids, folic acid, vitamin B12, vitamin A, biotin, or RGD peptides or RGD peptide mimetics. In certain embodiments, the ligand is multivalent galactose, for example, N-acetyl-galactosamine.

The sense strand and antisense strand of the RNA inhibitors disclosed herein can be conveniently and routinely prepared using well-known solid-phase synthesis techniques. Alternatively or additionally, any other method known in the art for such synthesis, such as liquid-phase synthesis or fermentation, may be used. Similar techniques for preparing other oligonucleotides (e.g., phosphorothioates and alkylated derivatives) are also known.

In certain embodiments, in addition to commercially available and standard nucleoside phosphoramidite monomers as well as non-standard nucleoside phosphoramidite monomers routinely used in oligonucleotide synthesis, the oligonucleotides or linked nucleotides disclosed herein can be synthesized using an automated synthesizer via a phosphoramidite approach employing phosphoramidite monomers derived from ligand-nucleoside phosphoramidites.

In certain embodiments, the ligand conjugation disclosed herein is achieved by coupling the ligand structure to the 5' and/or 3' terminus of the antisense strand and/or to the 5' and/or 3' terminus of the sense strand.

For example, the ligand structure may be coupled to the 5' and/or 3' terminus of the sense strand; or the ligand structure may be coupled to the 5' terminus of the antisense strand and the ligand structure coupled to the 3' terminus of the sense strand; or the ligand structure may be coupled to the 3' terminus of the antisense strand and the ligand coupled to the 5' terminus of the sense strand; or the ligand structure may be coupled to both the 5' and 3' termini of the sense strand; or the ligand structure may be coupled to the 3' terminus of the sense strand.

In certain embodiments, the ligand disclosed herein is [L96], as shown in the following structural formula:

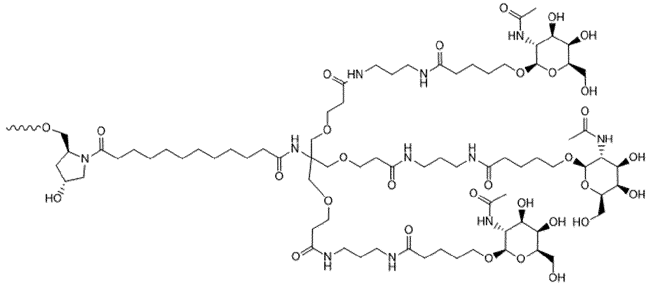

In certain embodiments, the ligand disclosed herein is [D02], as shown in the following structural formula:

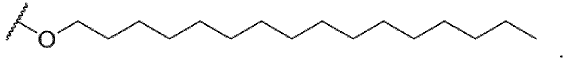

In the present invention, [D02] represents the residue of the D02 monomer. In certain embodiments, the D02 monomer structure is 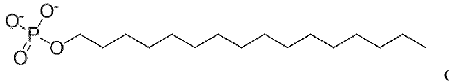 or 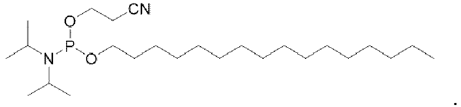 In certain embodiments, [D02] is bonded to the nucleotide via 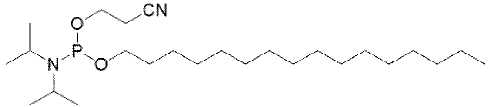 bond.

Taking 5'-[D02]*A-3' as an example, in certain embodiments, its structure is:

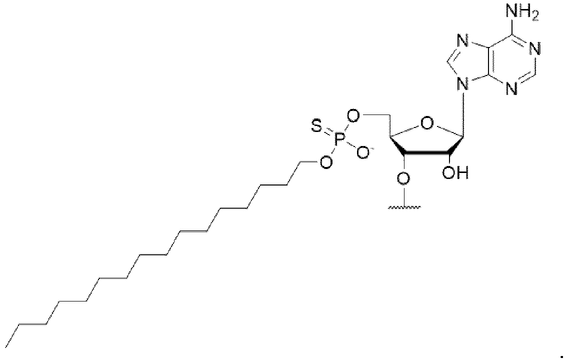

### Pharmaceutical composition

The present application also includes pharmaceutical compositions comprising the RNA inhibitors disclosed herein or pharmaceutically acceptable salts thereof.

In one embodiment, the present application provides a pharmaceutical composition comprising the RNA inhibitor described herein and a pharmaceutically acceptable excipient.

Pharmaceutical compositions comprising the RNA inhibitor can be used for preventing and/or treating IGF-1R-related disorders, for example, thyroid eye disease, osteoarthritis, and neuropathic pain. Such pharmaceutical compositions are formulated according to the mode of delivery. One exemplary regimen involves formulating a composition for parenteral systemic administration, for example, subcutaneous (SC), intramuscular (IM), or intravenous (IV) delivery. The pharmaceutical compositions of the present application can be administered at a dose sufficient to inhibit IGF-1R gene expression.

A pharmaceutically acceptable "excipient" or "carrier" is a pharmaceutically acceptable solvent, suspending agent, or any other pharmaceutically inert vehicle used for delivering one or more nucleic acids to an animal. The excipient may be liquid or solid and is selected considering the intended route of administration to provide the desired volume, consistency, etc., when combined with the nucleic acid and other components in the given pharmaceutical composition. The RNA inhibitor may be delivered in a manner targeting specific tissues (e.g., hepatocytes).

In certain embodiments, the pharmaceutical composition further comprises a delivery vehicle (e.g., nanoparticles, dendrimers, polymers, liposomes, or cationic delivery systems).

In certain embodiments, the delivery vehicle comprises liposomes.

In certain embodiments, the delivery vehicle comprises nanolipids capable of forming liposome-nucleic acid nanoparticles with nucleic acid molecules.

### Use

In another aspect, the present application provides the use of the aforementioned RNA inhibitor for inhibiting IGF-1R gene expression, or a pharmaceutically acceptable salt thereof, and the aforementioned pharmaceutical composition in the manufacture of a medicament for preventing or treating a disease or pathology or reducing the risk of a disease or pathology.

In certain embodiments, the disease or pathology includes diseases or pathologies associated with normal or elevated levels of IGF-1R.

In certain embodiments, the disease or pathology includes thyroid eye disease, osteoarthritis, and neuropathic pain.

In another aspect, the present application provides a method for preventing or treating a disease, disorder, or syndrome, the method comprising administering to a subject in need thereof an effective amount of the aforementioned RNA inhibitor for inhibiting IGF-1R gene expression, a pharmaceutically acceptable salt thereof, or the aforementioned pharmaceutical composition.

In certain embodiments, the RNA inhibitor for inhibiting IGF-1R gene expression, a pharmaceutically acceptable salt thereof, or the pharmaceutical composition is administered to the subject via intraorbital injection, intra-articular injection, intrathecal injection, subcutaneous administration, intravenous administration, oral administration, rectal administration, or intraperitoneal administration.

In certain embodiments, the method comprises administering to the local or lesion area tissue of a subject in need.

In certain embodiments, the local or lesion area tissue of the subject in need includes ocular tissue, joint tissue, central nervous tissue, peripheral nervous tissue, tumors, liver tissue, kidney tissue, muscle tissue, or adipose tissue.

In certain embodiments, the ocular tissue is the optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.

In certain embodiments, the ocular tissue is retinal ganglion, endothelial cell, extraocular muscle, or periorbital fat.

In another aspect, the present application provides a method for inhibiting IGF-1R mRNA or gene expression in a cell, tissue, or subject, comprising administering to a subject in need thereof an effective amount of an RNA inhibitor that inhibits IGF-1R gene expression as described above, a pharmaceutically acceptable salt thereof, or the pharmaceutical composition described above.

Cells suitable for treatment by the methods of the present application may be any cells expressing the IGF-1R gene. Cells suitable for use in the methods of the present application may be mammalian cells, wherein, upon contact with cells expressing the IGF-1R gene, the RNAi agent inhibits IGF-1R gene expression (e.g., human, primate, non-primate, or rat IGF-1R gene) by at least about 50%, as determined, for example, by PCR or branched DNA (bDNA)-based methods, or by protein-based methods such as immunofluorescence assay, Western blotting, or flow cytometry.

The term "inhibit" as used herein may be used interchangeably with "reduce," "decrease," "silence," "downregulate," "suppress," and other similar terms, and encompasses any level of inhibition. IGF-1R gene expression may be evaluated based on the level or change in level of any variable associated with IGF-1R gene expression, for example, IGF-1R mRNA levels or IGF-1R protein levels. This level may be analyzed in a single cell or a population of cells (including, for example, a sample derived from a subject). Inhibition may be evaluated by an absolute or relative decrease in the level of one or more variables associated with IGF-1R gene expression compared to a control level. The control level may be any type of control level employed in the art, for example, a baseline level prior to administration, or a level measured in a comparable subject, cell, or sample that has not been treated or has been treated with a control (such as, for example, a buffer-only control or an inactive agent control).

Inhibition of IGF-1R gene expression may be manifested by a reduction in the amount of mRNA produced by a first cell or population of cells (which may, for example, be present in a sample derived from a subject), wherein the IGF-1R gene has been transcribed and processed (e.g., by contacting one or more cells with the RNA inhibitor of the present application, or by administering the RNA inhibitor of the present application to a subject in which such cells are present), as compared to a second cell or population of cells that are substantially identical to the first cell or population of cells but have not been so treated (control cells not treated with the RNA inhibitor or not treated with an RNA inhibitor targeting the gene of interest). In preferred embodiments, inhibition is evaluated in a cell line with high IGF-1R gene expression using an appropriate concentration of siRNA according to the methods provided in the examples, and the mRNA level in the treated cells is expressed as a percentage of the mRNA level in untreated control cells.

In other embodiments, inhibition of IGF-1R gene expression may be evaluated by a reduction in a parameter functionally associated with IGF-1R gene expression, for example, IGF-1R protein levels in the blood or serum of a subject. IGF-1R gene silencing may be assessed in any cell expressing IGF-1R (endogenously or exogenously from a gene expression construct) using any assay known in the art.

Inhibition of IGF-1R protein expression may be manifested by a reduction in IGF-1R protein levels in a cell, population of cells, or subject sample (e.g., protein levels in a blood sample derived from a subject). As described above for mRNA inhibition evaluation, inhibition of protein expression levels in treated cells or populations of cells may similarly be expressed as a percentage of protein levels in control cells or populations of cells, or as a change in protein levels in a subject sample (e.g., blood or serum derived therefrom).

Control cells, populations of cells, or subject samples suitable for evaluating IGF-1R gene inhibition include cells, populations of cells, or subject samples that have not been contacted with the RNAi agent of the present application. For example, control cells, populations of cells, or subject samples may be derived from a single subject (e.g., a human or animal subject) prior to treatment with the RNAi agent or from an appropriately matched cohort control.

IGF-1R levels expressed by a cell or population of cells may be determined using any method known in the art for evaluating mRNA gene expression. For example, qRT-PCR is used to evaluate the reduction in gene expression. Reduction in protein production may be evaluated by any method known in the art, for example, ELISA. In certain embodiments, a percutaneous liver biopsy sample is used as tissue material for monitoring the reduction in IGF-1R gene or protein expression. In other embodiments, a blood sample is used as a subject sample for monitoring the reduction in IGF-1R protein expression.

Without being bound by any theory, the examples below are provided solely to illustrate the fusion proteins, preparation methods, and uses of the present application, and are not intended to limit the scope of the invention disclosed herein.

The present application provides the following embodiments:
1. An RNA inhibitor for inhibiting insulin-like growth factor 1 receptor (IGF-1R) gene expression, comprising an antisense strand that forms a complementary region with at least 15 consecutive nucleotides in the mRNA encoding IGF-1R (SEQ ID NO: 828), wherein the complementary region has 0, 1, 2, 3, 4, or 5 mismatches; preferably, the complementary region is 15-30 nucleotide pairs in length, more preferably 17-23 nucleotide pairs.
2. The RNA inhibitor according to embodiment 1, wherein the antisense strand forms a complementary region with any of the following sequences starting from the 5' end of the mRNA encoding IGF-1R (SEQ ID NO: 828): 15, 16, 17, 18, 19, 20, 21, 22, or 23 consecutive nucleotides beginning at:
   nucleotide 1118, nucleotide 1120, nucleotide 1154, nucleotide 1367, nucleotide 1407, nucleotide 1415, nucleotide 1532, nucleotide 1625, nucleotide 1627, nucleotide 1628, nucleotide 1631, nucleotide 3356, nucleotide 3357, nucleotide 3359, nucleotide 3792, nucleotide 4198, nucleotide 4200, nucleotide 4208, nucleotide 4685, nucleotide 5246, nucleotide 5392, nucleotide 5393, nucleotide 6329, nucleotide 6332, nucleotide 6333, nucleotide 6334, nucleotide 10215, nucleotide 10738, nucleotide 10740, nucleotide 10770, nucleotide 10772, nucleotide 10773, nucleotide 10946, nucleotide 10956, nucleotide 10957, nucleotide 1418, nucleotide 1541, nucleotide 2050, nucleotide 2055, nucleotide 2056, nucleotide 2233, nucleotide 2234, nucleotide 2456, nucleotide 2591, nucleotide 2603, nucleotide 2606, nucleotide 2609, nucleotide 2776, nucleotide 2890, nucleotide 2906, nucleotide 2909, nucleotide 2957, nucleotide 2960, nucleotide 3008, nucleotide 3195, nucleotide 3398, nucleotide 3608, nucleotide 3609, nucleotide 3650, nucleotide 3660, nucleotide 3815, nucleotide 3882, nucleotide 3996, nucleotide 4067, nucleotide 4121, nucleotide 4122, nucleotide 4292, nucleotide 4293, nucleotide 4295, nucleotide 4516, nucleotide 4519, nucleotide 5163, nucleotide 5309, nucleotide 5310, nucleotide 5377, nucleotide 5380, nucleotide 5381, nucleotide 5663, nucleotide 5700, nucleotide 5711, nucleotide 5712, nucleotide 5721, nucleotide 5896, nucleotide 5898, nucleotide 6203, nucleotide 6236, nucleotide 6237, nucleotide 6240, nucleotide 6245, nucleotide 6288, nucleotide 6299, nucleotide 6305, nucleotide 6309, nucleotide 6310, nucleotide 6343, nucleotide 6363, nucleotide 6365, nucleotide 6366, nucleotide 6373, nucleotide 6379, nucleotide 6381, nucleotide 6431, nucleotide 6434, nucleotide 6496, nucleotide 6518, nucleotide 6755, nucleotide 6760, nucleotide 6883, nucleotide 6885, nucleotide 6947, nucleotide 6948, nucleotide 6949, nucleotide 6952, nucleotide 6953, nucleotide 6954, nucleotide 7574, nucleotide 7621, nucleotide 7764, nucleotide 7830, nucleotide 7906, nucleotide 8540, nucleotide 8836, nucleotide 8892, nucleotide 8975, nucleotide 8977, nucleotide 9163, nucleotide 9175, nucleotide 9186, nucleotide 9265, nucleotide 9267, nucleotide 9340, nucleotide 9347, nucleotide 9352, nucleotide 9356, nucleotide 9478, nucleotide 9480, nucleotide 9606, nucleotide 9611, nucleotide 9634, nucleotide 9680, nucleotide 9681, nucleotide 9724, nucleotide 9725, nucleotide 9814, nucleotide 10015, nucleotide 10016, nucleotide 10189, nucleotide 10244, nucleotide 10266, nucleotide 10490, nucleotide 10526, nucleotide 10528, nucleotide 10534, nucleotide 10535, nucleotide 10537, nucleotide 10540, nucleotide 10541, nucleotide 10679, nucleotide 10694, nucleotide 10717, nucleotide 10728, nucleotide 10729, nucleotide 10730, nucleotide 10754, nucleotide 10763, nucleotide 10766, nucleotide 10942, nucleotide 11034, nucleotide 11078, nucleotide 11126, nucleotide 11156, nucleotide 11159, nucleotide 11160, nucleotide 11292, nucleotide 11340, nucleotide 11344, nucleotide 11383, nucleotide 11395, nucleotide 11789, nucleotide 11864, nucleotide 11865, nucleotide 11939, nucleotide 12092, nucleotide 12116, nucleotide 12130, nucleotide 12154, nucleotide 12156, nucleotide 12177, nucleotide 12195, nucleotide 1164, nucleotide 1163, nucleotide 1162, nucleotide 1161, nucleotide 1160, nucleotide 1165, nucleotide 1166, nucleotide 1167, nucleotide 1168.
3. The RNA inhibitor according to any one of embodiments 1-2, wherein the antisense strand forms a complementary region with the sequence between nucleotides 1160 and 1188, between nucleotides 6235 and 6465, or between nucleotides 6329 and 6455 of the mRNA encoding IGF-1R (SEQ ID NO: 828), counting from the 5' end.
4. The RNA inhibitor according to any one of embodiments 1-3, wherein the RNA inhibitor is ribonucleic acid; further, the RNA inhibitor is single-stranded ribonucleic acid or double-stranded ribonucleic acid.
5. The RNA inhibitor according to any one of embodiments 1-4, wherein the RNA inhibitor is an antisense oligonucleotide (ASO), shRNA, miRNA, or siRNA.
6. The RNA inhibitor according to any one of embodiments 1-5, comprising a sense strand capable of forming a complementary duplex with the antisense strand, wherein the antisense strand comprises a sequence forming a duplex complementary region with at least 15 consecutive nucleotides in the sense strand sequence, the duplex complementary region having 0, 1, 2, 3, 4, or 5 mismatches; preferably, the duplex complementary region is 15-30 nucleotide pairs in length, more preferably 17-23 nucleotide pairs.
7. The RNA inhibitor according to embodiment 6, wherein the sense strand and the antisense strand reside on two different nucleic acid strands; preferably, the RNA inhibitor is siRNA or shRNA; more preferably, the RNA inhibitor is siRNA.
8. The RNA inhibitor according to any one of embodiments 6-7, wherein the sense strand and the antisense strand reside on the same nucleic acid strand; preferably, the RNA inhibitor is shRNA.
9. The RNA inhibitor according to any one of embodiments 6-8, wherein the total length of the sense strand is 15-50 nucleotides; preferably, the total length of the sense strand is 16-30 nucleotides; more preferably, the total length of the sense strand is 17, 18, 19, 20, or 21 nucleotides.
10. The RNA inhibitor according to any one of embodiments 6-9, wherein the total length of the antisense strand is 19-50 nucleotides; preferably, the total length of the antisense strand is 19-30 nucleotides; more preferably, the total length of the antisense strand is 21, 22, 23, 24, 25, 26, or 27 nucleotides.
11. The RNA inhibitor according to any one of embodiments 6-10, wherein the sense strand and the antisense strand independently optionally comprise a 3' or 5' overhang of 1, 2, or 3 nucleotides.
12. The RNA inhibitor according to embodiment 11, wherein both the sense strand and the antisense strand have a 3' overhang of 1-3 nucleotides, or the sense strand has a 3' or 5' overhang of 1-3 nucleotides, or the antisense strand has a 3' or 5' overhang of 1-3 nucleotides.
13. The RNA inhibitor according to any one of embodiments 1-12, wherein the antisense strand comprises at least 15 consecutive nucleotides from any one of the sequences set forth in SEQ ID NO: 199-400.
14. The RNA inhibitor according to any one of embodiments 6-13, wherein the sense strand comprises at least 15 consecutive nucleotides from any one of the sequences set forth in SEQ ID NO: 1-198.
15. The RNA inhibitor according to any one of embodiments 1-14, comprising a duplex selected from the following: ds-n1, ds-n2, ds-n3, ds-n4, ds-n5, ds-n6, ds-n7, ds-n8, ds-n9, ds-n10, ds-n11, ds-n12, ds-n13, ds-n14, ds-n15, ds-n16, ds-n17, ds-n18, ds-n19, ds-n20, ds-n21, ds-n22, ds-n23, ds-n24, ds-n25, ds-n26, ds-n27, ds-n28, ds-n29, ds-n30, ds-n31, ds-n32, ds-n33, ds-n34, ds-n35, ds-n36, ds-n37, ds-n38, ds-n39, ds-n40, ds-n41, ds-n42, ds-n43, ds-n44, ds-n45, ds-n46, ds-n47, ds-n48, ds-n49, ds-n50, ds-n51, ds-n52, ds-n53, ds-n54, ds-n55, ds-n56, ds-n57, ds-n58, ds-n59, ds-n60, ds-n61, ds-n62, ds-n63, ds-n64, ds-n65, ds-n66, ds-n67, ds-n68, ds-n69, ds-n70, ds-n71, ds-n72, ds-n73, ds-n74, ds-n75, ds-n76, ds-n77, ds-n78, ds-n79, ds-n80, ds-n81, ds-n82, ds-n83, ds-n84, ds-n85, ds-n86, ds-n87, ds-n88, ds-n89, ds-n90, ds-n91, ds-n92, ds-n93, ds-n94, ds-n95, ds-n96, ds-n97, ds-n98, ds-n99, ds-n100, ds-n101, ds-n102, ds-n103, ds-n104, ds-n105, ds-n106, ds-n107, ds-n108, ds-n109, ds-n110, ds-n111, ds-n112, ds-n113, ds-n114, ds-n115, ds-n116, ds-n117, ds-n118, ds-n119, ds-n120, ds-n121, ds-n122, ds-n123, ds-n124, ds-n125, ds-n126, ds-n127, ds-n128, ds-n129, ds-n130, ds-n131, ds-n132, ds-n133, ds-n134, ds-n135, ds-n136, ds-n137, ds-n138, ds-n139, ds-n140, ds-n141, ds-n142, ds-n143, ds-n144, ds-n145, ds-n146, ds-n147, ds-n148, ds-n149, ds-n150, ds-n151, ds-n152, ds-n153, ds-n154, ds-n155, ds-n156, ds-n157, ds-n158, ds-n159, ds-n160, ds-n161, ds-n162, ds-n163, ds-n164, ds-n165, ds-n166, ds-n167, ds-n168, ds-n169, ds-n170, ds-n171, ds-n172, ds-n173, ds-n174, ds-n175, ds-n176, ds-n177, ds-n178, ds-n179, ds-n180, ds-n181, ds-n182, ds-n183, ds-n184, ds-n185, ds-n186, ds-n187, ds-n188, ds-n189, ds-n190, ds-n191, ds-n192, ds-n193, ds-n194, ds-n195, ds-n196, ds-n197, ds-n198, ds-n201, ds-n202, ds-n203, ds-n204.
16. The RNA inhibitor according to embodiment 15, comprising a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188, ds-n190, ds-n191, ds-n192, ds-n193, ds-n194, ds-n195, ds-n196, ds-n197, ds-n198.
17. The RNA inhibitor according to embodiment 15, comprising a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188.
18. The RNA inhibitor according to embodiment 16, comprising a duplex selected from the following: ds-n23, ds-n58, ds-n67, ds-n86, ds-n89, ds-n98, ds-n99, ds-n103, ds-n190, ds-n198, ds-n113.
19. The RNA inhibitor according to any one of embodiments 1-18, wherein the antisense strand further comprises a region B1 at its 3' terminus, and the sense strand further comprises a region A1 at its 5' terminus, wherein region A1 is 0-6 nucleotides and region B1 is 0-6 nucleotides.
20. The RNA inhibitor according to embodiment 19, wherein region B1 is 0, 1, 2, 3, 4, 5, or 6 nucleotides; preferably, region B1 is 0 or 2 nucleotides.
21. The RNA inhibitor according to any one of embodiments 16-20, wherein region A1 is 0, 1, 2, 3, 4, 5, or 6 nucleotides; preferably, region A1 is 0 or 2 nucleotides.
22. The RNA inhibitor according to embodiment 21, wherein the antisense strand further comprises a region X2 at its 5' terminus, and the sense strand or sense nucleic acid further comprises a region X1 at its 3' terminus, wherein regions X1 and X2 are complementary.
23. The RNA inhibitor according to embodiment 22, wherein X1 is A, U, modified A, or modified U, and X2 is U, A, modified U, or modified A.
24. The RNA inhibitor according to any one of embodiments 19-23, wherein the antisense strand, proceeding from the 3' to 5' direction at its 5' terminus, sequentially comprises X2, Y, Z, and N, wherein X2 and Y are independently A, U, modified A, or modified U, Z is G or modified G, and N comprises at least one nucleotide.
25. The RNA inhibitor according to any one of embodiments 19-24, wherein X2, Y, and Z, proceeding from the 3' to 5' direction, are AAG, AUG, UUG, or UAG, or partially or fully modified versions thereof.
26. The RNA inhibitor according to any one of embodiments 21-25, wherein X2, Y, and Z, proceeding from the 3' to 5' direction, are AAG, AUG, UUG, or UAG, or partially or fully modified versions thereof.
27. The RNA inhibitor according to any one of embodiments 21-26, wherein N is C or modified C.
28. The RNA inhibitor according to any one of embodiments 21-27, wherein X2, Y, Z, and N, proceeding from the 3' to 5' direction, are AAGC and UAGC, or partially or fully modified versions thereof.
29. The RNA inhibitor according to any one of embodiments 16-28, wherein the antisense strand comprises at least 15 consecutive nucleotides from any one of the sequences set forth in SEQ ID NO: 401-413.
30. The RNA inhibitor according to any one of embodiments 1-29, selected from ds-n190-1, ds-n191-1, ds-n192-1, ds-n193-1, ds-n194-1, ds-n195-1, ds-n196-1, ds-n197-1, ds-n198-1, ds-n201-1, ds-n202-1, ds-n203-1, ds-n204-1.
31. The RNA inhibitor according to any one of embodiments 1-30, wherein at least one nucleotide in the RNA inhibitor is a modified nucleotide.
32. The RNA inhibitor according to any one of embodiments 1-31, wherein at least 70%, 80%, 90%, or 95% of the nucleotides in the RNA inhibitor are modified nucleotides; preferably, all nucleotides are modified nucleotides.
33. The RNA inhibitor according to any one of embodiments 31-32, wherein the modification comprises one or more of the following: 2'-OMe (2'-O-methyl) modification, 2'-F (2'-deoxy-2'-fluoro) modification, 2'-O-MOE (2'-O-methoxyethyl) modification, 2'-deoxy (2'-d) modification, 5'-morpholino (5'-Mo) modification, unlocked nucleic acid (UNA) modification, glycol nucleic acid (GNA) modification, locked nucleic acid (LNA) modification, tricyclo-DNA (tcDNA) modification, (S)-constrained ethyl bicyclic nucleic acid ((S)-cEt-BNA) modification, phosphorothioate (PS) modification, dithiophosphate (PS2) modification, methylphosphonate (MP) modification, methoxypropyl methylphosphonate (MOP) modification, peptide nucleic acid (PNA) modification, 5'-(E)-vinyl phosphate (VP) modification (VP), N6-methyladenosine (m6A) modification, 5-methylcytidine (m5C) modification, 3-methyluridine (m3U) modification, 5-methyluridine (m5U) modification, pseudouridine modification, 2-thiouridine (s2U) modification, propynyluridine (5-pU) modification, linkage of an inverted abasic nucleotide (invAB) at the 5' or 3' end of a nucleotide, replacement of a nucleotide with an inverted abasic nucleotide (invAb) modification, replacement of a nucleotide with 2,4-difluorotolyl ribonucleotide (rF) modification, or replacement of a nucleotide with (S)-glycerol nucleic acid modification; preferably, the modifications are 2'-OMe modification, 2'-F modification, 2'-deoxy modification, VP modification, 5'-MP modification, PS modification, PS2 modification, MP modification, MOP modification, M06 modification, invAb modification, or invAB modification.
34. The RNA inhibitor according to any one of embodiments 31-33, wherein the 3' or 5' end of the antisense strand comprises a phosphate or phosphate mimic, or the 3' or 5' end of the sense strand comprises a phosphate or phosphate mimic.
35. The RNA inhibitor according to embodiment 34, wherein the phosphate mimic includes 5'-(E)-vinylphosphonate, 5'-methylphosphonate, (S)-5'-C-methyl analog, and 5'-thiophosphate (5'-PS).
36. The RNA inhibitor according to any one of embodiments 31-35, wherein the 5' end of the antisense strand comprises an (M06) modification: 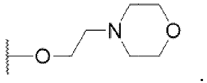
37. The RNA inhibitor according to any one of embodiments 31-36, wherein the 3' or 5' end of the antisense strand comprises an invAB modification, or the 3' or 5' end of the sense strand comprises an invAB modification.
38. The RNA inhibitor according to any one of embodiments 31-37, wherein the inverted abasic nucleotide or M06 is linked via a phosphorothioate to the 3' or 5' end of the antisense strand or to the 3' or 5' end of the sense strand.
39. The RNA inhibitor according to any one of embodiments 31-38, having the following combination of modifications:
   a) the sense strand comprises a first sequence of 16-21 nt, wherein the first sequence includes the following modifications: counting from the 5' end, nucleotides at positions 9, 10, and 11 bear 2'-F modifications;
   b) the antisense strand comprises a second sequence of 16-21 nt, wherein the second sequence includes the following modifications: counting from the 5' end, a phosphorothioate linkage exists between nucleotides 1 and 2, between nucleotides 2 and 3, and between nucleotides 3 and 4; additionally, counting from the 3' end, a phosphorothioate linkage exists between nucleotides 1 and 2; and counting from the 5' end, nucleotides at positions 2, 3, 4, 12, 14, and 16 bear 2'-F modifications.
40. The RNA inhibitor according to embodiment 39, wherein the length of the first sequence is preferably 16 nt, 17 nt, 18 nt, 19 nt, 20 nt, or 21 nt, more preferably 21 nt.
41. The RNA inhibitor according to embodiment 39, wherein the length of the second sequence is preferably 16 nt, 17 nt, 18 nt, 19 nt, 20 nt, or 21 nt, more preferably 21 nt.
42. The RNA inhibitor according to any one of embodiments 39-41, having the following combination of modifications:
   a) the sense strand is 21 nt in length and includes the following modifications: counting from the 5' end, nucleotides at positions 9, 10, and 11 bear 2'-F modifications;
   b) the antisense strand is 21 nt in length and includes the following modifications: counting from the 5' end, a phosphorothioate linkage exists between nucleotides 1 and 2, between nucleotides 2 and 3, and between nucleotides 3 and 4; additionally, counting from the 3' end, a phosphorothioate linkage exists between nucleotides 1 and 2; and counting from the 5' end, nucleotides at positions 2, 3, 4, 12, 14, and 16 bear 2'-F modifications.
43. The RNA inhibitor according to any one of embodiments 28-42, wherein the antisense strand comprises at least 15 consecutive nucleotides from any one of the sequences set forth in SEQ ID NO: 624-812.
44. The RNA inhibitor according to any one of embodiments 28-42, wherein the sense strand comprises at least 15 consecutive nucleotides from any one of the sequences set forth in SEQ ID NO: 414-602.
45. The RNA inhibitor according to any one of embodiments 28-44, selected from:
   ds-m1, ds-m2, ds-m3, ds-m4, ds-m5, ds-m6, ds-m7, ds-m8, ds-m9, ds-m10, ds-m11, ds-m12, ds-m13, ds-m14, ds-m15, ds-m16, ds-m17, ds-m18, ds-m19, ds-m20, ds-m21, ds-m22, ds-m23, ds-m24, ds-m25, ds-m26, ds-m27, ds-m28, ds-m29, ds-m30, ds-m31, ds-m32, ds-m33, ds-m34, ds-m35, ds-m36, ds-m37, ds-m38, ds-m39, ds-m40, ds-m41, ds-m42, ds-m43, ds-m44, ds-m45, ds-m46, ds-m47, ds-m48, ds-m49, ds-m50, ds-m51, ds-m52, ds-m53, ds-m54, ds-m55, ds-m56, ds-m57, ds-m58, ds-m59, ds-m60, ds-m61, ds-m62, ds-m63, ds-m64, ds-m65, ds-m66, ds-m67, ds-m68, ds-m69, ds-m70, ds-m71, ds-m72, ds-m73, ds-m74, ds-m75, ds-m76, ds-m77, ds-m78, ds-m79, ds-m80, ds-m81, ds-m82, ds-m83, ds-m84, ds-m85, ds-m86, ds-m87, ds-m88, ds-m89, ds-m90, ds-m91, ds-m92, ds-m93, ds-m94, ds-m95, ds-m96, ds-m97, ds-m98, ds-m99, ds-m100, ds-m101, ds-m102, ds-m103, ds-m104, ds-m105, ds-m106, ds-m107, ds-m108, ds-m109, ds-m110, ds-m111, ds-m112, ds-m113, ds-m114, ds-m115, ds-m116, ds-m117, ds-m118, ds-m119, ds-m120, ds-m121, ds-m122, ds-m123, ds-m124, ds-m125, ds-m126, ds-m127, ds-m128, ds-m129, ds-m130, ds-m131, ds-m132, ds-m133, ds-m134, ds-m135, ds-m136, ds-m137, ds-m138, ds-m139, ds-m140, ds-m141, ds-m142, ds-m143, ds-m144, ds-m145, ds-m146, ds-m147, ds-m148, ds-m149, ds-m150, ds-m151, ds-m152, ds-m153, ds-m154, ds-m155, ds-m156, ds-m157, ds-m158, ds-m159, ds-m160, ds-m161, ds-m162, ds-m163, ds-m164, ds-m165, ds-m166, ds-m167, ds-m168, ds-m169, ds-m170, ds-m171, ds-m172, ds-m173, ds-m174, ds-m175, ds-m176, ds-m177, ds-m178, ds-m179, ds-m180, ds-m181, ds-m182, ds-m183, ds-m184, ds-m185, ds-m186, ds-m187, ds-m188, ds-m189.
46. The RNA inhibitor according to any one of embodiments 28-39, wherein:
   a) the sense strand comprises a third sequence of 18-21 nt, wherein the third sequence includes the following modifications: counting from the 5' end, a phosphorothioate linkage exists between nucleotides 1 and 2 and between nucleotides 2 and 3 of the sense strand; nucleotides at positions 9, 10, 11, and 18 of the sense strand bear 2'-F modifications, and the remainder bear 2'-OMe modifications; counting from the 3' end, a phosphorothioate linkage exists between nucleotides 1 and 2 and between nucleotides 2 and 3;
   b) the antisense strand comprises a fourth sequence of 19-26 nt, wherein the fourth sequence includes the following modifications: counting from the 5' end, nucleotides at positions 1, 2, 5, 9, 17, and 19 of the antisense strand bear 2'-F modifications, and the remainder bear 2'-OMe modifications; a phosphorothioate linkage exists between nucleotides 4 and 5; counting from the 3' end, a phosphorothioate linkage exists between nucleotides 1 and 2 and between nucleotides 2 and 3.
47. The RNA inhibitor according to embodiment 46, wherein the length of the third sequence is preferably 18 nt, 19 nt, 20 nt, 21 nt, or 22 nt, more preferably 21 nt.
48. The RNA inhibitor according to embodiment 47, wherein the length of the fourth sequence is preferably 19 nt, 20 nt, 21 nt, 22 nt, 23 nt, 24 nt, 25 nt, or 26 nt, more preferably 26 nt.
49. The RNA inhibitor according to any one of embodiments 46-48, wherein:
   a) the sense strand is 21 nt in length; counting from the 5' end, a phosphorothioate linkage exists between nucleotides 1 and 2 and between nucleotides 2 and 3 of the sense strand; nucleotides at positions 9, 10, 11, and 18 of the sense strand bear 2'-F modifications, and the remainder bear 2'-OMe modifications; counting from the 3' end, a phosphorothioate linkage exists between nucleotides 1 and 2 and between nucleotides 2 and 3;
   b) the antisense strand is 26 nt in length; counting from the 5' end, nucleotides at positions 1, 2, 5, 9, 17, and 19 of the antisense strand bear 2'-F modifications, and the remainder bear 2'-OMe modifications; a phosphorothioate linkage exists between nucleotides 4 and 5; counting from the 3' end, a phosphorothioate linkage exists between nucleotides 1 and 2 and between nucleotides 2 and 3.
50. The RNA inhibitor according to any one of embodiments 31-49, wherein the antisense strand comprises at least 15 consecutive nucleotides from any one of the sequences set forth in SEQ ID NO: 813-827.
51. The RNA inhibitor according to any one of embodiments 31-50, wherein the sense strand comprises at least 15 consecutive nucleotides from any one of the sequences set forth in SEQ ID NO: 603-617.
52. The RNA inhibitor according to any one of embodiments 31-51, comprising a duplex selected from the following:
   ds-m190, ds-m191, ds-m192, ds-m193, ds-m194, ds-m195, ds-m196, ds-m197, ds-m198, ds-m199, ds-m200, ds-m201, ds-m202, ds-m203, and ds-m204.
53. The RNA inhibitor according to any one of embodiments 1-52, further comprising a delivery system, wherein the delivery system is conjugated to the sense strand and/or the antisense strand, and the delivery system enables the RNA inhibitor to reach the target tissue to achieve gene silencing of the target RNA.
54. The RNA inhibitor according to embodiment 53, wherein the target tissue is ocular tissue, joint tissue, central nervous tissue, peripheral nervous tissue, tumor, liver tissue, kidney tissue, muscle tissue, or adipose tissue.
55. The RNA inhibitor according to embodiment 54, wherein the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.
56. The RNA inhibitor according to embodiment 55, wherein the ocular tissue is retinal ganglion, endothelial cell, extraocular muscle, or periorbital fat.
57. The RNA inhibitor according to any one of embodiments 53-56, wherein the delivery system is independently conjugated to one or more internal positions of the double-stranded ribonucleic acid.
58. The RNA inhibitor according to embodiment 57, wherein the internal position is on the nucleobase, sugar ring, methylphosphonate bond, phosphorothioate diester bond, or phosphodiester bond.
59. The RNA inhibitor according to any one of embodiments 53-58, wherein the delivery system is a lipophilic moiety, the lipophilic moiety comprising a lipophilic group and a linker, wherein the lipophilic group is linked to the double-stranded ribonucleic acid via the linker.
60. The RNA inhibitor according to embodiment 59, wherein the lipophilic moiety is selected from aliphatic, alicyclic, and polycyclic aliphatic compounds.
61. The RNA inhibitor according to embodiment 60, wherein the lipophilic moiety contains saturated or unsaturated C16 or C22.
62. The RNA inhibitor according to any one of embodiments 59-61, wherein the linker is selected from a single bond, ether, thioether, urea, carbonate, amine, amide, maleimide-thioether, disulfide, phosphodiester, sulfonamide bond, product of a click reaction, and carbamate.
63. The RNA inhibitor according to embodiments 53-62, wherein the delivery system is 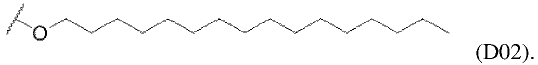
64. The RNA inhibitor according to any one of embodiments 53-63, wherein the delivery system is conjugated to the 5' end and/or 3' end of the antisense strand or antisense nucleic acid fragment.
65. The RNA inhibitor according to any one of embodiments 53-64, wherein the delivery system is conjugated to the 5' end and/or 3' end of the sense strand or sense nucleic acid fragment.
66. The RNA inhibitor according to any one of embodiments 53-65, wherein the delivery system is conjugated to the 5' end of the antisense strand or antisense nucleic acid fragment, and the ligand is conjugated to the 3' end of the sense strand or sense nucleic acid fragment, wherein the two ligands are the same or different.
67. The RNA inhibitor according to any one of embodiments 53-66, wherein the delivery system is conjugated to the 3' end of the antisense strand or antisense nucleic acid fragment, and the ligand is conjugated to the 5' end of the sense strand or sense nucleic acid fragment, wherein the two ligands are the same or different.
68. The RNA inhibitor according to any one of embodiments 53-67, wherein the delivery system is conjugated to the 5' end of the antisense strand or antisense nucleic acid fragment, and the ligand is conjugated to the 5' end of the sense strand or sense nucleic acid fragment, wherein the two ligands are the same or different.
69. The RNA inhibitor according to any one of embodiments 53-68, wherein the delivery system is conjugated to the 3' end of the antisense strand or antisense nucleic acid fragment, and the ligand is conjugated to the 3' end of the sense strand or sense nucleic acid fragment, wherein the two ligands are the same or different.
70. The RNA inhibitor according to any one of embodiments 53-69, wherein the number of delivery systems is 1, 2, 3, 4, 5, or 6.
71. The RNA inhibitor according to any one of embodiments 53-70, wherein the antisense strand comprises at least 15 consecutive nucleotides of the sequence as set forth in any one of SEQ ID NO: 822-827.
72. The RNA inhibitor according to any one of embodiments 53-71, wherein the sense strand comprises at least 15 consecutive nucleotides of the sequence as set forth in any one of SEQ ID NO: 618-623.
73. The RNA inhibitor according to any one of embodiments 53-72, selected from: Z1, Z2, Z3, Z4, Z5, Z6.
74. A pharmaceutical composition comprising the RNA inhibitor according to any one of embodiments 1-73, and/or a physiologically acceptable excipient, and/or carrier, and/or diluent.
75. The composition according to embodiment 74, wherein the pharmaceutically acceptable carrier comprises or is selected from an aqueous carrier, liposome, polymer, or polypeptide.
76. Use of an IGF-1R-targeting RNA inhibitor and its pharmaceutical composition in the manufacture of a medicament for treating an IGF-1R-related disease or pathology; preferably, the RNA inhibitor is siRNA; more preferably, the siRNA is administered locally or to the lesion site tissue of a subject in need thereof.
77. The use according to embodiment 76, wherein the IGF-1R-related disease or pathology includes diseases or symptoms associated with elevated IGF-1R levels.
78. The use according to any one of embodiments 76-77, wherein the IGF-1R-related disease or pathology includes thyroid eye disease, osteoarthritis, and neuropathic pain.
79. The use according to any one of embodiments 76-78, wherein the RNA inhibitor and its pharmaceutical composition are the RNA inhibitor according to any one of embodiments 1-73 or the pharmaceutical composition according to any one of embodiments 74-75.
80. Use of the RNA inhibitor according to any one of embodiments 1-73 or the pharmaceutical composition according to any one of embodiments 74-75 in the manufacture of a medicament for preventing or treating a disease or pathology or reducing the risk of a disease or symptom.
81. The use according to embodiment 80, wherein the disease or pathology includes diseases or symptoms associated with elevated IGF-1R levels.
82. The use according to any one of embodiments 80-81, wherein the disease or pathology includes thyroid eye disease, osteoarthritis, and neuropathic pain.
83. A method for preventing or treating an IGF-1R-related disease or symptom, comprising administering an effective amount of an RNA inhibitor and its pharmaceutical composition to a subject in need thereof.
84. The method according to embodiment 83, wherein the RNA inhibitor and its pharmaceutical composition are the RNA inhibitor according to any one of embodiments 1-73 or the pharmaceutical composition according to any one of embodiments 74-75.
85. The method according to embodiments 83-84, wherein the RNA inhibitor, its pharmaceutically acceptable salt, or the pharmaceutical composition is administered to the subject via intraorbital injection, intra-articular injection, intrathecal injection, subcutaneous administration, intravenous administration, oral administration, rectal administration, or intraperitoneal administration.
86. The method according to any one of embodiments 83-85, comprising administering to the local or lesion site tissue of a subject in need thereof.
87. The method according to embodiment 86, wherein the local or lesion site tissue of the subject in need thereof includes ocular tissue, joint tissue, central nervous tissue, peripheral nervous tissue, tumor, liver tissue, kidney tissue, muscle tissue, or adipose tissue.
88. The method according to embodiment 87, wherein the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.
89. The method according to embodiment 88, wherein the ocular tissue is retinal ganglion, endothelial cell, extraocular muscle, or periorbital fat.
90. The method according to any one of embodiments 83-89, wherein the IGF-1R-related disease or pathology includes diseases or symptoms associated with elevated IGF-1R levels.
91. The method according to any one of embodiments 83-90, wherein the IGF-1R-related disease or pathology includes thyroid eye disease, osteoarthritis, and neuropathic pain.
92. A method for inhibiting IGF-1R gene expression in a cell, tissue, or subject, comprising administering an effective amount of the RNA inhibitor according to any one of embodiments 1-73 or the pharmaceutical composition according to any one of embodiments 74-75 to the cell, tissue, or subject.
93. The method according to embodiment 92, wherein the disease or pathology includes diseases or symptoms associated with elevated IGF-1R levels.
94. The method according to embodiment 93, wherein the disease or pathology includes thyroid eye disease, osteoarthritis, and neuropathic pain.
95. A method for preventing or treating a disease or symptom, comprising administering an effective amount of the RNA inhibitor according to any one of embodiments 1-73 or the pharmaceutical composition according to any one of embodiments 74-75 to a subject in need thereof.
96. The method according to embodiment 95, wherein the RNA inhibitor, its pharmaceutically acceptable salt, or the pharmaceutical composition is administered to the subject via intraorbital injection, intra-articular injection, intrathecal injection, subcutaneous administration, intravenous administration, oral administration, rectal administration, or intraperitoneal administration.
97. The method according to any one of embodiments 95-96, comprising administering to the local or lesion site tissue of a subject in need thereof.
98. The method according to embodiment 97, wherein the local or lesion site tissue of the subject in need thereof includes ocular tissue, joint tissue, central nervous tissue, peripheral nervous tissue, tumor, liver tissue, kidney tissue, muscle tissue, or adipose tissue.
99. The method according to embodiment 98, wherein the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.
100. The method according to embodiment 99, wherein the ocular tissue is retinal ganglion, endothelial cell, extraocular muscle, or periorbital fat.

### Examples

### Example 1 Design and Synthesis of siRNA Molecules

The human IGF-1R gene transcript (source: NCBI website, transcript accession number: NM_000875.5) was obtained, and siRNA primary sequences targeting the IGF-1R gene were designed based on the full-length region of human IGF-1R mRNA (including 5'-UTR, CDS, and 3'-UTR).

Oligoribonucleotides were synthesized using phosphoramidite solid-phase synthesis technology. Synthesis was performed on universal controlled-pore glass (CPG). All 2'-modified RNA phosphoramidites and auxiliary reagents were commercially available. All phosphoramidites were dissolved in anhydrous acetonitrile with molecular sieves added, and coupling was performed for 1.0 minute using 5-ethylthio-1H-tetrazole as the activator. A phosphorothioate linkage was introduced using a solution of 50 mM 3-((dimethylamino-methylene)amino)-3H-1,2,4-dithiazole-3-thione in anhydrous acetonitrile/pyridine (v/v = 1/1), with a reaction time of 1.8 minutes. All sequences were synthesized with final removal of the DMT group.

Cleavage and deprotection of oligomers bound to CPG: After completion of solid-phase synthesis, the protecting groups were removed by treatment with 20% diethylamine in acetonitrile for 30 minutes without cleaving the oligonucleotide from the CPG. Subsequently, the dried CPG was treated with concentrated ammonium hydroxide at 40°C for 18 hours. After centrifugation, the supernatant was transferred to a new tube and the CPG was washed with ammonium hydroxide. The combined solutions were concentrated to yield a solid mixture.

Purification of single-stranded oligoribonucleotides: Oligomers were purified by HPLC using NanoQ anion-exchange chromatography. Buffer A was 10 mM sodium perchlorate, 20 mM Tris, 1 mM EDTA, pH 7.4, containing 20% acetonitrile, and buffer B was 500 mM sodium perchlorate, 20 mM Tris, 1 mM EDTA, pH 7.4, containing 20% acetonitrile. The target product was isolated and desalted using a reverse-phase C18 column.

Annealing of single-stranded oligoribonucleotides to generate siRNA: Single-stranded oligoribonucleotides to be annealed were prepared as 200 µM solutions in sterile, RNase-free water. The annealing reaction was set up as follows: a total volume of 100 µL containing 10 nmol of oligonucleotides was heated at 95°C for 10 minutes (for amounts ≥100 nmol, heating for 20 minutes is required) → rapidly transferred to a 60°C water bath and allowed to cool naturally → the annealed solution should not be stored at elevated temperatures. Complementary strands were formed by combining equimolar solutions of single-stranded oligoribonucleotides. Unmodified siRNA sequences are shown in Table 1.

The siRNA duplexes were further optimized by sequence modification, wherein lowercase letters "g", "c", "a", and "u" denote 2'-methoxy modified nucleotides; uppercase letters "Gf", "Cf", "Af", and "Uf" denote 2'-fluoro modified nucleotides; and * indicates a phosphorothioate linkage between the two adjacent monomers (e.g., nucleotides) flanking the *.

invAB refers to an inverted abasic nucleotide linked to the 5' or 3' end of a nucleotide.

In the present invention, (M06) 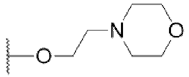 is the residue of M06 monomer

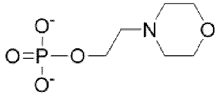

. In certain embodiments, (M06) is linked 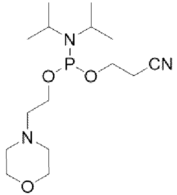 to the nucleotide via a bond.

In certain embodiments, [D02] 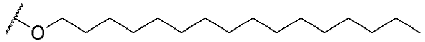 is linked 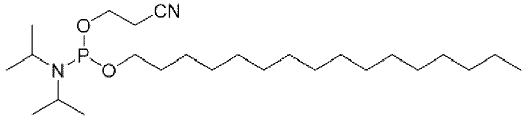 to the nucleotide via a bond.

The modified siRNA sequences are shown in Table 2.

### Example 2 In Vitro Efficacy Testing of Chemically Modified Duplex siRNA in SY5Y Cells

Duplex siRNAs selected according to Example 1 were further validated for their in vitro inhibitory efficacy in SY5Y cells.

### 1) SY5Y Cell Transfection

SY5Y cells were cultured in DMEM medium supplemented with 10% fetal bovine serum, 1% glutamine, 1% NEAA, and 1% penicillin-streptomycin, maintained in a humidified incubator at 37°C with 5% CO2, and transfected when the cells were in logarithmic growth phase and in good condition. SY5Y cells were seeded into 96-well plates and transfected with siRNA using RNAiMAX according to the manufacturer's instructions. The final siRNA concentrations tested were 30 nM and 0.1 nM. A control group containing RNAiMAX without compound was also included.

### 2) RNA Extraction and Reverse Transcription

Twenty-four hours post-transfection, the culture medium was removed and cells were collected for RNA extraction. Total RNA was extracted using the RNeasy^{®} 96 Kit (QIAGEN-74182) according to the kit instructions. cDNA was synthesized using the FastKing RT Kit (With gDNase) (Tiangen-KR116-02) according to the manufacturer's instructions.

### 3) qPCR Detection of Target Gene mRNA Expression Levels

Target cDNA was detected by SYBR Green qPCR, with GAPDH cDNA simultaneously detected in parallel as an internal control. 8 µL of prepared PCR reaction mix and 2 µL of sample cDNA were added to each well of a 384-well plate. The qPCR program was as follows: 2 min at 50°C, 10 min at 95°C, followed by 40 cycles of 15 sec at 95°C and 1 min at 60°C.

### 4) Data Analysis

The mRNA expression level of the target gene in each sample was calculated using the ΔΔCt relative quantification method. The relative amount of the target gene is expressed as 2^(-ΔΔCT).

The calculation formulas are as follows: $ΔCT = average Ct value of target gene - average Ct value of reference gene$ $ΔΔCT = ΔCT \left(treated group\right) - ΔCT \left(RNAiMAX control group\right)$ $Relative expression level of target gene IGF - 1 R = {2}^{∧} \left(-ΔΔCT\right)$ $IGF - 1 R inhibition rate \left(%\right) = \left(1-value of sample/Ave. value of RNAiMAX Control\right) \times 100$

Results are shown in Table 3.

**Table 3 Inhibition Results of Duplex siRNA in SY5Y Cells (Percent Inhibition)**

| **Double-Stranded Ribonucleic Acid Sequence Number** | **SY5Y (30 nM, %)** | **SY5Y (0.1 nM, %)** |
|---|---|---|
| ds-m1 | 30.34 | -8.35 |
| ds-m2 | 15.99 | -7.23 |
| ds-m3 | 7.18 | -29.91 |
| ds-m4 | 29.84 | -18.55 |
| ds-m5 | 6.80 | -14.90 |
| ds-m6 | 38.89 | -24.42 |
| ds-m7 | 6.64 | -14.08 |
| ds-m8 | 41.21 | -5.61 |
| ds-m9 | 10.67 | -21.50 |
| ds-m10 | 46.79 | -1.35 |
| ds-m11 | 23.35 | -22.23 |
| ds-m12 | 31.84 | -28.47 |
| ds-m13 | 37.81 | -25.87 |
| ds-m14 | 30.45 | -23.51 |
| ds-m15 | 28.71 | -12.47 |
| ds-m16 | 48.25 | -10.17 |
| ds-m17 | 49.97 | 12.45 |
| ds-m18 | 10.67 | -11.13 |
| ds-m19 | 15.43 | -18.49 |
| ds-m20 | 22.49 | -19.55 |
| ds-m21 | 30.54 | 11.66 |
| ds-m22 | 42.56 | 9.61 |
| ds-m23 | 48.37 | 28.09 |
| ds-m24 | 32.63 | 21.75 |
| ds-m25 | 33.65 | 27.46 |
| ds-m26 | 44.24 | 3.60 |
| ds-m27 | 43.26 | -16.29 |
| ds-m28 | 38.95 | -4.25 |
| ds-m29 | 49.91 | -4.87 |
| ds-m30 | 39.28 | 24.91 |
| ds-m31 | 50.04 | 31.56 |
| ds-m32 | 38.96 | 12.56 |
| ds-m33 | 41.56 | 11.21 |
| ds-m34 | 28.20 | -1.47 |
| ds-m35 | 24.78 | -8.39 |
| ds-m36 | 10.07 | -11.70 |
| ds-m37 | 4.41 | -30.73 |
| ds-m38 | 39.16 | -0.57 |
| ds-m39 | 51.73 | 15.02 |
| ds-m40 | 46.16 | 6.03 |
| ds-m41 | 38.32 | 6.95 |
| ds-m42 | 36.96 | 2.27 |
| ds-m43 | 15.98 | -9.45 |
| ds-m44 | 20.16 | -5.17 |
| ds-m45 | 45.20 | 5.28 |
| ds-m46 | 44.03 | -10.29 |
| ds-m47 | 12.92 | -38.48 |
| ds-m48 | -18.49 | -44.13 |
| ds-m49 | 23.37 | -14.79 |
| ds-m50 | 28.26 | -10.00 |
| ds-m51 | 35.18 | -3.26 |
| ds-m52 | 41.70 | 2.44 |
| ds-m53 | 31.44 | -11.11 |
| ds-m54 | -5.68 | -12.91 |
| ds-m55 | 40.16 | -6.99 |
| ds-m56 | 21.90 | -7.12 |
| ds-m57 | 2.14 | 0.66 |
| ds-m58 | 46.24 | 19.24 |
| ds-m59 | 2.87 | -20.14 |
| ds-m60 | 45.39 | 12.01 |
| ds-m61 | 26.71 | -7.95 |
| ds-m62 | 20.44 | -8.41 |
| ds-m63 | 32.78 | -5.93 |
| ds-m64 | 46.24 | 5.34 |
| ds-m65 | 28.78 | 1.09 |
| ds-m66 | 24.62 | -1.34 |
| ds-m67 | 51.52 | 16.89 |
| ds-m68 | 50.65 | 20.24 |
| ds-m69 | 40.61 | 13.55 |
| ds-m70 | 46.77 | 14.64 |
| ds-m71 | 32.95 | 2.86 |
| ds-m72 | 37.13 | 7.73 |
| ds-m73 | 46.75 | 30.11 |
| ds-m74 | 46.58 | 29.81 |
| ds-m75 | 47.29 | 22.90 |
| ds-m76 | 49.81 | 17.20 |
| ds-m77 | 49.24 | 16.37 |
| ds-m78 | 47.66 | 27.33 |
| ds-m79 | 34.14 | 24.86 |
| ds-m80 | 46.23 | 20.13 |
| ds-m81 | 50.78 | 23.29 |
| ds-m82 | 34.30 | 20.26 |
| ds-m83 | 53.88 | 34.16 |
| ds-m84 | 50.79 | 38.22 |
| ds-m85 | 38.60 | 17.79 |
| ds-m86 | 48.65 | 46.35 |
| ds-m87 | 31.51 | 37.91 |
| ds-m88 | 50.59 | 24.21 |
| ds-m89 | 63.51 | 49.92 |
| ds-m90 | 23.24 | -18.42 |
| ds-m91 | 47.24 | -41.22 |
| ds-m92 | 4.74 | -50.54 |
| ds-m93 | 49.09 | -13.25 |
| ds-m94 | 59.44 | -11.76 |
| ds-m95 | 55.31 | 36.33 |
| ds-m96 | 52.07 | 51.11 |
| ds-m97 | 59.56 | 17.05 |
| ds-m98 | 62.06 | 36.70 |
| ds-m99 | 67.43 | 44.10 |
| ds-m100 | 53.83 | 16.66 |
| ds-m101 | 53.59 | 8.98 |
| ds-m102 | 5.08 | 29.67 |
| ds-m103 | 58.63 | 35.03 |
| ds-m104 | 68.67 | 22.93 |
| ds-m105 | 54.36 | 28.59 |
| ds-m106 | 60.96 | 5.11 |
| ds-m107 | 51.44 | 27.20 |
| ds-m108 | 39.69 | 5.20 |
| ds-m109 | 53.85 | 21.16 |
| ds-m110 | 41.21 | 5.92 |
| ds-m111 | 44.59 | 13.63 |
| ds-m112 | 50.83 | 40.36 |
| ds-m113 | 50.90 | 53.75 |
| ds-m114 | 43.23 | 30.95 |
| ds-m115 | 51.84 | 44.19 |
| ds-m116 | 51.42 | 24.76 |
| ds-m117 | 53.47 | 28.72 |
| ds-m118 | 56.68 | 26.05 |
| ds-m119 | 42.04 | -1.52 |
| ds-m120 | 41.43 | -2.68 |
| ds-m121 | 37.59 | -22.22 |
| ds-m122 | 54.43 | -10.95 |
| ds-m123 | -1.91 | -44.00 |
| ds-m124 | 46.26 | 1.31 |
| ds-m125 | 47.75 | 2.02 |
| ds-m126 | 31.09 | -21.12 |
| ds-m127 | 66.72 | 9.85 |
| ds-m128 | 58.24 | 20.99 |
| ds-m129 | 39.42 | -4.69 |
| ds-m130 | 58.12 | -13.10 |
| ds-m131 | 30.68 | -22.31 |
| ds-m132 | 4.79 | -23.58 |
| ds-m133 | 49.33 | -10.08 |
| ds-m134 | 55.30 | -0.14 |
| ds-m135 | 50.93 | -16.79 |
| ds-m136 | 17.20 | -42.59 |
| ds-m137 | 36.55 | -18.25 |
| ds-m138 | -7.82 | -15.52 |
| ds-m139 | 24.36 | -12.24 |
| ds-m140 | 55.91 | -10.46 |
| ds-m141 | 65.63 | 19.10 |
| ds-m142 | 36.98 | -23.97 |
| ds-m143 | 43.56 | -16.46 |
| ds-m144 | 23.16 | -2.96 |
| ds-m145 | 68.15 | 9.09 |
| ds-m146 | 70.18 | -9.52 |
| ds-m147 | 47.08 | -23.84 |
| ds-m148 | 47.51 | -24.39 |
| ds-m149 | 41.94 | -2.98 |
| ds-m150 | 45.28 | -15.73 |
| ds-m151 | 64.38 | 10.35 |
| ds-m152 | 63.07 | -36.52 |
| ds-m153 | 61.65 | 35.35 |
| ds-m154 | 68.19 | 39.23 |
| ds-m155 | 56.76 | 4.25 |
| ds-m156 | 79.36 | 30.23 |
| ds-m157 | 77.23 | 31.27 |
| ds-m158 | 71.67 | 36.96 |
| ds-m159 | 58.92 | 17.55 |
| ds-m160 | 66.33 | 8.84 |
| ds-m161 | 54.53 | 55.35 |
| ds-m162 | 51.20 | 50.71 |
| ds-m163 | 73.21 | 68.84 |
| ds-m164 | 76.10 | 30.80 |
| ds-m165 | 75.63 | 42.42 |
| ds-m166 | 63.48 | 24.92 |
| ds-m167 | 69.29 | 28.82 |
| ds-m168 | 60.55 | 1.85 |
| ds-m169 | 44.30 | 14.32 |
| ds-m170 | 58.23 | 2.46 |
| ds-m171 | 64.16 | 20.28 |
| ds-m172 | 56.38 | 7.52 |
| ds-m173 | 63.06 | 5.78 |
| ds-m174 | 56.66 | -4.96 |
| ds-m175 | 48.05 | -8.45 |
| ds-m176 | 58.11 | 2.12 |
| ds-m177 | 34.40 | 29.50 |
| ds-m178 | 54.45 | 3.69 |
| ds-m179 | 59.55 | 5.82 |
| ds-m180 | 61.38 | 1.56 |
| ds-m181 | 67.61 | 13.20 |
| ds-m182 | 62.33 | 29.54 |
| ds-m183 | 65.15 | 29.66 |
| ds-m184 | 49.00 | 37.92 |
| ds-m185 | 64.96 | 22.57 |
| ds-m186 | 62.33 | 45.46 |
| ds-m187 | 65.83 | 35.43 |
| ds-m188 | 82.16 | 23.55 |
| ds-m189 | 42.72 | 20.19 |

The results indicated that the aforementioned double-stranded siRNA could significantly suppress IGF-1R mRNA levels in SY5Y cells.

### Example 3 In Vitro Evaluation of Chemically Modified Double-Stranded siRNA in HeLa Cells

The double-stranded siRNA selected in Example 1 was further validated for its in vitro inhibitory efficacy in HeLa cells.

### 1) HeLa Cell Transfection

HeLa cells were cultured in DMEM medium supplemented with 10% fetal bovine serum and 1% penicillin-streptomycin, maintained in a humidified incubator at 37°C with 5% CO₂. Transfection was performed when the cells were in the logarithmic growth phase and in good condition. HeLa cells were seeded into 96-well plates and transfected with double-stranded siRNA using Lipofectamine RNAiMAX and Opti-MEM according to the manufacturer's instructions. In this experiment, the final concentrations of siRNA were 30 nM, 5 nM, and 0.5 nM.

### 2) RNA Extraction, Reverse Transcription, and qPCR

Total RNA was extracted using the E-Z 96 Total RNA Kit (Omega, R1034-02) according to the kit instructions. cDNA was synthesized using the FastKing RT Kit (With gDNase) (Tiangen, KR116-02) according to the manufacturer's instructions.

### 3) qPCR Analysis of Target Gene mRNA Expression Levels

The experimental procedure was identical to that described in Example 2.

### 4) Data Analysis

The data analysis procedure was the same as in Example 2.

The results are shown in Table 4.

**Table 4 Inhibition results of duplex siRNA in HeLa cells (percentage inhibition)**

| **double-stranded ribonucleic acid ID** | **HeLa (30 nM, %) 30 nM** | **HeLa (5 nM, %) 5 nM** | **HeLa (0.5 nM, %) 0.5 nM** |
|---|---|---|---|
| ds-m17 | 66.61 | 66.82 | 41.21 |
| ds-m23 | 73.41 | 76.22 | 69.86 |
| ds-m31 | 44.79 | 62.16 | 45.07 |
| ds-m39 | 82.03 | 83.06 | 60.20 |
| ds-m45 | 79.80 | 71.74 | 49.74 |
| ds-m58 | 77.52 | 68.14 | 60.06 |
| ds-m60 | 75.49 | 74.22 | 52.87 |
| ds-m64 | 76.70 | 58.78 | 41.24 |
| ds-m67 | 79.14 | 73.44 | 32.75 |
| ds-m68 | 72.76 | 53.58 | 21.47 |
| ds-m73 | 57.63 | 47.50 | 29.63 |
| ds-m74 | 52.69 | 34.63 | 11.42 |
| ds-m76 | 39.60 | 35.49 | 6.36 |
| ds-m77 | 45.44 | 11.20 | 2.75 |
| ds-m80 | 44.09 | 25.85 | -6.88 |
| ds-m81 | 62.29 | 29.58 | 6.58 |
| ds-m83 | 42.42 | 29.61 | 1.48 |
| ds-m84 | 32.18 | 36.87 | 7.14 |
| ds-m86 | 76.61 | 72.61 | 53.88 |
| ds-m88 | 69.46 | 65.77 | 57.50 |
| ds-m89 | 71.89 | 75.12 | 48.64 |
| ds-m98 | 76.06 | 69.79 | 48.01 |
| ds-m99 | 79.57 | 76.34 | 59.32 |
| ds-m103 | 71.81 | 56.86 | 47.80 |
| ds-m104 | 48.63 | 43.52 | 29.53 |
| ds-m112 | 35.32 | 56.73 | 28.79 |
| ds-m113 | 44.78 | 63.59 | 37.16 |
| ds-m115 | 41.82 | 44.47 | 28.44 |
| ds-m153 | 32.23 | 36.75 | 29.24 |
| ds-m154 | 42.24 | 43.04 | 35.39 |
| ds-m156 | 37.31 | 37.47 | 37.32 |
| ds-m157 | 36.98 | 45.19 | 29.64 |
| ds-m163 | 46.81 | 56.90 | 46.19 |
| ds-m164 | 40.06 | 53.30 | 38.23 |
| ds-m165 | 44.92 | 56.37 | 39.90 |
| ds-m188 | 23.65 | 33.76 | 7.45 |
| ds-m190 | 80.00 | 67.59 | 39.28 |
| ds-m191 | 72.45 | 68.88 | 20.59 |
| ds-m192 | 73.42 | 69.21 | 37.08 |
| ds-m193 | 78.63 | 66.16 | 35.04 |
| ds-m194 | 75.13 | 68.09 | 39.21 |
| ds-m195 | 67.16 | 71.22 | 43.24 |
| ds-m196 | 62.82 | 62.02 | 40.53 |
| ds-m197 | 71.95 | 70.32 | 46.82 |
| ds-m198 | 80.35 | 82.70 | 65.60 |

The results indicate that the aforementioned duplex siRNAs can significantly suppress IGF-1R mRNA levels in HeLa cells.

### Example 4 In vitro efficacy testing of chemically modified duplex siRNA in ARPE cells

Duplex siRNAs selected according to Example 1 were further validated for their in vitro inhibitory efficacy in ARPE cells.

### 1) ARPE cell transfection

ARPE-19 cells were cultured in DMEM medium containing 10% fetal bovine serum in a 5% CO₂ incubator at 37°C, and transfected when the cells were in logarithmic growth phase and in good condition. ARPE-19 cells were seeded into 96-well plates. Duplex siRNAs were transfected into HeLa cells using Lipofectamine RNAiMAX and Opti-MEM according to the manufacturer's instructions. The final siRNA concentrations tested were 30 nM, 0.5 nM, and 0.05 nM.

### 2) RNA extraction, reverse transcription, and qPCR

The experimental procedures were identical to those described in Example 2.

### 3) qPCR analysis of target gene mRNA expression levels

The experimental procedures were identical to those described in Example 2.

### 4) Data analysis

Data analysis was performed as described in Example 2.

The results are shown in Table 5.

**Table 5 Inhibition results of duplex siRNA in ARPE cells (percentage inhibition)**

| **double-stranded ribonucleic acid ID** | **ARPE (30 nM, %) 30 nM** | **ARPE (0.5 nM, %) 0.5 nM** | **ARPE (0.05 nM, %) 0.05 nM** |
|---|---|---|---|
| ds-m17 | 26.63 | 47.14 | 10.71 |
| ds-m23 | 39.43 | 63.42 | 59.58 |
| ds-m31 | 47.23 | 68.74 | 66.74 |
| ds-m39 | 40.82 | 28.61 | 16.70 |
| ds-m45 | 33.97 | 26.34 | 27.44 |
| ds-m58 | 42.36 | 24.59 | 8.85 |
| ds-m60 | 52.98 | 13.00 | -5.34 |
| ds-m64 | 52.32 | 25.59 | -0.52 |
| ds-m67 | 56.46 | 47.30 | 21.92 |
| ds-m68 | 73.21 | 60.73 | 43.02 |
| ds-m73 | 80.29 | 80.49 | 68.14 |
| ds-m74 | 65.88 | 57.87 | 60.85 |
| ds-m76 | 71.36 | 40.17 | 13.62 |
| ds-m77 | 53.39 | 43.92 | 15.37 |
| ds-m80 | 62.67 | 55.81 | 31.19 |
| ds-m81 | 75.94 | 74.01 | 53.08 |
| ds-m83 | 69.50 | 60.02 | 27.36 |
| ds-m84 | 62.14 | 40.64 | 19.48 |
| ds-m86 | 81.93 | 78.93 | 71.84 |
| ds-m88 | 73.07 | 60.59 | 33.55 |
| ds-m89 | 78.95 | 85.68 | 74.98 |
| ds-m98 | 75.91 | 83.07 | 72.99 |
| ds-m99 | 77.81 | 85.50 | 78.63 |
| ds-m103 | 78.85 | 74.86 | 67.70 |
| ds-m104 | 70.30 | 58.99 | 35.45 |
| ds-m112 | 76.37 | 70.93 | 62.98 |
| ds-m113 | 69.66 | 69.64 | 60.79 |
| ds-m115 | 77.26 | 69.49 | 63.12 |
| ds-m153 | 70.56 | 73.80 | 57.02 |
| ds-m154 | 76.38 | 78.43 | 63.01 |
| ds-m156 | 72.51 | 61.00 | 36.02 |
| ds-m157 | 70.69 | 70.04 | 41.87 |
| ds-m163 | 83.12 | 76.46 | 59.25 |
| ds-m164 | 66.07 | 64.96 | 39.32 |
| ds-m165 | 73.75 | 71.79 | 52.15 |
| ds-m188 | 76.11 | 64.21 | 34.16 |

The results indicated that the aforementioned double-stranded siRNA significantly suppressed IGF-1R mRNA levels in ARPE cells.

### Example 5 IC₅₀ Assay of Chemically Modified Double-Stranded siRNA in Human Primary Mature Adipocytes

The double-stranded siRNAs selected in Example 1 were further validated for their IC50 efficacy in mature human adipocytes.

### 5) Transfection of Human Primary Mature Adipocytes

Human primary mature adipocytes were cultured in adipocyte-specific medium in a humidified incubator with 5% CO₂ at 37°C. Transfection was performed when the cells were in the logarithmic growth phase and in good condition. Mature adipocytes were seeded into 24-well plates and transfected with double-stranded siRNA using Lipofectamine RNAiMAX and Opti-MEM according to the manufacturer's instructions.

### 6) RNA Extraction, Reverse Transcription, and qPCR

The experimental procedures were identical to those described in Example 2.

### 7) qPCR Analysis of Target Gene mRNA Expression Levels

The experimental procedures were identical to those described in Example 2.

### 8) Data Analysis

The results are shown in Table 6.

**Table 6 Inhibition Results of Double-Stranded siRNA in Human Primary Mature Adipocytes**

| **Double-Stranded Ribonucleic Acid ID** | **IC₅₀ (pM)** |
|---|---|
| ds-m23 | 41.8 |
| ds-m58 | 153.6 |
| ds-m67 | 894.1 |
| ds-m86 | 164.9 |
| ds-m89 | 124.8 |
| ds-m98 | 19.2 |
| ds-m99 | 16.0 |
| ds-m103 | 65.9 |
| ds-m190 | 47.9 |
| ds-m198 | 49.3 |
| ds-m113 | 1287.0 |

The results indicated that the aforementioned double-stranded siRNA significantly suppressed IGF-1R mRNA levels in human primary mature adipocytes.

### Example 6 Pharmacodynamic Study of Intravitreal Administration in Cynomolgus Monkeys

### Group Design

Six female cynomolgus monkeys were divided into two groups. Prior to dosing, a biopsy of the left eye was performed to collect small amounts of extraocular muscle and peribulbar fat. The collected samples were separately placed into homogenization tubes containing 0.5 mL RNAlater (2 tubes for extraocular muscle, 1 tube for fat), stored at 2°C-8°C for approximately 22-26 hours, and then the RNAlater was completely removed. The homogenization tubes containing tissue were rapidly frozen on dry ice and subsequently stored in a freezer at <-60°C. On Day 1 of the study, the left eye received PBS, while the right eye received the test article **Z4** at different doses (2 mg/kg, 10 mg/kg).

### Study Procedures

### 1. Clinical Observations

All animals underwent at least one detailed clinical observation prior to dosing, followed by detailed clinical observations on Days 2, 7, 14, 21, and 28 post-dosing.

### 2. Body Weight

All animals were weighed at least once prior to dosing and again on Day 28 or on the scheduled necropsy day.

### 3. Ophthalmic Examinations

All animals underwent at least one ophthalmic examination prior to dosing, followed by ophthalmic examinations on Days 2, 7, 14, 21, and 28 post-dosing.

### 4. Intraocular Pressure (IOP)

Intraocular pressure (IOP) of both eyes of all animals was measured using a TonoVet tonometer prior to dosing and again on Days 2 and 7 post-dosing.

### 5. Fundus Photography (FP)

Fundus photography (FP) of both eyes of all animals was performed at least once prior to dosing and again in Week 4, with images captured.

### 6. Flash Visual Evoked Potential (fVEP)

fVEP examination of both eyes of all animals was performed once in Week 4. Animals were anesthetized via intramuscular injection of ketamine (10-30 mg/kg) and xylazine (0.5 to 1.0 mg/kg). Animals were mydriaticized using an appropriate mydriatic agent. Flash VEP was conducted according to the following steps:
Select the FVEP protocol and enter animal information;
Place the animal in prone position on an adjustable platform and connect the relevant electrodes;
Check electrode impedance; once acceptable, click start to collect electrophysiological signals;
After evaluating the results, save the examination report.

### 7. Cytokine Analysis

Blood samples were collected from all available animals for cytokine analysis in serum. Blood sampling was performed once prior to dosing and once 24 hours post-dosing. Cytokine levels of IL-2, IL-4, IL-5, IL-6, IL-10, IL-13, TNF-α, and IFN-γ in serum were analyzed using a validated flow cytometry method.

### Terminal Necropsy

All study animals were euthanized on Day 29, and tissues were collected. During tissue collection, gross observations were made of the eyeballs, extraocular muscles (including superior rectus, superior oblique, medial rectus, lateral rectus, inferior oblique, and inferior rectus), and peribulbar fat.

Extraocular muscles (including superior rectus, superior oblique, medial rectus, lateral rectus, inferior oblique, and inferior rectus) and peribulbar fat were collected from both eyes of all study animals.

### IGF1R mRNA qPCR Analysis

IGF1R mRNA expression in cynomolgus monkey tissues was detected by RT-qPCR using a dye-based method. CT values of the target gene (IGF1R) and reference gene (CypA) were used to calculate changes in target gene expression. Tissue types included: extraocular muscles and peribulbar fat.

### 1. Key Primer Reagents

| Characteristic | Primer Lyophilized Powder Name | Primer Sequence (5' - 3') | Storage Conditions |
|---|---|---|---|
| Forward Primer (Cyp) | cCypA-F4 | GTCTCCTTCGAGCTGTTTGC | -30°C to -10°C |
| Reverse Primer (Cyp) | cCypA-R4 | CCACCCTGACACATAAACCCT | -30°C to -10°C |
| Forward Primer (GFI) | cIGF1R-F3 | CATCCGACGGGGGAATAACAT | -30°C to -10°C |
| Reverse Primer (GFI) | cIGF1R-R3 | GGTCCCAGTCCCATAGTTGC | -30°C to -10°C |

### 2. Key Equipment

qPCR thermal cycler, PCR thermal cycler, biosafety cabinet, homogenizer, cryogenic mill, electronic balance CyBio-SELMA liquid handling workstation, UV spectrophotometer, TECAN liquid handling workstation

### 3. RNA Extraction

Each tissue sample was mixed with 1000 µL TRNzol Universal RNA Reagent and three grinding beads (3 mm in diameter). The homogenizer program was set as follows: 65 Hz, 45 s/cycle, 4 cycles, with 30 s intervals between cycles (or 5.65 m/s, 1 min/cycle, 3 cycles, with 30 s intervals between cycles), yielding tissue lysates. Each tissue lysate was centrifuged (conditions: room temperature, 12000xg, 5 min), and the entire supernatant was transferred to a new Eppendorf tube. Incubate at room temperature for 5 minutes to allow complete dissociation of the nuclear protein complexes. Add 200 µL of chloroform to each tissue lysate, vortex to mix thoroughly, spin briefly, and incubate at room temperature for 3 min. Centrifuge (centrifugation conditions: 4°C, 12,000×g, 5 min). After centrifugation, the tissue lysate separates into upper, middle, and lower aqueous layers; transfer 450 µL of the upper aqueous layer from each sample into a new Eppendorf tube. Add 45 µL of 3 M Sodium Acetate Solution, pH 5.2, to each sample, vortex to mix thoroughly, and incubate at room temperature for 1 min. After incubation, add 500 µL of isopropanol (pre-chilled) to each sample, vortex to mix thoroughly, and incubate at -30°C to -10°C for 30 min. After incubation, centrifuge (centrifugation conditions: 4°C, 12,000×g, 15 min); after centrifugation, an RNA pellet is visible at the bottom of the tube-discard the supernatant. After discarding the supernatant, add 1,000 µL of 75% ethanol solution (pre-chilled) to each sample, vortex to mix thoroughly, and centrifuge (centrifugation conditions: 4°C, 12,000×g, 10 min). After centrifugation, discard the supernatant, air-dry at room temperature for 10 min, then add 40 µL of DEPC-treated water (pre-warmed to 55°C) to each sample, vortex to mix thoroughly, to obtain RNA. Determine purity and concentration by measuring A260/A280 using a UV spectrophotometer and record the results. The RNA can be used directly for RT-qPCR or stored at < -60°C. The RNA has a shelf life of 1 year.

### 4 qPCR Analysis

The RNA is processed through a genomic DNA removal procedure to yield "RNA template (DNA-free)," which is then subjected to a PCR thermal cycler program to generate cDNA product as the "qPCR template." All cDNA samples are analyzed by qPCR in 384-well plates with triplicate wells for each sample. Relative expression data will be acquired using QuantStudio 7 Flex PCR, including: mean Ct, ΔCt, and ΔΔCt. All IGF1R mRNA KD% values were calculated following normalization to biopsy samples.

### Experimental Results:

1. No abnormalities were observed in clinical observations, body weight, intraocular pressure, fundus photography, flash visual evoked potentials (fVEP), or cytokine analysis;
2. KD% experimental results: as shown in Figure 1.

Conclusion: Orbital administration of the double-stranded compound Z4 demonstrated excellent safety and significantly inhibited IGF1R mRNA expression in the target tissue.

### Example 7: Mouse Acute Exophthalmos Model

Objective: To evaluate the efficacy of mouse IGF1R siRNA Z8 and Z9 administered via orbital injection in a BALB/c mouse model of thyroid eye disease.

### 1 Grouping and Dosing Schedule

On Day -5, animals were anesthetized with Telazol (25-50 mg/kg, i.p.) and xylazine hydrochloride (5 mg/kg, i.p.), followed by a single orbital injection of mouse IGF1R siRNA into both eyes (100 µL per eye).

### 2 Model Induction

On Day 0 and Day 6, animals were anesthetized with Telazol (25-50 mg/kg, i.p.) and xylazine hydrochloride (5 mg/kg, i.p.), followed by periocular injection of mouse IGF-1 protein into both eyes (5 µg per eye) to induce the model. After injection, both eyes of the animals were treated with levofloxacin eye drops and ofloxacin ointment once in the morning and once in the afternoon for three consecutive days.

### 3 Assessment Parameters

### 3.1 Clinical Observations

Animals were observed once daily at the cage side for general condition, mental status, behavior, activity, and food intake.

### 3.2 GO (Graves' Ophthalmopathy) Scoring

GO scoring was performed on Day 0 (pre-modeling), Day 3, Day 6 (pre-modeling), and Day 9 to monitor persistent exophthalmos symptoms.

### 3.3 Ocular Examination

Slit-lamp examinations of the ocular surface and fundus were performed on Day -5 (before and after dosing), Day 0, Day 3, Day 7, and Day 14. Photographic documentation was made if any abnormalities were observed.

### 3.4 Tissue Collection

On Day 9, four animals per group were euthanized; peri-orbital tissues, retina-sclera complexes, liver, skeletal muscle, and ovarian fat were immediately preserved in RNAprotect Tissue Reagent.

### 3.5 qPCR Analysis

RNA was extracted from peri-orbital tissues and subjected to reverse transcription and qPCR analysis.

### 3.6 Histopathology

On Day 9, eyeballs (with attached peri-orbital tissues) from four mice per group were fixed, paraffin-embedded, and evaluated for peri-orbital tissue hyperplasia and cellular infiltration (H&E staining, UCPI, and CD3 staining).

Orbital administration of mouse IGF1R siRNA significantly suppressed IGF1R mRNA expression and alleviated exophthalmos symptoms, suggesting that orbital administration of the compounds described herein may effectively treat thyroid eye disease in humans.

### Experimental Example 7: Rat Surgically Induced Osteoarthritis Model

At 10 weeks of age, 24 rats underwent surgical transection of the anterior cruciate ligament, medial collateral ligament, and medial meniscotibial ligament (anterior cruciate ligament transection and partial medial meniscectomy model; ACLT + pMMx). One week post-surgery, all rats were randomly assigned to receive intra-articular injections of mouse IGF1R siRNA Z8, Z9, or control solution (12 rats per group). Thirteen weeks post-surgery, knee joints were harvested and fixed in 10% formalin, decalcified, and embedded in paraffin blocks. Anterior sections were cut at different levels (5 µm thickness, spaced 100 µm apart to ensure reproducibility) and stained with Safranin O/Fast Green. At least 12 sections per rat were collected and imaged using light microscopy.

### Osteoarthritis Research Society International (OARSI) Scoring, Cartilage Protection, and Regeneration

Histological evaluation was performed by two blinded observers. Images were scored according to the OARSI histological scoring system for cartilage, based on the depth of cartilage damage and the extent of joint involvement. Briefly, femur and tibia were assessed separately; each site was scored based on the grade of cartilage damage (0-6, where 0 = intact surface and 6 = severe deformation) and the stage of cartilage damage (0-4, where 0 = normal joint and 4 = >50% joint involvement). The total score was the product of grade and stage (0 = normal joint; 24 = severe osteoarthritis). Twelve sections per rat were scored, and no rats were excluded. After scoring, the study was unblinded, and the four lowest-scoring sections per rat (representing the mildest cartilage damage) were excluded from further analysis. The mean OARSI score per rat was calculated as the average of the scores from the two blinded observers. Additionally, histological evaluation was repeated by four independent blinded observers using a revised objective quantitative histological OARSI scoring system. Safranin O staining intensity and cartilage thickness were measured using ImageJ.

Intra-articular administration of mouse IGF1R siRNA significantly suppressed IGF1R mRNA expression and reduced OARSI scores, suggesting that intra-articular administration of the compounds described herein may effectively treat osteoarthritis in humans.

### Example 8: Spinal Nerve Ligation (SNL) Model

Animals were initially anesthetized with 5% isoflurane and maintained under 2% isoflurane. The L5 transverse process was removed to expose the L3 and L4 spinal nerves. The L4 spinal nerve was then isolated and tightly ligated with 6-0 silk suture. For the sham surgery group, the L4 spinal nerve was exposed but not ligated. Under isoflurane anesthesia, mouse IGF1R siRNA Z8, Z9, or control solution was administered via lumbar puncture, followed by behavioral testing.

### Behavioral Testing

### 50% Paw Withdrawal Mechanical Threshold (PWT):

Acclimatization: Rats were acclimated to the testing area for at least three days prior to measurements. The plantar surface of the paw was stimulated vertically using von Frey filaments of varying strengths (0.6 g, 1.0 g, 1.4 g, 2.0 g, 4.0 g, 6.0 g, 8.0 g, 10.0 g, and 15.0 g). Measurements were taken one day before surgery and on postoperative Days 4, 7, 10, and 14. Testing began with the 4.0 g filament, and the 50% PWT threshold for each rat was calculated using the up-down method. The proportion of paw withdrawal responses to increasing mechanical stimuli was measured. Three groups of rats were sequentially evaluated, with five measurements per side (each separated by 5 minutes). The final paw withdrawal response rate was calculated as the proportion of responses to the 6.0 g mechanical stimulus across the five trials.

### Cold Allodynia Threshold:

To assess cold sensitivity of the paw, the rat's response to acetone was measured. While the rat was quiet, precooled acetone (100 µL) was applied to the plantar surface of the paw. The duration of positive responses (e.g., paw lifting, shaking, licking) was recorded over 30 seconds. Each paw was tested three times (each separated by 5 minutes), and the average of the three measurements was recorded as the response time for that side.

Intrathecal administration of mouse IGF1R siRNA significantly suppressed IGF1R mRNA expression and improved both PWT and cold allodynia threshold scores, inhibiting pain responses, suggesting that intrathecal administration of the compounds described herein may effectively treat neuropathic pain in humans.

## Claims

1. An RNA inhibitor for suppressing the expression of the insulin-like growth factor 1 receptor (IGF-1R) gene, comprising an antisense strand that forms a complementary region with at least 15 consecutive nucleotides in the mRNA encoding IGF-1R (SEQ ID NO: 828), wherein the complementary region has 0, 1, 2, 3, 4, or 5 mismatches; preferably, the length of the complementary region is 15-30 nucleotide pairs, more preferably 17-23 nucleotide pairs.

2. The RNA inhibitor according to claim 1, wherein the antisense strand forms a complementary region with 15, 16, 17, 18, 19, 20, 21, 22, or 23 consecutive nucleotides starting from any one of the following positions in the mRNA encoding IGF-1R (SEQ ID NO: 828), as counted from the 5' end:
the nucleotide at position 1118, the nucleotide at position 1120, the nucleotide at position 1154, the nucleotide at position 1367, the nucleotide at position 1407, the nucleotide at position 1415, the nucleotide at position 1532, the nucleotide at position 1625, the nucleotide at position 1627, the nucleotide at position 1628, the nucleotide at position 1631, the nucleotide at position 3356, the nucleotide at position 3357, the nucleotide at position 3359, the nucleotide at position 3792, the nucleotide at position 4198, the nucleotide at position 4200, the nucleotide at position 4208, the nucleotide at position 4685, the nucleotide at position 5246, the nucleotide at position 5392, the nucleotide at position 5393, the nucleotide at position 6329, the nucleotide at position 6332, the nucleotide at position 6333, the nucleotide at position 6334, the nucleotide at position 10215, the nucleotide at position 10738, the nucleotide at position 10740, the nucleotide at position 10770, the nucleotide at position 10772, the nucleotide at position 10773, the nucleotide at position 10946, the nucleotide at position 10956, the nucleotide at position 10957, the nucleotide at position 1418, the nucleotide at position 1541, the nucleotide at position 2050, the nucleotide at position 2055, the nucleotide at position 2056, the nucleotide at position 2233, the nucleotide at position 2234, the nucleotide at position 2456, the nucleotide at position 2591, the nucleotide at position 2603, the nucleotide at position 2606, the nucleotide at position 2609, the nucleotide at position 2776, the nucleotide at position 2890, the nucleotide at position 2906, the nucleotide at position 2909, the nucleotide at position 2957, the nucleotide at position 2960, the nucleotide at position 3008, the nucleotide at position 3195, the nucleotide at position 3398, the nucleotide at position 3608, the nucleotide at position 3609, the nucleotide at position 3650, the nucleotide at position 3660, the nucleotide at position 3815, the nucleotide at position 3882, the nucleotide at position 3996, the nucleotide at position 4067, the nucleotide at position 4121, the nucleotide at position 4122, the nucleotide at position 4292, the nucleotide at position 4293, the nucleotide at position 4295, the nucleotide at position 4516, the nucleotide at position 4519, the nucleotide at position 5163, the nucleotide at position 5309, the nucleotide at position 5310, the nucleotide at position 5377, the nucleotide at position 5380, the nucleotide at position 5381, the nucleotide at position 5663, the nucleotide at position 5700, the nucleotide at position 5711, the nucleotide at position 5712, the nucleotide at position 5721, the nucleotide at position 5896, the nucleotide at position 5898, the nucleotide at position 6203, the nucleotide at position 6236, the nucleotide at position 6237, the nucleotide at position 6240, the nucleotide at position 6245, the nucleotide at position 6288, the nucleotide at position 6299, the nucleotide at position 6305, the nucleotide at position 6309, the nucleotide at position 6310, the nucleotide at position 6343, the nucleotide at position 6363, the nucleotide at position 6365, the nucleotide at position 6366, the nucleotide at position 6373, the nucleotide at position 6379, the nucleotide at position 6381, the nucleotide at position 6431, the nucleotide at position 6434, the nucleotide at position 6496, the nucleotide at position 6518, the nucleotide at position 6755, the nucleotide at position 6760, the nucleotide at position 6883, the nucleotide at position 6885, the nucleotide at position 6947, the nucleotide at position 6948, the nucleotide at position 6949, the nucleotide at position 6952, the nucleotide at position 6953, the nucleotide at position 6954, the nucleotide at position 7574, the nucleotide at position 7621, the nucleotide at position 7764, the nucleotide at position 7830, the nucleotide at position 7906, the nucleotide at position 8540, the nucleotide at position 8836, the nucleotide at position 8892, the nucleotide at position 8975, the nucleotide at position 8977, the nucleotide at position 9163, the nucleotide at position 9175, the nucleotide at position 9186, the nucleotide at position 9265, the nucleotide at position 9267, the nucleotide at position 9340, the nucleotide at position 9347, the nucleotide at position 9352, the nucleotide at position 9356, the nucleotide at position 9478, the nucleotide at position 9480, the nucleotide at position 9606, the nucleotide at position 9611, the nucleotide at position 9634, the nucleotide at position 9680, the nucleotide at position 9681, the nucleotide at position 9724, the nucleotide at position 9725, the nucleotide at position 9814, the nucleotide at position 10015, the nucleotide at position 10016, the nucleotide at position 10189, the nucleotide at position 10244, the nucleotide at position 10266, the nucleotide at position 10490, the nucleotide at position 10526, the nucleotide at position 10528, the nucleotide at position 10534, the nucleotide at position 10535, the nucleotide at position 10537, the nucleotide at position 10540, the nucleotide at position 10541, the nucleotide at position 10679, the nucleotide at position 10694, the nucleotide at position 10717, the nucleotide at position 10728, the nucleotide at position 10729, the nucleotide at position 10730, the nucleotide at position 10754, the nucleotide at position 10763, the nucleotide at position 10766, the nucleotide at position 10942, the nucleotide at position 11034, the nucleotide at position 11078, the nucleotide at position 11126, the nucleotide at position 11156, the nucleotide at position 11159, the nucleotide at position 11160, the nucleotide at position 11292, the nucleotide at position 11340, the nucleotide at position 11344, the nucleotide at position 11383, the nucleotide at position 11395, the nucleotide at position 11789, the nucleotide at position 11864, the nucleotide at position 11865, the nucleotide at position 11939, the nucleotide at position 12092, the nucleotide at position 12116, the nucleotide at position 12130, the nucleotide at position 12154, the nucleotide at position 12156, the nucleotide at position 12177, the nucleotide at position 12195, the nucleotide at position 1164, the nucleotide at position 1163, the nucleotide at position 1162, the nucleotide at position 1161, the nucleotide at position 1160, the nucleotide at position 1165, the nucleotide at position 1166, the nucleotide at position 1167, the nucleotide at position 1168.

3. The RNA inhibitor according to any one of claims 1-2, wherein the antisense strand forms a complementary region with the sequence between nucleotide 1160 and nucleotide 1188, or between nucleotide 6235 and nucleotide 6465, or between nucleotide 6329 and nucleotide 6455, counting from the 5' end of the mRNA encoding IGF-1R (SEQ ID NO: 828).

4. The RNA inhibitor according to any one of claims 1-3, wherein the RNA inhibitor is ribonucleic acid; further, the RNA inhibitor is single-stranded ribonucleic acid or double-stranded ribonucleic acid.

5. The RNA inhibitor according to any one of claims 1-4, wherein the RNA inhibitor is an antisense oligonucleotide (ASO), shRNA, miRNA, or siRNA.

6. The RNA inhibitor according to any one of claims 1-5, comprising a sense strand capable of forming a complementary duplex with the antisense strand, wherein the antisense strand comprises a sequence forming a duplex complementary region with at least 15 consecutive nucleotides of the sense strand, wherein the duplex complementary region has 0, 1, 2, 3, 4, or 5 mismatches; preferably, the length of the duplex complementary region is 15-30 nucleotide pairs, more preferably 17-23 nucleotide pairs.

7. The RNA inhibitor according to claim 6, wherein the sense strand and the antisense strand reside on two separate nucleic acid strands; preferably, the RNA inhibitor is siRNA or shRNA; more preferably, the RNA inhibitor is siRNA.

8. The RNA inhibitor according to any one of claims 6-7, wherein the sense strand and the antisense strand reside on the same nucleic acid strand; preferably, the RNA inhibitor is shRNA.

9. The RNA inhibitor according to any one of claims 6-8, wherein the total length of the sense strand is 15-50 nucleotides; preferably, the total length of the sense strand is 16-30 nucleotides; more preferably, the total length of the sense strand is 17, 18, 19, 20, or 21 nucleotides.

10. The RNA inhibitor according to any one of claims 6-9, wherein the total length of the antisense strand is 19-50 nucleotides; preferably, the total length of the antisense strand is 19-30 nucleotides; more preferably, the total length of the antisense strand is 21, 22, 23, 24, 25, 26, or 27 nucleotides.

11. The RNA inhibitor according to any one of claims 6-10, wherein the sense strand and the antisense strand independently optionally comprise a 3' or 5' overhang of 1, 2, or 3 nucleotides.

12. The RNA inhibitor according to claim 11, wherein both the sense strand and the antisense strand have a 3' overhang of 1-3 nucleotides, or the sense strand has a 3' or 5' overhang of 1-3 nucleotides, or the antisense strand has a 3' or 5' overhang of 1-3 nucleotides.

13. The RNA inhibitor according to any one of claims 1-12, wherein the antisense strand comprises at least 15 consecutive nucleotides from any one of the sequences set forth in SEQ **ID** NO: 199-400.

14. The RNA inhibitor according to any one of claims 6-13, wherein the sense strand comprises at least 15 consecutive nucleotides from any one of the sequences set forth in SEQ **ID** NO: 1-198.

15. The RNA inhibitor according to any one of claims 1-14, comprising a duplex selected from the following: ds-n1, ds-n2, ds-n3, ds-n4, ds-n5, ds-n6, ds-n7, ds-n8, ds-n9, ds-n10, ds-n11, ds-n12, ds-n13, ds-n14, ds-n15, ds-n16, ds-n17, ds-n18, ds-n19, ds-n20, ds-n21, ds-n22, ds-n23, ds-n24, ds-n25, ds-n26, ds-n27, ds-n28, ds-n29, ds-n30, ds-n31, ds-n32, ds-n33, ds-n34, ds-n35, ds-n36, ds-n37, ds-n38, ds-n39, ds-n40, ds-n41, ds-n42, ds-n43, ds-n44, ds-n45, ds-n46, ds-n47, ds-n48, ds-n49, ds-n50, ds-n51, ds-n52, ds-n53, ds-n54, ds-n55, ds-n56, ds-n57, ds-n58, ds-n59, ds-n60, ds-n61, ds-n62, ds-n63, ds-n64, ds-n65, ds-n66, ds-n67, ds-n68, ds-n69, ds-n70, ds-n71, ds-n72, ds-n73, ds-n74, ds-n75, ds-n76, ds-n77, ds-n78, ds-n79, ds-n80, ds-n81, ds-n82, ds-n83, ds-n84, ds-n85, ds-n86, ds-n87, ds-n88, ds-n89, ds-n90, ds-n91, ds-n92, ds-n93, ds-n94, ds-n95, ds-n96, ds-n97, ds-n98, ds-n99, ds-n100, ds-n101, ds-n102, ds-n103, ds-n104, ds-n105, ds-n106, ds-n107, ds-n108, ds-n109, ds-n110, ds-n111, ds-n112, ds-n113, ds-n114, ds-n115, ds-n116, ds-n117, ds-n118, ds-n119, ds-n120, ds-n121, ds-n122, ds-n123, ds-n124, ds-n125, ds-n126, ds-n127, ds-n128, ds-n129, ds-n130, ds-n131, ds-n132, ds-n133, ds-n134, ds-n135, ds-n136, ds-n137, ds-n138, ds-n139, ds-n140, ds-n141, ds-n142, ds-n143, ds-n144, ds-n145, ds-n146, ds-n147, ds-n148, ds-n149, ds-n150, ds-n151, ds-n152, ds-n153, ds-n154, ds-n155, ds-n156, ds-n157, ds-n158, ds-n159, ds-n160, ds-n161, ds-n162, ds-n163, ds-n164, ds-n165, ds-n166, ds-n167, ds-n168, ds-n169, ds-n170, ds-n171, ds-n172, ds-n173, ds-n174, ds-n175, ds-n176, ds-n177, ds-n178, ds-n179, ds-n180, ds-n181, ds-n182, ds-n183, ds-n184, ds-n185, ds-n186, ds-n187, ds-n188, ds-n189, ds-n190, ds-n191, ds-n192, ds-n193, ds-n194, ds-n195, ds-n196, ds-n197, ds-n198, ds-n201, ds-n202, ds-n203, ds-n204.

16. The RNA inhibitor according to claim 15, comprising a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188, ds-n190, ds-n191, ds-n192, ds-n193, ds-n194, ds-n195, ds-n196, ds-n197, ds-n198.

17. The RNA inhibitor according to claim 15, comprising a duplex selected from the following: ds-n17, ds-n23, ds-n31, ds-n39, ds-n45, ds-n58, ds-n60, ds-n64, ds-n67, ds-n68, ds-n73, ds-n74, ds-n76, ds-n77, ds-n80, ds-n81, ds-n83, ds-n84, ds-n86, ds-n88, ds-n89, ds-n98, ds-n99, ds-n103, ds-n104, ds-n112, ds-n113, ds-n115, ds-n153, ds-n154, ds-n156, ds-n157, ds-n163, ds-n164, ds-n165, ds-n188.

18. The RNA inhibitor according to claim 16, comprising a duplex selected from the following: ds-n23, ds-n58, ds-n67, ds-n86, ds-n89, ds-n98, ds-n99, ds-n103, ds-n190, ds-n198, ds-n113.

19. The RNA inhibitor according to any one of claims 1-18, wherein the antisense strand further comprises a region B1 at its 3' end, and the sense strand further comprises a region A1 at its 5' end, wherein region A1 includes 0-6 nucleotides and region B1 includes 0-6 nucleotides.

20. The RNA inhibitor according to claim 19, wherein region B1 includes 0, 1, 2, 3, 4, 5, or 6 nucleotides; preferably, region B1 includes 0 or 2 nucleotides.

21. The RNA inhibitor according to any one of claims 16-20, wherein region A1 includes 0, 1, 2, 3, 4, 5, or 6 nucleotides; preferably, region A1 includes 0 or 2 nucleotides.

22. The RNA inhibitor according to claim 21, wherein the antisense strand further comprises a region X2 at its 5' end, and the sense strand or sense nucleic acid further comprises a region X1 at its 3' end, wherein regions X1 and X2 are complementary and base-pair with each other.

23. The RNA inhibitor according to claim 22, wherein X1 is A, U, modified A, or modified U, and X2 is U, A, modified U, or modified A.

24. The RNA inhibitor according to any one of claims 19-23, wherein the antisense strand, proceeding from the 3' to the 5' direction at its 5' end, sequentially comprises X2, Y, Z, and N, wherein X2 and Y are independently A, U, modified A, or modified U, Z is G or modified G, and N comprises at least one nucleotide.

25. The RNA inhibitor according to any one of claims 19-24, wherein X2, Y, and Z, proceeding from the 3' to the 5' direction, are sequentially AAG, AUG, UUG, or UAG, or partially or fully modified sequences thereof.

26. The RNA inhibitor according to any one of claims 21-25, wherein X2, Y, and Z, proceeding from the 3' to the 5' direction, are sequentially AAG, AUG, UUG, or UAG, or partially or fully modified sequences thereof.

27. The RNA inhibitor according to any one of claims 21-26, wherein N is C or modified C.

28. The RNA inhibitor according to any one of claims 21-27, wherein X2, Y, Z, and N, proceeding from the 3' to the 5' direction, are sequentially AAGC and UAGC, or partially or fully modified sequences thereof.

29. The RNA inhibitor according to any one of claims 16-28, wherein the antisense strand comprises at least 15 consecutive nucleotides from any one of the sequences set forth in SEQ ID NO: 401-413.

30. The RNA inhibitor according to any one of claims 1-29, selected from ds-n190-1, ds-n191-1, ds-n192-1, ds-n193-1, ds-n194-1, ds-n195-1, ds-n196-1, ds-n197-1, ds-n198-1, ds-n201-1, ds-n202-1, ds-n203-1, ds-n204-1.

31. The RNA inhibitor according to any one of claims 1-30, wherein at least one nucleotide in the RNA inhibitor is a modified nucleotide.

32. The RNA inhibitor according to any one of claims 1-31, wherein at least 70%, 80%, 90%, or 95% of the nucleotides in the RNA inhibitor are modified nucleotides; preferably, all nucleotides are modified nucleotides.

33. The RNA inhibitor according to any one of claims 31-32, wherein the modification comprises one or more of the following: 2'-OMe (2'-O-methyl) modification, 2'-F (2'-deoxy-2'-fluoro) modification, 2'-O-MOE (2'-O-methoxyethyl) modification, 2'-deoxy (2'-d) modification, 5'-morpholino (5'-Mo) modification, unlocked nucleic acid (UNA) modification, glycol nucleic acid (GNA) modification, locked nucleic acid (LNA) modification, tricyclo-DNA (tcDNA) modification, (S)-constrained ethyl bicyclic nucleic acid ((S)-cEt-BNA) modification, phosphorothioate (PS) modification, dithiophosphate (PS2) modification, methylphosphonate (MP) modification, methoxypropyl methylphosphonate (MOP) modification, peptide nucleic acid (PNA) modification, 5'-(E)-vinylphosphonate (VP) modification, N6-methyladenosine (m6A) modification, 5-methylcytidine (m5C) modification, 3-methyluridine (m3U) modification, 5-methyluridine (m5U) modification, pseudouridine modification, 2-thiouridine (s2U) modification, propargyluridine (5-pU) modification, linkage of an inverted abasic nucleotide (invAB) at the 5' or 3' end of a nucleotide, replacement of a nucleotide with an inverted abasic nucleotide (invAb), replacement of a nucleotide with 2,4-difluorotolyl ribonucleotide (rF), or replacement of a nucleotide with (S)-glycerol nucleic acid modification; preferred modifications are 2'-OMe modification, 2'-F modification, 2'-deoxy modification, VP modification, 5'-MP modification, PS modification, PS2 modification, MP modification, MOP modification, M06 modification, invAb modification, or invAB modification.

34. The RNA inhibitor according to any one of claims 31-33, wherein the 3' or 5' end of the antisense strand comprises a phosphate or phosphate mimic, or the 3' or 5' end of the sense strand comprises a phosphate or phosphate mimic.

35. The RNA inhibitor according to claim 34, wherein the phosphate mimic includes 5'-(E)-vinylphosphonate, 5'-methylphosphonate, (S)-5'-C-methyl analog, and 5'-phosphorothioate (5'-PS).

36. The RNA inhibitor according to any one of claims 31-35, wherein the 5' end of the antisense strand comprises an (M06) modification:

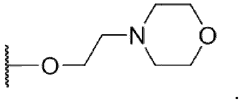

37. The RNA inhibitor according to any one of claims 31-36, wherein the 3' or 5' end of the antisense strand bears an invAB modification, or the 3' or 5' end of the sense strand bears an invAB modification.

38. The RNA inhibitor according to any one of claims 31-37, wherein the inverted abasic nucleotide or M06 is linked via a phosphorothioate bond to the 3' or 5' end of the antisense strand or to the 3' or 5' end of the sense strand.

39. The RNA inhibitor according to any one of claims 31-38, having the following combination of modifications:
a) the sense strand comprises a first sequence of 16-21 nt, wherein the first sequence comprises the following modifications: counting from the 5' end, the nucleotides at positions 9, 10, and 11 bear 2'-F modifications;
b) the antisense strand comprises a second sequence of 16-21 nt, wherein the second sequence comprises the following modifications: counting from the 5' end, a phosphorothioate linkage exists between nucleotides 1 and 2, between nucleotides 2 and 3, and between nucleotides 3 and 4; and counting from the 3' end, a phosphorothioate linkage exists between nucleotides 1 and 2; and counting from the 5' end, the nucleotides at positions 2, 3, 4, 12, 14, and 16 bear 2'-F modifications.

40. The RNA inhibitor according to claim 39, wherein the length of the first sequence is preferably 16 nt, 17 nt, 18 nt, 19 nt, 20 nt, or 21 nt, more preferably 21 nt.

41. The RNA inhibitor according to claim 39, wherein the length of the second sequence is preferably 16 nt, 17 nt, 18 nt, 19 nt, 20 nt, or 21 nt, more preferably 21 nt.

42. The RNA inhibitor according to any one of claims 39-41, having the following combination of modifications:
a) the sense strand is 21 nt in length and comprises the following modifications: counting from the 5' end, the nucleotides at positions 9, 10, and 11 bear 2'-F modifications;
b) the antisense strand is 21 nt in length and comprises the following modifications: counting from the 5' end, a phosphorothioate linkage exists between nucleotides 1 and 2, between nucleotides 2 and 3, and between nucleotides 3 and 4; and counting from the 3' end, a phosphorothioate linkage exists between nucleotides 1 and 2; and counting from the 5' end, the nucleotides at positions 2, 3, 4, 12, 14, and 16 bear 2'-F modifications.

43. The RNA inhibitor according to any one of claims 28-42, wherein the antisense strand comprises at least 15 consecutive nucleotides from any one of the sequences set forth in SEQ ID NO: 624-812.

44. The RNA inhibitor according to any one of claims 28-42, wherein the sense strand comprises at least 15 consecutive nucleotides from any one of the sequences set forth in SEQ ID NO: 414-602.

45. The RNA inhibitor according to any one of claims 28-44, selected from:
ds-m1, ds-m2, ds-m3, ds-m4, ds-m5, ds-m6, ds-m7, ds-m8, ds-m9, ds-m10, ds-m11, ds-m12, ds-m13, ds-m14, ds-m15, ds-m16, ds-m17, ds-m18, ds-m19, ds-m20, ds-m21, ds-m22, ds-m23, ds-m24, ds-m25, ds-m26, ds-m27, ds-m28, ds-m29, ds-m30, ds-m31, ds-m32, ds-m33, ds-m34, ds-m35, ds-m36, ds-m37, ds-m38, ds-m39, ds-m40, ds-m41, ds-m42, ds-m43, ds-m44, ds-m45, ds-m46, ds-m47, ds-m48, ds-m49, ds-m50, ds-m51, ds-m52, ds-m53, ds-m54, ds-m55, ds-m56, ds-m57, ds-m58, ds-m59, ds-m60, ds-m61, ds-m62, ds-m63, ds-m64, ds-m65, ds-m66, ds-m67, ds-m68, ds-m69, ds-m70, ds-m71, ds-m72, ds-m73, ds-m74, ds-m75, ds-m76, ds-m77, ds-m78, ds-m79, ds-m80, ds-m81, ds-m82, ds-m83, ds-m84, ds-m85, ds-m86, ds-m87, ds-m88, ds-m89, ds-m90, ds-m91, ds-m92, ds-m93, ds-m94, ds-m95, ds-m96, ds-m97, ds-m98, ds-m99, ds-m100, ds-m101, ds-m102, ds-m103, ds-m104, ds-m105, ds-m106, ds-m107, ds-m108, ds-m109, ds-m110, ds-m111, ds-m112, ds-m113, ds-m114, ds-m115, ds-m116, ds-m117, ds-m118, ds-m119, ds-m120, ds-m121, ds-m122, ds-m123, ds-m124, ds-m125, ds-m126, ds-m127, ds-m128, ds-m129, ds-m130, ds-m131, ds-m132, ds-m133, ds-m134, ds-m135, ds-m136, ds-m137, ds-m138, ds-m139, ds-m140, ds-m141, ds-m142, ds-m143, ds-m144, ds-m145, ds-m146, ds-m147, ds-m148, ds-m149, ds-m150, ds-m151, ds-m152, ds-m153, ds-m154, ds-m155, ds-m156, ds-m157, ds-m158, ds-m159, ds-m160, ds-m161, ds-m162, ds-m163, ds-m164, ds-m165, ds-m166, ds-m167, ds-m168, ds-m169, ds-m170, ds-m171, ds-m172, ds-m173, ds-m174, ds-m175, ds-m176, ds-m177, ds-m178, ds-m179, ds-m180, ds-m181, ds-m182, ds-m183, ds-m184, ds-m185, ds-m186, ds-m187, ds-m188, ds-m189.

46. The RNA inhibitor according to any one of claims 28-39, wherein
a) the sense strand comprises a third sequence of 18-21 nt, wherein the third sequence comprises the following modifications: counting from the 5' end, a phosphorothioate linkage exists between nucleotides 1 and 2 and between nucleotides 2 and 3 of the sense strand; the nucleotides at positions 9, 10, 11, and 18 of the sense strand bear 2'-F modifications, and the remaining nucleotides bear 2'-OMe modifications; counting from the 3' end, a phosphorothioate linkage exists between nucleotides 1 and 2 and between nucleotides 2 and 3;
b) the antisense strand comprises a fourth sequence of 19-26 nt, wherein the fourth sequence comprises the following modifications: counting from the 5' end, the nucleotides at positions 1, 2, 5, 9, 17, and 19 of the antisense strand bear 2'-F modifications, and the remaining nucleotides bear 2'-OMe modifications; a phosphorothioate linkage exists between nucleotides 4 and 5; counting from the 3' end, a phosphorothioate linkage exists between nucleotides 1 and 2 and between nucleotides 2 and 3.

47. The RNA inhibitor according to claim 46, wherein the length of the third sequence is preferably 18 nt, 19 nt, 20 nt, 21 nt, or 22 nt, more preferably 21 nt.

48. The RNA inhibitor according to claim 47, wherein the length of the fourth sequence is preferably 19 nt, 20 nt, 21 nt, 22 nt, 23 nt, 24 nt, 25 nt, or 26 nt, more preferably 26 nt.

49. The RNA inhibitor according to any one of claims 46-48, wherein
a) the sense strand is 21 nt in length; counting from the 5' end, a phosphorothioate linkage exists between nucleotides 1 and 2 and between nucleotides 2 and 3 of the sense strand; the nucleotides at positions 9, 10, 11, and 18 of the sense strand bear 2'-F modifications, and the remaining nucleotides bear 2'-OMe modifications; counting from the 3' end, a phosphorothioate linkage exists between nucleotides 1 and 2 and between nucleotides 2 and 3;
b) the antisense strand is 26 nt in length; counting from the 5' end, the nucleotides at positions 1, 2, 5, 9, 17, and 19 of the antisense strand bear 2'-F modifications, and the remaining nucleotides bear 2'-OMe modifications; a phosphorothioate linkage exists between nucleotides 4 and 5; counting from the 3' end, a phosphorothioate linkage exists between nucleotides 1 and 2 and between nucleotides 2 and 3.

50. The RNA inhibitor according to any one of claims 31-49, wherein the antisense strand comprises at least 15 consecutive nucleotides from any one of the sequences set forth in SEQ ID NO: 813-827.

51. The RNA inhibitor according to any one of claims 31-50, wherein the sense strand comprises at least 15 consecutive nucleotides from any one of the sequences set forth in SEQ ID NO: 603-617.

52. The RNA inhibitor according to any one of claims 31-51, comprising a duplex selected from the following:
ds-m190, ds-m191, ds-m192, ds-m193, ds-m194, ds-m195, ds-m196, ds-m197, ds-m198, ds-m199, ds-m200, ds-m201, ds-m202, ds-m203, and ds-m204.

53. The RNA inhibitor according to any one of claims 1-52, further comprising a delivery system, wherein the delivery system is conjugated to the sense strand and/or the antisense strand, and the delivery system enables the RNA inhibitor to reach the target tissue to exert a gene silencing effect on the target RNA.

54. The RNA inhibitor according to claim 53, wherein the target tissue is ocular tissue, joint tissue, central nervous tissue, peripheral nervous tissue, tumor, liver tissue, kidney tissue, muscle tissue, or adipose tissue.

55. The RNA inhibitor according to claim 54, wherein the ocular tissue is optic nerve, trabecular meshwork, juxtacanalicular tissue, ganglion, episcleral vein, Schlemm's canal, or peripheral ocular tissue; preferably, the joint tissue comprises cartilage tissue, joint connective tissue, and bone tissue; preferably, the central nervous tissue comprises spinal cord tissue and brain tissue; preferably, the peripheral nervous tissue comprises intra-articular neural tissue and muscular neural tissue; preferably, the adipose tissue comprises subcutaneous adipose tissue and visceral adipose tissue.

56. The RNA inhibitor according to claim 55, wherein the ocular tissue is retinal ganglion, endothelial cell, extraocular muscle, or periorbital fat.

57. The RNA inhibitor according to any one of claims 53-56, wherein the delivery system is independently conjugated to one or more internal positions of the double-stranded ribonucleic acid.

58. The RNA inhibitor according to claim 57, wherein the internal positions are on the nucleobase, sugar ring, methylphosphonate bond, phosphorothioate diester bond, or phosphodiester bond.

59. The RNA inhibitor according to any one of claims 53-58, wherein the delivery system is a lipophilic structure comprising a lipophilic group and a linker, wherein the lipophilic group is connected to the double-stranded ribonucleic acid via the linker.

60. The RNA inhibitor according to claim 59, wherein the lipophilic structure is selected from aliphatic, alicyclic, and polycyclic compounds.

61. The RNA inhibitor according to claim 60, wherein the lipophilic structure contains saturated or unsaturated C16 or C22.

62. The RNA inhibitor according to any one of claims 59-61, wherein the linker is selected from a single bond, ether, thioether, urea, carbonate, amine, amide, maleimide-thioether, disulfide, phosphodiester, sulfonamide bond, product of a click reaction, and carbamate.

63. The RNA inhibitor according to claims 53-62, wherein the delivery system is

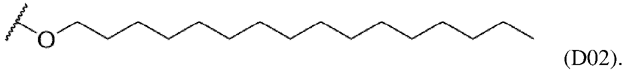

64. The RNA inhibitor according to any one of claims 53-63, wherein the delivery system is conjugated to the 5' end and/or 3' end of the antisense strand or antisense nucleic acid fragment.

65. The RNA inhibitor according to any one of claims 53-64, wherein the delivery system is conjugated to the 5' end and/or 3' end of the sense strand or sense nucleic acid fragment.

66. The RNA inhibitor according to any one of claims 53-65, wherein the delivery system is conjugated to the 5' end of the antisense strand or antisense nucleic acid fragment, and the ligand is conjugated to the 3' end of the sense strand or sense nucleic acid fragment, wherein the two ligands are identical or different.

67. The RNA inhibitor according to any one of claims 53-66, wherein the delivery system is conjugated to the 3' end of the antisense strand or antisense nucleic acid fragment, and the ligand is conjugated to the 5' end of the sense strand or sense nucleic acid fragment, wherein the two ligands are identical or different.

68. The RNA inhibitor according to any one of claims 53-67, wherein the delivery system is conjugated to the 5' end of the antisense strand or antisense nucleic acid fragment, and the ligand is conjugated to the 5' end of the sense strand or sense nucleic acid fragment, wherein the two ligands are identical or different.

69. The RNA inhibitor according to any one of claims 53-68, wherein the delivery system is conjugated to the 3' end of the antisense strand or antisense nucleic acid fragment, and the ligand is conjugated to the 3' end of the sense strand or sense nucleic acid fragment, wherein the two ligands are identical or different.

70. The RNA inhibitor according to any one of claims 53-69, wherein the number of delivery systems is 1, 2, 3, 4, 5, or 6.

71. The RNA inhibitor according to any one of claims 53-70, wherein the antisense strand comprises at least 15 consecutive nucleotides from any one of the sequences set forth in SEQ ID NO: 822-827.

72. The RNA inhibitor according to any one of claims 53-71, wherein the sense strand comprises at least 15 consecutive nucleotides from any one of the sequences set forth in SEQ ID NO: 618-623.

73. The RNA inhibitor according to any one of claims 53-72, selected from: Z1, Z2, Z3, Z4, Z5, Z6.

74. A pharmaceutical composition comprising the RNA inhibitor according to any one of claims 1-73, and/or a physiologically acceptable excipient, and/or carrier, and/or diluent.

75. The composition according to claim 74, wherein the pharmaceutically acceptable carrier comprises or is selected from an aqueous carrier, liposome, polymer, or polypeptide.

76. Use of an IGF-1R-targeting RNA inhibitor and its pharmaceutical composition in the manufacture of a medicament for treating an IGF-1R-related disease or pathology; preferably, the RNA inhibitor is siRNA; more preferably, the siRNA is administered locally or to the lesion site tissue of a subject in need thereof.

77. The use according to claim 76, wherein the IGF-1R-related disease or pathology includes diseases or symptoms associated with elevated IGF-1R levels.

78. The use according to any one of claims 76-77, wherein the IGF-1R-related disease or pathology includes thyroid eye disease, osteoarthritis, or neuropathic pain.

79. The use according to any one of claims 76-78, wherein the RNA inhibitor and its pharmaceutical composition are the RNA inhibitor according to any one of claims 1-73 or the pharmaceutical composition according to any one of claims 74-75.

80. Use of the RNA inhibitor according to any one of claims 1-73 or the pharmaceutical composition according to any one of claims 74-75 in the manufacture of a medicament for preventing or treating a disease or pathology or for reducing the risk of a disease or symptom.

81. The use according to claim 80, wherein the disease or pathology includes diseases or symptoms associated with elevated IGF-1R levels.

82. The use according to any one of claims 80-81, wherein the disease or pathology includes thyroid eye disease, osteoarthritis, or neuropathic pain.

83. A method for preventing or treating an IGF-1R-related disease or symptom, comprising administering an effective amount of an RNA inhibitor and its pharmaceutical composition to a subject in need thereof.

84. The method according to claim 83, wherein the RNA inhibitor and its pharmaceutical composition are the RNA inhibitor according to any one of claims 1-73 or the pharmaceutical composition according to any one of claims 74-75.

85. The method according to claims 83-84, wherein the RNA inhibitor, its pharmaceutically acceptable salt, or the pharmaceutical composition is administered to the subject via intraorbital injection, intra-articular injection, intrathecal injection, subcutaneous administration, intravenous administration, oral administration, rectal administration, or intraperitoneal administration.

86. The method according to any one of claims 83-85, comprising administering the RNA inhibitor locally or to the lesion site tissue of a subject in need thereof.

87. The method according to claim 86, wherein the local or lesion site tissue of the subject in need thereof includes ocular tissue, joint tissue, central nervous tissue, peripheral nervous tissue, tumor, liver tissue, kidney tissue, muscle tissue, or adipose tissue.

88. The method of claim 87, wherein said ocular tissue is optic nerve, trabecular meshwork, juxtacanalicular tissue, ganglion, episcleral vein, Schlemm's canal, or peripheral ocular tissue; preferably, said joint tissue comprises cartilage tissue, joint connective tissue, bone tissue; preferably, said central nervous tissue comprises spinal cord tissue, brain tissue; preferably, said peripheral nervous tissue comprises intra-articular neural tissue, muscular neural tissue; preferably, adipose tissue comprises subcutaneous adipose tissue, visceral adipose tissue.

89. The method of claim 88, wherein said ocular tissue is retinal ganglion, endothelial cell, extraocular muscle, or periorbital fat.

90. The method of any one of claims 83-89, wherein said IGF-1R-related disease or pathology comprises a disease or symptom associated with elevated IGF-1R levels.

91. The method of any one of claims 83-90, wherein said IGF-1R-related disease or pathology comprises thyroid eye disease, osteoarthritis, or neuropathic pain.

92. A method for inhibiting IGF-1R gene expression in a cell, tissue, or subject, comprising administering to the cell, tissue, or subject an effective amount of the RNA inhibitor of any one of claims 1-73 or the pharmaceutical composition of any one of claims 74-75.

93. The method of claim 92, wherein said disease or pathology comprises a disease or symptom associated with elevated IGF-1R levels.

94. The method of claim 93, wherein said disease or pathology comprises thyroid eye disease, osteoarthritis, or neuropathic pain.

95. A method for preventing or treating a disease or symptom, comprising administering an effective amount of the RNA inhibitor according to any one of claims 1-73 or the pharmaceutical composition according to any one of claims 74-75 to a subject in need thereof.

96. The method of claim 95, wherein the RNA inhibitor, a pharmaceutically acceptable salt thereof, or the pharmaceutical composition is administered to the subject via intraorbital injection, intra-articular injection, intrathecal injection, subcutaneous administration, intravenous administration, oral administration, rectal administration, or intraperitoneal administration.

97. The method of any one of claims 95-96, comprising administering to local or lesion site tissue of a subject in need thereof.

98. The method of claim 97, wherein the local or lesion site tissue of the subject in need thereof comprises ocular tissue, joint tissue, central nervous tissue, peripheral nervous tissue, tumor, liver tissue, kidney tissue, muscle tissue, or adipose tissue.

99. The method of claim 98, wherein said ocular tissue is optic nerve, trabecular meshwork, juxtacanalicular tissue, ganglion, episcleral vein, Schlemm's canal, or peripheral ocular tissue; preferably, said joint tissue comprises cartilage tissue, joint connective tissue, and bone tissue; preferably, said central nervous tissue comprises spinal cord tissue and brain tissue; preferably, said peripheral nervous tissue comprises intra-articular neural tissue and muscular neural tissue; preferably, adipose tissue comprises subcutaneous adipose tissue and visceral adipose tissue.

100. The method of claim 99, wherein said ocular tissue is retinal ganglion, endothelial cell, extraocular muscle, or periorbital fat.
